(19) Europäisches Patentamt
European Patent Office
Office européen des brevets

(11) Publication number: **0 011 283**
**B1**

(12) # EUROPEAN PATENT SPECIFICATION

(45) Date of publication of patent specification: **28.07.82**

(21) Application number: **79104479.5**

(22) Date of filing: **14.11.79**

(51) Int. Cl.³: **C 12 P 21/02,**
C 07 C 103/52,
A 61 K 37/02 //C12R1/465

(54) **New lactyl tetrapeptide, processes for preparation thereof and pharmaceutical compositions containing it.**

(30) Priority: **14.11.78 GB 4434678**
**31.07.79 GB 7926705**
**29.10.79 GB 7937343**

(43) Date of publication of application:
**28.05.80 Bulletin 80/11**

(45) Publication of the grant of the patent:
**28.07.82 Bulletin 82/30**

(84) Designated Contracting States:
**AT BE CH DE FR GB IT LU NL SE**

(56) References cited:
**TADEUSZ KORZYBISKI: "ANTIBIOTICS" Pergamon Press, PWN-Polish Scientific Publishers, Warszawa volume 1, pages 405—407 "Aspartocin"**

(73) Proprietor: **Fujisawa Pharmaceutical Co. Ltd.**
**3, Doshomachi 4-chome Higashi-ku**
**Osaka-shi, Osaka 541 (JP)**

(72) Inventor: **Kitaura, Yoshihiko**
**No. 523 Oudono**
**Sakurai (JP)**
Inventor: **Nakaguchi, Osamu**
**Green-Heights-Sone 418, No. 1-5-10**
**Shiroyamacho, Toyonaka (JP)**
Inventor: **Hemmi, Keiji**
**Otokoyamadanchi B-17-402**
**Otokoyamayumioka 1, Yawata (JP)**
Inventor: **Aratani, Matsuhiko**
**Villa Hirayama 201 No. 52, Awaji-Shimmachi**
**Higashiyodogawa-ku, Osaka (JP)**
Inventor: **Takeno, Hidekazu**
**No. 21, Kitamachi**
**Higashimuki, Nara (JP)**
Inventor: **Okada, Satoshi**
**No. 42, 4-chome**
**Nipponbashisuji, Naniwa-ku Osaka (JP)**
Inventor: **Tanaka, Hirokazu**
**No. 3-10-21 Hanayashikisouen**
**Takarazuka (JP)**

EP 0 011 283 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(72) Inventors (cont.)

Inventor: **Hashimoto, Masashi**
**No. 1-6-17, Satsukidai**
**Nakayama, Takarazuka (JP)**
Inventor: **Kuroda, Yoshio**
**No. 4-2-8, Kosobecho**
**Takatsuki (JP)**
Inventor: **Iguchi, Eiko**
**No. 6-6-14, Hannancho**
**Abeno-ku, Osaka (JP)**
Inventor: **Kohsaka, Masanobu**
**No. 5-26-8, Akasakadai**
**Sakai (JP)**
Inventor: **Aoki, Hatsuo**
**No. 4-13-3, Hata**
**Ikeda (JP)**
Inventor: **Imanaka, Hiroshi**
**No. 4-19-25, Sakurai**
**Shimamotocho, Mishima-gun Osaka (JP)**

(74) Representative: **Türk, Dietmar, Dr. rer. nat. et al,**
**Redies, Redies, Türk & Gille Bruckner Strasse 20**
**D-4000 Düsseldorf 13 (DE)**

# New lactyl tetrapeptide, processes for preparation thereof and pharmaceutical compositions containing it

This invention relates to a new lactyl tetrapeptide. More particularly, this invention relates to a new lactyl tetrapeptide and the pharmaceutically acceptable salt thereof, which have pharmacological activites, respectively to processes for the preparation thereof and to the pharmaceutical composition comprising the same.

Firstly, it is to be noted that this invention is originated from and based on the first and new discovery of the new active lactyl tetrapeptide, i.e. FR-900156 substance. That is, the FR-900156 substance was firstly and newly isolated in pure form from a culture broth obtained by fermentation of a new strain of belonging to the genus and characterized by the physico-chemical properties.

Thereafter, as a result of extensive study for elucidation of the chemical structure of FR-900156 substance, the inventors of this invention succeeded in determining the chemical structure thereof by commanding physical and chemical techniques so that they could give the sequential structure of the formula (1) indicated below to FR-900156 substance and proposed with a firm belief the possible stereoisomeric structure of the formula (Ia) indicated below for the same.

Formula (I):

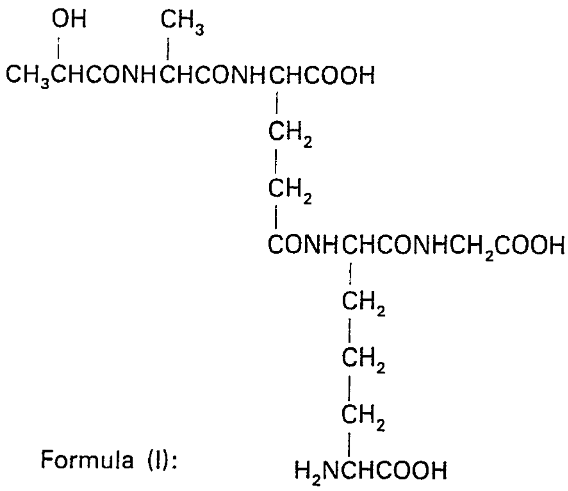

Formula (Ia):

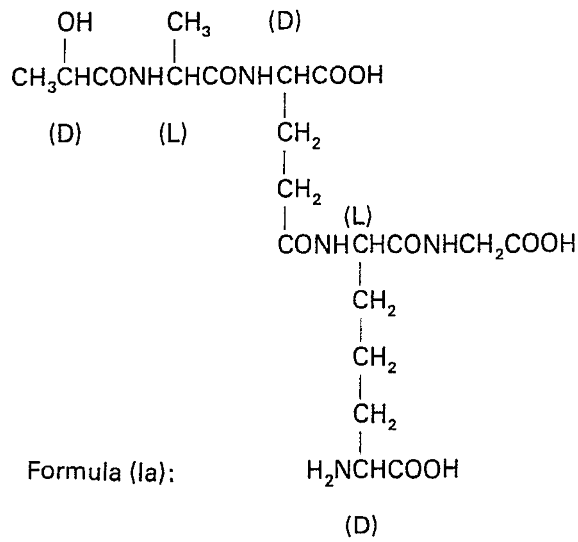

(D-Lactyl-L-alanyl-γ-D-glutamyl-(L)-meso-diaminopimelyl(L)-glycine.)

Further, after the structural elucidation of FR-900156 substance as explained above, inventors of the invention have continued extensive studies on total syntheses of the lactyl tetrapeptide (I) including the compound of the formula (Ia) and have succeeded in completing the industrially advantageous and applicable synthetic processes for preparation of the same, and further in preparing various stereoisomers included within the scope of the compound of the formula (I).

Details of the processes for preparation of lactyl tetrapeptide (I) including the stereoisomers thereof will be apparent from the following description.

(1) Synthesis:

① Process 1

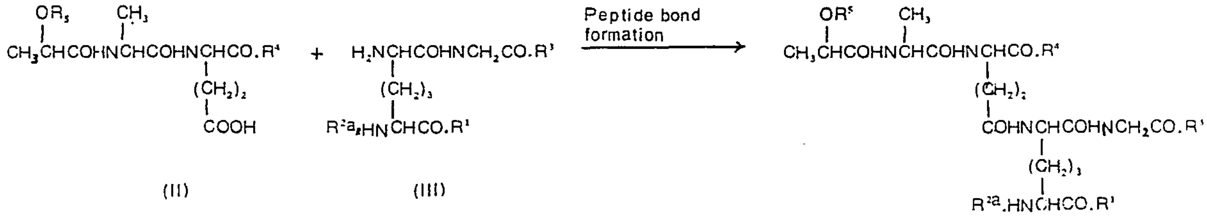


(II) (III) (I-1)

② Process 2

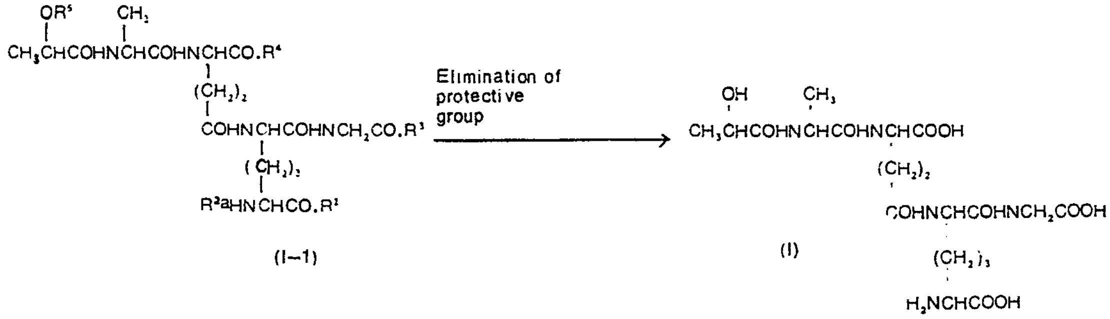


(I–1) (I)

(2) Fermentation:

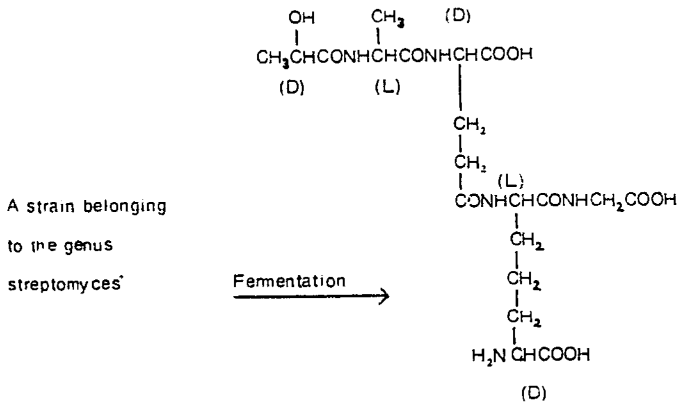


($I^a$)

In the above formulae,

$R^1$ is a hydroxy group, a protected hydroxy group or a protected hydrazino group of the formula: —NHNHY wherein Y is an amino protective group,

$R^{2a}$ is an amino protective group,

$R^3$ is a hydroxy group or a protected hydroxy group,

$R^4$ is a protected hydroxy group, and

$R^5$ is a hydroxy protective group.

Particulars of the above definitions and the preferred examples thereof are explained as follows.

a) Re. Amino protective group for $R^{2a}$

The amino protective group includes a conventional protective group for tentatively protecting an amino group, which is used in the field of amino acid and peptide chemistry. That is, in the peptide synthesis, it is understood that, for bonding a desired "reactive" amino group (—$NH_2$) with a desired "reactive" carboxy group (—COOH) to form a desired peptide bond (—CONH—) between them, it is preferable to tentatively protect the other undesired "reactive" amino group to convert it into an unreactive or less reactive protected amino group in the reaction in order to avoid the side reaction between the undesired "reactive" amino group and desired "reactive" carboxy groups. Further, it is understood that it is preferable that a protective group in such protected amino group is easily eliminable according to the necessity in the post treatment of the object peptide. Accordingly, an amino protective group to meet the above requirements can be used and suitable one should be selected according to the kind and property of the component to be used in this invention.

As preferred examples of the amino protective group, there is exemplified acyl, particularly organic acyl, for example, alkoxycarbonyl (e.g., methoxycarbonyl, ethoxycarbonyl, propoxycarbonyl, butoxycarbonyl, t-butoxycarbonyl, t-pentoxycarbonyl, etc.), substituted or unsubstituted aralkoxycarbonyl (e.g., benzyloxycarbonyl, p-nitrobenzyloxycarbonyl, p-phenylazobenzyloxycarbonyl, p-(p'-methoxy-phenylazo)-benzyloxycarbonyl, p-chlorobenzyloxycarbonyl, p-bromobenzyloxycarbonyl, $\alpha$-naphthylmethoxycarbonyl, p-biphenylisopropoxycarbonyl, etc.); and the like.

b) Re. Amino protective group for Y:

As to an amino protective group for Y in a protected hydrazino group of the formula: —NHNHY also, as the preferred examples of the said amino protective group, there are exemplified the same ones as illustrated in the preferred examples of amino protective group for $R^{2a}$.

c) Re. Protected hydroxy group for $R^1$, $R^3$, and $R^4$:

The groups $—CO{\cdot}R^1$, $—CO{\cdot}R^3$ and $—CO{\cdot}R^4$, in which each of $R^1$, $R^3$, and $R^4$ is a protected hydroxy group, mean "protected carboxy", and preferred examples of the protected carboxy may be exemplified by a conventional esterified carboxy such as an aliphatic hydrocarbon ester and an ester containing aromatic group.

In the peptide synthesis, it is understood that, for bonding a desired "reactive" carboxy group ($—COOH$) with a desired "reactive" amino group ($—NH_2$) to form the desired peptide bond ($—CONH—$), it is preferable to tentatively protect the other undesired "reactive" carboxy group to convert it into an "unreactive or less reactive" protected carboxy group in the reaction in order to avoid the side reaction between the undesired "reactive" carboxy group and the desired "reactive" amino groups, in which it is preferable that the reactivity of such protected carboxy group with amino group is less than that of carboxy group *per se* or its reactive derivative to provide a peptide in high yield. Further, it is understood that it is preferable that a carboxy protective group, particularly a protective group in a protected hydroxy ($R^1$, $R^3$, and $R^4$) of the protected carboxy group ($—CO{\cdot}R^1$, $—CO{\cdot}R^3$ and $—CO{\cdot}R^4$) is easily eliminable according to the necessity in the post treatment of the object peptide compound. Accordingly, any conventional protected hydroxy ($R^1$, $R^3$ and $R^4$) to meet the above requirements and suitable one should be selected according to the kind and property of the components to be used in this invention.

As preferred examples of the esterified carboxy for $—CO{\cdot}R^1$, $—CO{\cdot}R^3$, and $—CO{\cdot}R^4$ as mentioned above, there may be exemplified an aliphatic hydrocarbon ester such as alkyl (e.g., methyl, ethyl, propyl, isopropyl, butyl, tert-butyl, etc.) ester, an ester containing an aromatic group such as aralkyl (e.g., benzyl, phenethyl, etc.) ester, and the like.

From the above explanation, preferred examples of the protected hydroxy group for $R^1$, $R^3$ and $R^4$ can be exemplified by aliphatic hydrocarbonyloxy such as alkoxy, aralkyloxy, and the like.

d) Re. Hydroxy protective group for $R^5$.

It is understood that, in the presence of a hydroxy group in the component, in order to avoid a undesired side reaction, especially between said "reactive" hydroxy group and the other "reactive" carboxy group, it is preferable to tentatively protect a "reactive" hydroxy group to convert it into an "unreactive or less reactive" protected hydroxy, i.e. the group of $—OR^5$ of the Compound (II), wherein $R^5$ is a hydroxy protective group. Further, it is understood that it is preferable that said hydroxy protective group is easily eliminable according to the necessity in the post treatment of the object peptide compound. Accordingly, any conventional hydroxy protective group to meet the above requirements can be used and suitable one should be selected according to the kind and property of the component to be used.

Some preferred examples of the hydroxy protective group may be exemplified by an acyl such as alkanoyl (e.g., formyl, acetyl, propyonyl, butyril, isobutyrul, valeryl, bromoacethyl, dichloroacetyl, trifluoroacetyl, etc.) or the like.

A pharmaceutically acceptable salt of the lactyl tetrapeptides of the formula (I) may include a salt with an inorganic or organic base such as a sodium salt, potassium salt, calcium salt, ammonium salt, ethanolamine salt, triethylamine salt, dicyclohexylamine salt and the like, and an acid addition salt with organic or inorganic acid such as acetate, lactate, maleate, fumarate, tartarate, citrate, methane sulfonate, hydrochloride, sulfate, nitrate, phosphate and the like.

Detailed explanation of processes for preparation of a lactyl tetrapeptide of the formula (I) will be made in the following.

In advance of explaining the synthetic processes of this invention, the following is to be generally noted.

Firstly, as understood from the aforementioned formula (I), the lactyl tetrapeptide of this invention is composed of five components i.e. lactic acid and four amino acids, as indicated below.

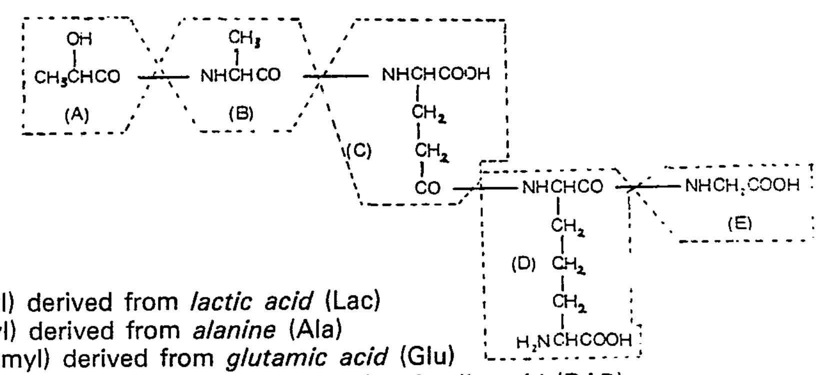

Note:—

A: Component (Lactyl) derived from *lactic acid* (Lac)
B: Component (Alanyl) derived from *alanine* (Ala)
C: Component (Glutamyl) derived from *glutamic acid* (Glu)
D: Component (Diaminopimelyl) derived from *diaminopimelic acid* (DAP)
E: Component (Carboxymethylamine) derived from *glycine* (Gly)

Accordingly, the compound (I) including the compound (I-a) can be synthesized by a stepwise condensation of each of the components, i.e., Lac, Ala, Glu, DAP and Gly according to a conventional method.

According to this invention, the lactyl tetrapeptide (I) and the pharmaceutically acceptable salt are prepared by the following scheme:

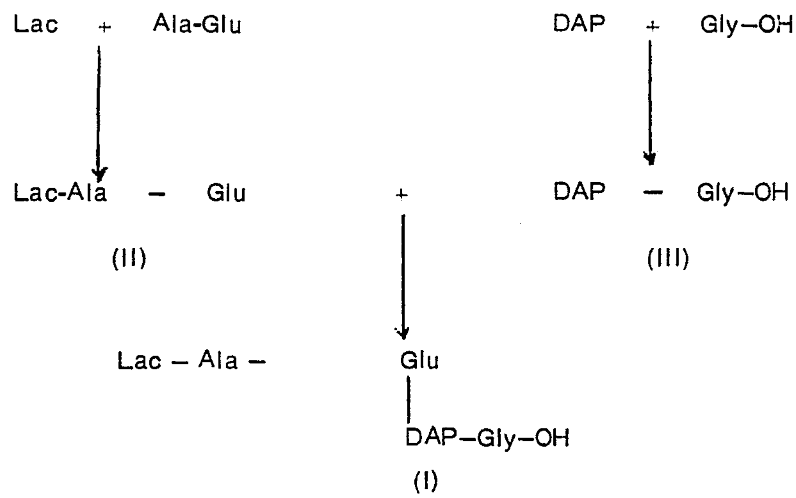

(II) (III) (I)

(1) Process 1: Compound (II) + Compound (III) → Compound (I-1)

This process relates to a method for preparing Compound (I-1) by reacting Compound (II) or its salt with a compound (III) or its salt.

The reaction of this process can be conducted as follows; that is, in one case, as the first step, the carboxy group of Compound (II) or its salt is usually activated in a conventional manner for the method such as an acid halide method, an azide method, acid anhydride or a mixed anhydride method, an activated ester method and the like, to be applied for the reaction with the Compound (III) to give Compound (I-1), and in another case, the Compound (II) or its salt is reacted with the Compound (III) or its salt directly in the presence of a conventional condensing agent such as N,N-dicyclohexylcarbodiimide and the like. Among these activation methods, preferred activation method for the carboxy group of the Compound (II) and preferred condensing agent as mentioned above are selected according to kinds of the carboxy protective group(s) of the Compound (II) and (III), and further to the reaction conditions (e.g., the kinds of the reaction solvent, reaction temperature and so on).

This reaction is preferably carried out in a solvent such as methylene chloride, chloroform, tetrahydrofuran, dioxane, ethyl acetate, methanol, ethanol, water and the like under ice-cooling at ambient temperature and the reaction in the presence of a condensing agent is usually carried out in an anhydrous, but not critical, conditions.

(2) Process 2: Compound (I-1) → Compound (I)

This process relates to a method for preparing Compound (I) by subjecting Compound (I-1) to removal reaction of protective groups of amino, carboxy and hydroxy groups for $R^{2a}$, $R^1$, $R^3$, $R^4$ and $R^5$.

Process 2-1: Elimination of amino protective group for $R^{2a}$ and Y

The reaction for removal of amino protective group for $R^{2a}$ and Y is carried out by conventional methods such as catalytic reduction method, liquidammonia-alkali metal method, acid method, zinc-acid method, base method, hydrazine method.

1) Catalytic reduction method:

This method is preferably applied in case that the amino protective group for $R^{2a}$ and Y of Compound (I-1) is one which is removable by catalytic reduction. As preferred examples of such an amino protective group there may be exemplified substituted or unsubstituted aralkoxycarbonyl such as benzyloxycarbonyl, p-phenylazobenzyloxycarbonyl, p-(p'-methoxyphenylazo)-benzyloxycarbonyl, p-nitrobenzyloxycarbonyl, etc.

This catalytic reduction is carried out in a conventional manner, and suitable catalysts to be used in catalytic reduction are conventional ones such as platinum catalysts (e.g., platinum plate, spongy platinum, platinum black, colloidal platinum, platinum oxide or platinum wire, etc.), palladium catalysts (e.g., spongy palladium, palladium black, palladium oxide, palladium on carbon, colloidal palladium, palladium on barium sulfate, palladium on barium carbonate, etc.), nickel catalysts (e.g., reduced nickel, nickel oxide, Raney nickel, etc.), cobalt catalysts (e.g., reduced cobalt, Raney cobalt, etc.), iron catalysts (e.g., reduced iron, Raney iron, etc.), copper catalysts (e.g., reduced copper, Raney copper, Ullman

copper, etc.) and the like.

The reduction is usually carried out in a solvent. A suitable solvent to be used may be, e.g., water, methanol, ethanol, propanol, ethyl acetate, tetrahydrofuran, dioxane, N,N-dimethylformamide, acetic acid, a mixture of water and methanol, ethanol, tetrahydrofuran, dioxane or ethyl acetate, and other conventional organic solvent or mixture thereof.

Further, the reduction is preferably carried out in the presence of an acid such as acetic acid and the like.

The reaction is preferably carried out under somewhat milder conditions such as cooling or warming.

In this method, in case that a protected hydroxy group for $R^3$ and $R^4$ is, for example, substituted or unsubstituted aralkyl ester type one (e.g., benzyl ester, p-nitrobenzyl ester, p-chlorophenyl ester, p-phenylazobenzyl ester, etc.), such a protective group is simultaneously removed in this process to give a Compound (I).

ii) Acid method:

ii)-1 Method of use of trifluoroacetic acid or formic acid:

This method is preferably applied in case that the amino protective group for $R^{2a}$ and Y is one which is removable by treating with trifluoro-acetic acid or formic acid. Preferred examples of such an amino protective group may be exemplified by a group such as branched alkoxycarbonyl, (e.g., t-butoxycarbonyl, t-pentoxycarbonyl, etc.), substituted or unsubstituted aralkoxycarbonyl (e.g., p-methoxybenzyloxycarbonyl, etc.).

This reaction is conventionally carried out in a solvent such as methylene chloride, chloroform, acetic acid, water and the like in the presence of trifluoroacetic acid, or formic acid, and anisole is preferably added thereto.

Trifluoroacetic acid and formic acid are also used as the solvent.

This reaction is usually carried out under ice-cooling to at ambient temperature.

In this method, in case that a protected hydroxy group for $R^3$ and $R^4$ is, for example, a branched alkyl ester (e.g., t-butyl ester, etc.), substituted or unsubstituted aralkyl ester (e.g., diphenylmethyl ester, p-methoxybenzyl ester, etc.), such a protective group is simultaneously removed to give a Compound (I).

ii)-2 Method of use of hydrochloric acid or p-toluenesulfonic acid:

This method is preferably applied in case that an amino protective group for $R^{2a}$ and Y is one which is removed by treating with hydrochloric acid or p-toluenesulfonic acid.

Preferred examples of such an amino protective group may be exemplified by e.g., substituted or unsubstituted branched alkoxycarbonyl (e.g., t-butoxycarbonyl, 1-(p-biphenyl)-1-methylethoxycarbonyl, etc.) and the like in addition to one as illustrated in the above ii)-1.

This reaction is carried out in a solvent such as ethyl acetate, methylene chloride, chloroform, tetrahydrofuran and the like in the presence of hydrochloric acid or p-toluenesulfonic acid or the like.

In this method, p-toluenesulfonic acid is used as an acid, anisole is preferably added.

This reaction is preferably carried out under ice-cooling to at ambient temperature.

In this method, in case that a protected hydroxy group for $R^3$ and $R^4$ is, for example, a branched alkyl ester (e.g., t-butyl ester etc.), substituted or unsubstituted aralkyl ester (e.g., diphenylmethyl ester, p-methoxybenzyl ester, etc.), such a protective group is simultaneously removed to give a Compound (I).

Process 2-2: Elimination of protective group of the protected hydroxy group for $R^1$, $R^3$ and $R^4$.

The reaction for removal of the protective group of the protected hydroxy group for $R^1$, $R^3$ and $R^4$ (i.e. a protective group of carboxy group for $CO.R^1$, $CO.R^3$ and $CO.R^4$) is conducted by a conventional method such as hydrolysis, reduction or the like, which are explained in the following.

(i) For hydrolysis which refers to the same meaning as solvolysis including, for example, acidolysis, alcoholysis, aminolysis, hydrozinolysis, etc.:

Hydrolysis is preferably carried out in the presence of an acid or base.

Suitable acid includes an inorganic acid (e.g., hydrochloric acid, hydrobromic acid, sulfuric acid, etc.), an organic acid (e.g., formic acid, acetic acid, trifluoroacetic acid, propionic acid, benzenesulfonic acid, p-toluenesulfonic acid, etc.), an acidic ion-exchange resin and the like.

Suitable base includes an inorganic base such as alkali or alkaline earth metal hydroxide, carbonate or bicarbonate (e.g., sodium hydroxide, potassium carbonate, sodium bicarbonate, calcium hydroxide, magnesium hydroxide, etc.), ammonium hydroxide or the like; an organic base such as an alkoxide or phenoxide of the above metal, (e.g., sodium ethoxide, sodium methoxide, lithium phenoxide etc.), an amine such as mono-, di- or trialkylamine (e.g., methylamine, ethylamine, propylamine, isopropylamine, butylamine, N,N-dimethyl-1,3-propanediamine, trimethylamine, triethylamine, etc.); unsubstituted, mono- or disubstituted arylamine (e.g., aniline, N-methylaniline, N,N-dimethylaniline, etc.); or a heterocyclic base (e.g., pyrrolidine, morpholine, N-methylmorpholine, N-methylpiperidine,

N,N-dimethylpiperazine, pyridine, etc.), hydrazines (e.g., hydrazine, methylhydrazine, ethylhydrazine, etc.), a basic ion-exchange resin or the like.

The hydrolysis is preferably conducted under somewhat milder conditions such as cooling or warming and usually is a solvent which does not have adverse influence to the reaction, e.g., water, a hydrophilic solvent such as methanol, ethanol, propanol, acetone, N,N-dimethylformamide, tetrahydrofuran, dioxane, dimethylsulfoxide, etc. or a mixture thereof, and other hydrophobic solvent such as benzene diethylether, etc. may also be used as a solvent. A liquid abovementioned acid or base can also be used as a solvent.

(ii) For reduction:

Reduction, including chemical reduction and catalytic reduction, is carried out in a conventional manner.

Suitable reducing agents to be used in chemical reduction are a metal (e.g., tin, zinc, iron, etc.), or a combination of such metal and/or metallic compound (e.g., chromium chloride, chromium acetate, etc.) and an organic or inorganic acid (e.g., formic acid, acetic acid, propionic acid, trifluoroacetic acid, p-toluenesulfonic acid, hydrochloric acid, hydrobromic acid, etc.).

Suitable catalysts to be used in catalytic reduction are conventional ones such as platinum catalysts (e.g., platinum plate, spongy platinum, platinum black, colloidal platinum, platinum oxide or platinum wire, etc.), palladium catalysts (e.g., spongy palladium, palladium black, palladium oxide, palladium on carbon, colloidal palladium, palladium on barium sulfate, palladium on barium carbonate, etc.), nickel catalysts (e.g., reduced nickel, nickel oxide, Raney nickel, etc.), cobalt catalysts (e.g., reduced cobalt, Raney cobalt, etc.), iron catalysts (e.g., reduced iron, Raney iron, etc.), copper catalysts (e.g., reduced copper, Raney copper, Ullman copper, etc.) or the like.

The reduction is usually carried out in a solvent. A suitable solvent to be used may be, e.g., water, methanol, ethanol, propanol, and other conventional organic solvent or a mixture thereof. Additionally, liquid above-mentioned acids to be used in chemical reduction can also be used as a solvent. Further, a suitable solvent to be used in catalytic reduction may be, e.g., the above-mentioned solvent and the other conventional solvent, such as diethyl ether, dioxane, tetrahydrofuran, etc. or a mixture thereof.

The reaction is preferably carried out under somewhat milder conditions such as cooling or warming.

Among these methods for removal of protective groups, preferred one and appropriate combination methods are to be selected according to kinds of the protective group in the protected hydroxy to be removed off.

It is to be noted that this process includes the following cases of removal of the protective groups, that is, one case that all of the protective group in the protected hydroxy group for $R^3$ and $R^4$, the amino protective group for $R^{2a}$ and the hydroxy protective group for $R^5$ in the Compound (I-1) are simultaneously removed by this method to be employed in the reaction, and the other case that protective group in the protected hydroxy for $R^3$ and $R^4$, the amino protective group for $R^{2a}$ and the hydroxy protective group are sequencially and stepwise removed by this method which, however, is appropriately selected according to the kinds of the protective group to be removed.

Process 2-3: Removal of hydrazino group

A protected hydrazino group of the formula: —NHNHY, wherein Y is an amino protective group, in —CO.NHNHY group, can be removed by subjecting the Compound (I-1) at first to the reaction of Process 2-1 for eliminating the amino protective group, for $R^{2a}$ and Y to give $—CO.NHNH_2$ group and then subjecting the reaction product to the reaction of this step to give COOH group, particular of this reaction step is as follows.

The reaction of this step is carried out in a conventional manner by treating the Compound (I-1) with a conventional oxidizing agent which is capable of oxidizing a group of the formula: $—CONHNH_2$ to form into a group of the formula: —COOH and accordingly preferred example of such an oxidizing agents may be halogen such as iodine, etc., perhalogenic acid such as periodic acid or its salt (e.g., sodium salt, potassium salt, etc.), perchloric acid, etc., N-haloimide such as N-bromosuccinimide, etc., lead tetraacetate, hydrogen peroxide or its salt (e.g., nickel peroxide, etc.), metal oxide such as mercuric oxide, manganese dioxide, etc., cupric compound (e.g., cupric acetate, cupric sulfate, etc.) and the like.

This reaction is usually carried out in a solvent such as water, acetic acid, methanol, ethanol, tetrahydrofuran, dioxane and the like and a mixture thereof, which should be appropriately selected in accordance with the kind of oxidizing agent to be used.

This reaction is usually carried out under ice-cooling to at ambient temperature, or under reflux.

Process 2-4: Elimination of hydroxy protective group for $R^5$

This reaction for removal of hydroxy protective group for $R^5$ is carried out by conventional methods such as illustrated for the Process 2-2, as explained hereinabove, among which the hydrolysis, which is conducted in the presence of a base is preferably employed. Accordingly particulars of hydrolysing agent, reaction conditions, etc. are to be referred to those as explained in details for Process 2-2, especially for hydrolysis therein.

(2) Fermentation: A strain of Streptomyces → Compound (Ia)

The compound (Ia) (hereinafter referred to as FR-900156 substance) can be produced by fermentation of a FR-900156 substance producing strain belonging to the genus Streptomyces in a nutrient medium, details of which are explained in the following.

The microorganism which can be used for the production of the FR-900156 substance is a strain belonging to the genus Streptomyces, among which a strain of Streptomyces olivaceogriseus and Streptomyces violaceus have been newly isolated from a soil sample as a suitable strain of a FR-900156 substance-producing strain belonging to the genus Streptomyces.

I. Re. Streptomyces olivaceogriseus nov. sp. C—353:

Streptomyces olivaceogriseus nov. sp. C—353 has been isolated from a soil sample collected at Kochi Prefecture, Japan and deposited with and added to a permanent stock culture collection of the America Type Culture Collection under the number ATCC 31427.

Streptomyces olivaceogriseus nov. sp. C—353 (ATCC 31427) has the following morphological, cultural and physiological characteristics.

(1) Morphological characteristics:

Microscopic observations were made on cultures which were grown from 10 to 14 days on sucrose-nitrate agar, glycerin-asparagine agar, yeast-malt extract agar, oatmeal agar, starch-inorganic salts agar and Bennett agar. Sporophore morphology was observed on undisturbed plates cultures.

1. Type of branching of spore-forming hyphae:
   Monopodial branching
2. Form of spore-forming hyphae:
   Retinaculiaperti
   (closed spirals, loops)
3. Number of spores:
   5—20 spores
4. Surface appearance and size of spores:
   Smooth
   0.6—1.2 x 1.0—1.9 micron
5. Existence of zoospores:
   Not observed
6. Existence of sporangium:
   Not observed
7. Formation of spores:
   At aerial mycelium
8. Fragmentation of substrate mycelium:
   Not observed

(2) Cultural characteristics:

The following observations were made on slant cultures which were grown on various media at 30°C for 10—14 days.

| Medium | Aerial mass color | Reverse side of colony | Soluble Pigment |
|---|---|---|---|
| Sucrose-nitrate agar | none or very thin, powdery | pale yellow, small colonies | none |
| Glucose-asparagine agar | light gray to greenish gray, powdery | pale yellow to pale yellowish brown, small colonies | none |
| Glycerin-asparagine agar | thin, powdery light gray | pale yellowish brown, small colonies | none or trace |
| Starch-inorganic salts agar | olive gray, powdery | grayish yellow brown, small colonies | none |
| Tyrosine agar | light olive gray, thin powdery | yellowish brown, small colonies | light brown |
| Nutrient agar | none | pale yellow, flat | none |
| Yeast-malt extract agar | greenish gray, powdery | yellowish brown, wrinkled colonies | none |
| Oatmeal agar | light gray to greenish gray, powdery | colorless, small colonies | none |
| Peptone-yeast iron agar | none | colorless to pale yellow, slightly wrinkled | light brown |
| Glucose-peptone gelatin stab | white, thin powdery | colorless to pale yellow, wrinkled colonies | brown |
| Milk | white, very thin powdery | colorless, surface ring growth | none |

(3) Biological and physiological properties:

1. Temperature requirements (on Bennett agar slants)
   growth from 15°C to 40°C optimum 28°C
2. Hydrolysis of starch (on starch-inorganic salts agar)
   hydrolyzed weakly
3. Liquefaction of gelatin (on glucose-peptone gelatin stab)
   negative
4. Action on milk
   no coagulation
   no peptonization
5. Production of melanin (on tyrosine agar, peptone-yeast iron agar and tryptone-yeast broth)
   positive
6. Utilization of various carbon compounds (on Pridham-Gottlieb basal agar medium)

| | |
|---|---|
| L-Arabinose | — |
| D-Xylose | ± |
| D-Glucose | + |
| D-Fructose | + |
| D-Galactose | + |

| | |
|---|---|
| Sucrose | + |
| Glycerin | + |
| Inositol | + |
| Lactose | + |
| L-Rhamnose | — |
| Maltose | + |
| Raffinose | — |
| D-Mannitol | + |
| D-Mannose | + |
| Salicin | — |

Symbols: +, good utilization; ±, doubtful utilization; —, no utilization

7. Cell wall pattern I (LL-diaminopimelic acid)

As a result of looking up the strain possessing the characteristics mentioned above by referring to the literature, namely "Bergey's Manual of Determinative Bacteriology", eighth edition (1975), and "The International Streptomyces Project Reports" written by E. B. Shirling and D. Gottlieb cf. International Journal of Systematic Bacteriology Vol. 18, pages 69 and 279 (1968), Vol. 19, pages 391 (1969) and Vol. 22, pages 265 (1972), Streptomyces eurythermus and Streptomyces galbus (Okami) have been detected as species having relatively analogous characteristics to those of the strain ATCC 31427.

The strain ATCC 31427, however, is different from the analogous species in the following:

Streptomyces eurythermus (Okami):

A strain of the species can assimilate arabinose and can not assimilate inositol. Assimilation of rhamnose and raffinose by a strain of the species is indefinite. Loops are not formed. Straights or flexous mycelium are sometimes observed.

Streptomyces galbus:

Open-spirals are generally formed. A strain of the species produces a soluble yellow—yellow green pigment, and can not assimilate sucrose.

In view of the result of the observation, the strain ATCC 31427 can be judged to be a new species belonging to the genus Streptomyces and this has been designated as Streptomyces olivaceogriseus nov. sp. C—353.

II. Re. Streptomyces violaceus No. 6724

Streptomyces violaceus No. 6724 was isolated from a soil sample collected at Ishigaki island, at Okinawa Prefecture, Japan, and deposited with and added to a permanent stock culture collection of the American Type Culture Collection under the number ATCC 31481.

Streptomyces violaceus No. 6724 has following morphological, cultural and physiological characteristics.

(1) Morphological characteristics

Microscopic observations were made on cultures which were grown on sucrose-nitrate agar, glycerin-asparagine agar, starch-inorganic salts agar, yeast-malt extract agar and oatmeal agar at 30°C for 10 to 14 days.

1. Type of branching of spore-forming hyphae:
   Monopodial branching
2. Form of spore-forming hyphae:
   Spirales
3. Numbers of spores:
   10—50
4. Surface appearance and size of spore:
   Spiny
   0.3—0.7 × 0.6—1.1 $\mu$
5. Existence of zoospore and sporangium:
   Not observed
6. Formation of spores:
   At aerial mycelium
7. Fragmentation of substrate mycelium:
   Not observed

(2) Cultural characteristics

The following observations were made on cultures which were grown on various media at 30°C

for 10 to 14 days.

| Medium | Aerial mass color | Reverse side of colony | Soluble Pigment |
|---|---|---|---|
| Sucrose-nitrate agar | purplish white, powdery | small colonies | purple |
| Glucose-asparagine agar | purplish white, powdery | yellowish red, small colonies | pink |
| Glycerin-asparagine agar | pinkish white-pink, powdery | Yellowish red, small colonies, slightly wrinkled | pink-red |
| Starch-inorganic salts agar | whitish red, short cottony | yellowish red, slightly wrinkled | red |
| Tyrosine agar | none | red, wrinkled colonies | none or trace |
| Nutrient agar | none or very thin powdery | colorless-purple, flat | reddish purple |
| Yeast-malt extract agar | pink-purplish pink, short cottony | reddish brown – purplish brown, wrinkled colonies | reddish purple |
| Oatmeal agar | pink-purplish pink, powdery | colorless-purplish pink, small colonies | pink-purple |
| Peptone-yeast iron agar | none | colorless, wrinkled colonies | brown |
| Glucose-peptone gelatin stab | white, thin powdery | red, wrinkled colonies | faint brown |
| Milk | thin powdery | red growth on surface | reddish yellow |

Reverse mycelium pigment is pH indicator, changing from red to violet (purple) with addition of 0.05N NaOH or from violet to red (pink) with addition of 0.05N HCl. Soluble pigment is also pH sensitive, showing the same changes for reverse mycelium pigment.

(3) Biological and physiological properties

1. Temperature requirements (on Bennett agar slants)
   growth from 15°C to 40°C (optimum 28°C)
2. Liquefaction of gelatin (on glucose-peptone gelatin stab)
   negative
3. Hydrolysis of starch (on starch-inorganic salts agar)
   positive
4. Action on milk
   coagulation, no peptonization
5. Production of melanoid pigment
   (on tyrosine agar, peptone-yeast iron agar and tryptone-yeast extract broth)
   positive
   very weak or not on tyrosine agar
6. Utilization of various carbon compounds
   (on Pridham-Gottlieb basal agar medium)

| | |
|---|---|
| L-Arabinose | + |
| D-Xylose | + |
| L-Rhamnose | + |
| D-Glucose | + |
| D-Fructose | + |
| D-Mannose | + |
| D-Galactose | + |
| Sucrose | + |
| Lactose | + |
| Maltose | + |
| Raffinose | + |
| Inulin | ± |
| Cellulose | — |
| Chitin | — |
| Glycerin | + |
| D-Mannitol | + |
| Salicin | + |
| Inositol | + |
| Na-Acetate | — |
| Na-Citrate | + |
| Na-Succinate | + |

Symbols: +, good utilization
±, doubtful utilization
—, no utilization

The FR-900156 substance of this invention is produced when the FR-900156 substance-producing strain belonging to the genus Streptomyces (e.g. Streptomyces olivaceogriseus nov. sp. C—353 and Streptomyces violaceus, etc.) is grown in a nutrient medium containing sources of assimilable carbon and nitrogen under aerobic conditions (e.g. shaking culture, submerged culture, etc.).

The preferred sources of carbon in the nutrient medium are carbohydrates such as glucose, fructose, glycerin, starch and the like. Other sources which may be included are lactose, arabinose, xylose, dextrin, molasses and the like.

The preferred sources of nitrogen are yeast extract, peptone, gluten meal, cottonseed meal, soybean meal, corn steep liquor, dried yeast, wheat germ, etc., as well as inorganic and organic nitrogen compounds such as ammonium salts (e.g. ammonium nitrate, ammonium sulphate, ammonium phosphate, etc.), urea, amino acid and the like.

The carbon and nitrogen sources, though advantageously employed in combination, need not be used in their pure form because less pure materials, which contain traces of growth factors and considerable quantities of mineral nutrients, are also suitable for use. When desired, there may be added to the medium mineral salts such as calcium carbonate, sodium or potassium phosphate, sodium or potassium chloride, magnesium salts, copper salts and the like. If necessary, especially when the culture medium foams seriously a defoaming agent, such as liquid paraffin, fatty oil, plant oil, mineral oil or silicone may be added.

As in the case of the preferred methods used for the production of other antibiotics in massive amounts, submerged aerobic cultural conditions are preferred for the production of the FR-900156 substance in massive amounts. For the production in small amounts, a shaking or surface culture in a flask or bottle is employed. Furthermore, when the growth is carried out in large tanks, it is preferable to use the vegetative form of the organism for inoculation in the production tanks in order to avoid growth lag in the process of production of the FR-900156 substance. Accordingly, it is desirable first to produce a vegetative inoculum of the organism by inoculating a relatively small quantity of culture medium with spores or mycelia of the organism and culture them and then to transfer the cultured vegetative inoculum aseptically to large tanks. The medium, in which the vegerative inoculum is produced, is substantially the same as or different from the medium utilized for the production of the FR-900156 substance.

Agitation and aeration of the culture mixture may be accomplished in a variety of ways. Agitation may be provided by a propeller or similar mechanical agitation equipment, by revolving or shaking the fermentor, by various pumping equipment or by the passage of sterile air through the medium. Aeration may be effected by passing sterile air through the fermentation mixture.

The fermentation is usually conducted at a temperature between about 20°C and 40°C, preferably 30°C, for a period of about 50 hours to 100 hours.

The FR-900156 substance can be recovered from the culture medium by conventional means which are commonly used for the recovery of other known antibiotics.

In general, most of the FR-900156 substance produced are found in the cultured broth, and accordingly the FR-900156 substance can be separated from the filtrate, which is obtained by filtering of centrifuging the culture broth, by a conventional method such as concentration under reduced pressure, lyophilization, pH adjustment, treatment with a resin (e.g. anion or cation exchange resin, non-ionic adsorption resin, etc.), treatment with an adsorbent (e.g. activated charcoal, silicic acid, silica gel, cellulose, alumina, etc.), crystallization, recrystallization and the like.

The FR-900156 substance thus produced in the culture broth can be isolated in the free form, i.e., FR-900156 substance per se and when the solution or its concentrate containing the FR-900156 substance is treated with a base, i.e. with an inorganic base such as an alkali metal compound (e.g. sodium hydroxide, sodium carbonate, sodium bicarbonate, potassium hydroxide, etc.), an alkaline earth metal compound (e.g. calcium hydroxide, magnesium hydroxide, etc.), ammonia and the like, with an organic base (e.g. ethanolamine, triethylamine, dicyclohexylamine, etc.); or with an acid i.e. with an inorganic acid (e.g. hydrochloric acid, sulfuric acid, phosphoric acid, etc.); or with an organic acid (e.g. formic acid, acetic acid, p-toluenesulfonic acid, citric acid, tartaric acid etc.) during operation of the processes, e.g. extraction, isolation or purification processes, the FR-900156 substance may be transformed into and isolated in the form of the corresponding salts thereof. Alternatively, thus prepared salts of the FR-900156 substance can easily be converted to the free form, i.e. FR-900156 substance *per se* in the conventional manner.

Further, the FR-900156 substance obtained in the free form may be converted to the corresponding salts thereof with a base or an acid as mentioned above in a conventional manner.

Accordingly, it is to be understood that this invention includes within the scope thereof the FR-900156 substance as well as salts thereof as mentioned above.

The FR-900156 substance possesses the following physical and chemical properties (The following data are those of the product obtained by Example for fermentation (3)):

1) Form and color:
   White powder
2) Nature of substance:
   Amphoteric
3) Color reaction:
   Positive; each reaction with ninhydrin, potassium permanganate and sulfuric acid
   Negative; Dragendorff reaction and Ehrlich reaction
4) Solubility:
   Soluble; water
   Sparingly soluble; methanol
   Insoluble; ethanol, acetone, ethyl acetate, benzene, hexane, chloroform
5) MP;
   143—148 (dec.
6) Specific rotation
   $[\alpha]_D^{25} = -27.1$ (c=0.4 in water)
7) Ultraviolet absorption spectrum:
   end absorption
8) Infrared absorption spectrum (KBr):
   1050, 1130, 1235, 1340, 1400, 1450, 1535, 1660, 1735, 2950, 2980, 3080, 3350 $cm^{-1}$
9) Elemental analysis:
   Qualitative analysis revealed that the FR-900156 substance comprises the following elements: Carbon, Hydrogen, Nitrogen and Oxygen
10) Thin layer chromatography:

| Stationary phase | Developing Solvent | Rf Value |
|---|---|---|
| Eastman cellulose sheet*1 | BuOH, Acetic acid, Water (4:1:2) | 0.35 |
| Silicagel sheet Merck*2 | 60% i-propanol-water | 0.65 |

Note) *1: Trade name, made by Eastman Kodak Co.
*2: Trade name, made by Merck & Co.

11) Molecular weight:
   Mass spectrometory (Field desorption method):
   M = 519 (base peak: M+1 = 520)
12) Nuclear magnetic resonance absorption spectrum:
   As shown in the Figure of accompanying drawing.
   (Solvent: $D_2O$, Reference: TMSP)

13) Amino acid analysis:

| | Molar ratio |
|---|---|
| Glycine | 1.00 |
| Glutamic acid | 1.06 |
| Alanine | 1.04 |
| $\alpha,\varepsilon$-Diaminopimelic acid | 1.03 |

(Molar ratio is expressed as assuming Glycine = 1.00)

14) Molecular formula:

$C_{20}H_{33}N_5O_{11}$

Further, physical and chemical properties of more purified sample of FR-900156 substance (i.e. a product obtained by Example for fermentation (4)) was measured, as a result of which MP of said sample is 147—153°C (dec.) and others properties thereof were the same as the above.

From analysis of the above physicochemical properties and the further investigation for elucidation of chemical structure, the chemical structure of the FR-900156 substance has been elucidated as follows:

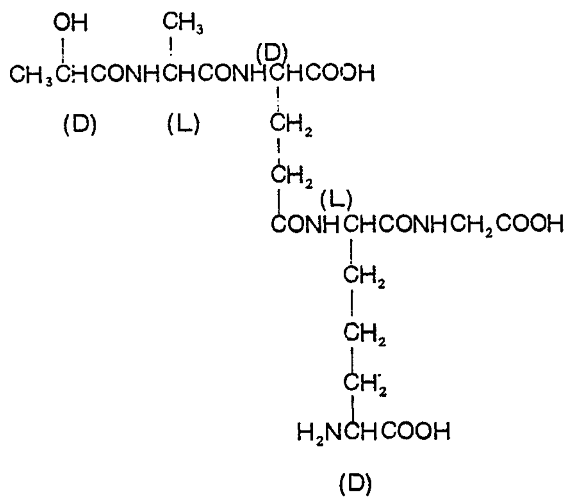

(D-Lactyl-L-alanyl-$\gamma$-D-glutamyl-(L)-meso-diaminopimelyl(L)-glycine)

It is noted that the object compound, lactyl tetrapeptide (I), there may be one or more stereoisomers being due to the asymmetric carbon atoms in the molecule and all of such isomers are included within the scope of the object compound of this invention.

The starting Compounds (II) and (III) each includes new compound and can be prepared by conventional aminopeptide synthesis methods according to the following synthesis chart. Particulars of said synthesis methods are explained by the following Preparations 1—37 and so are to be referred to the explanation thereof.

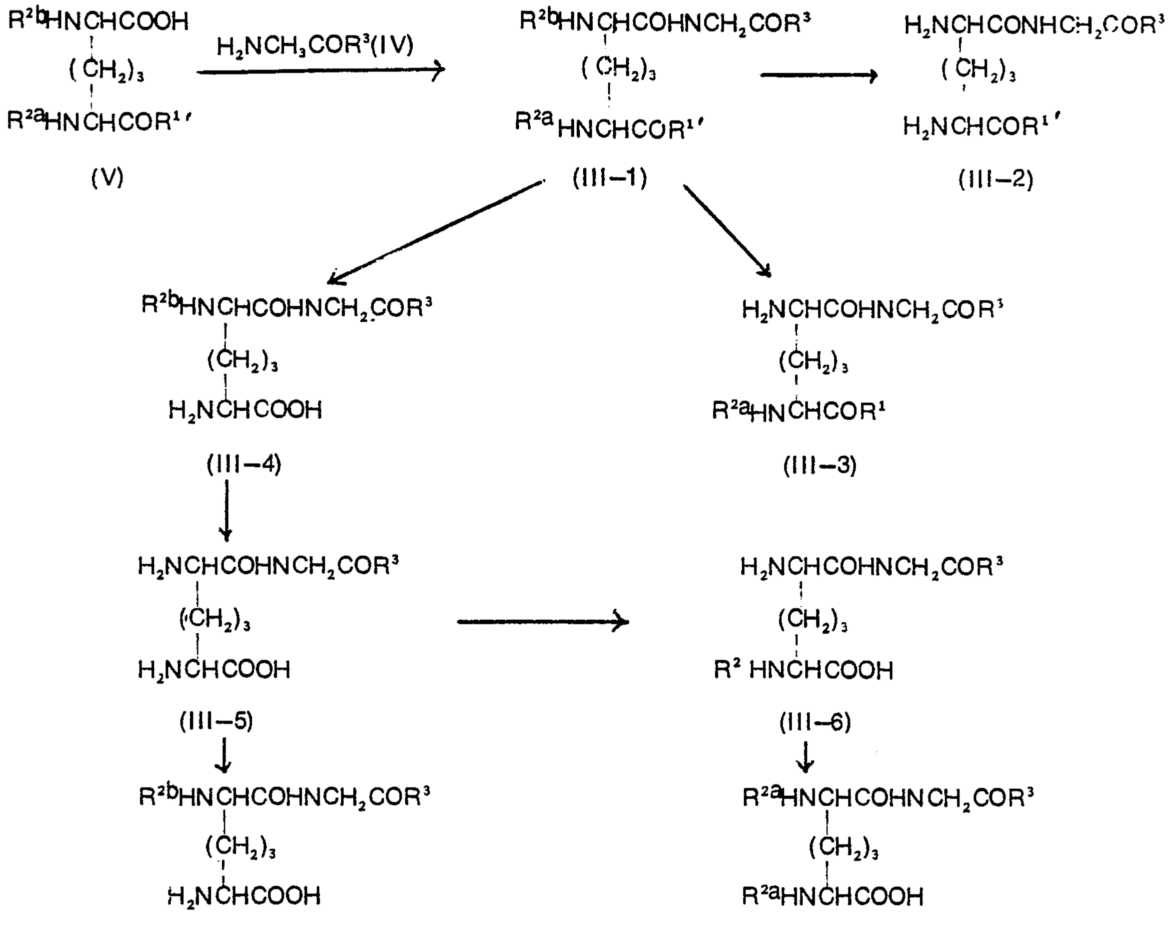


(V) (III–1) (III–2) (III–4) (III–3) (III–5) (III–6) (III–7) (III–8)

Process 12

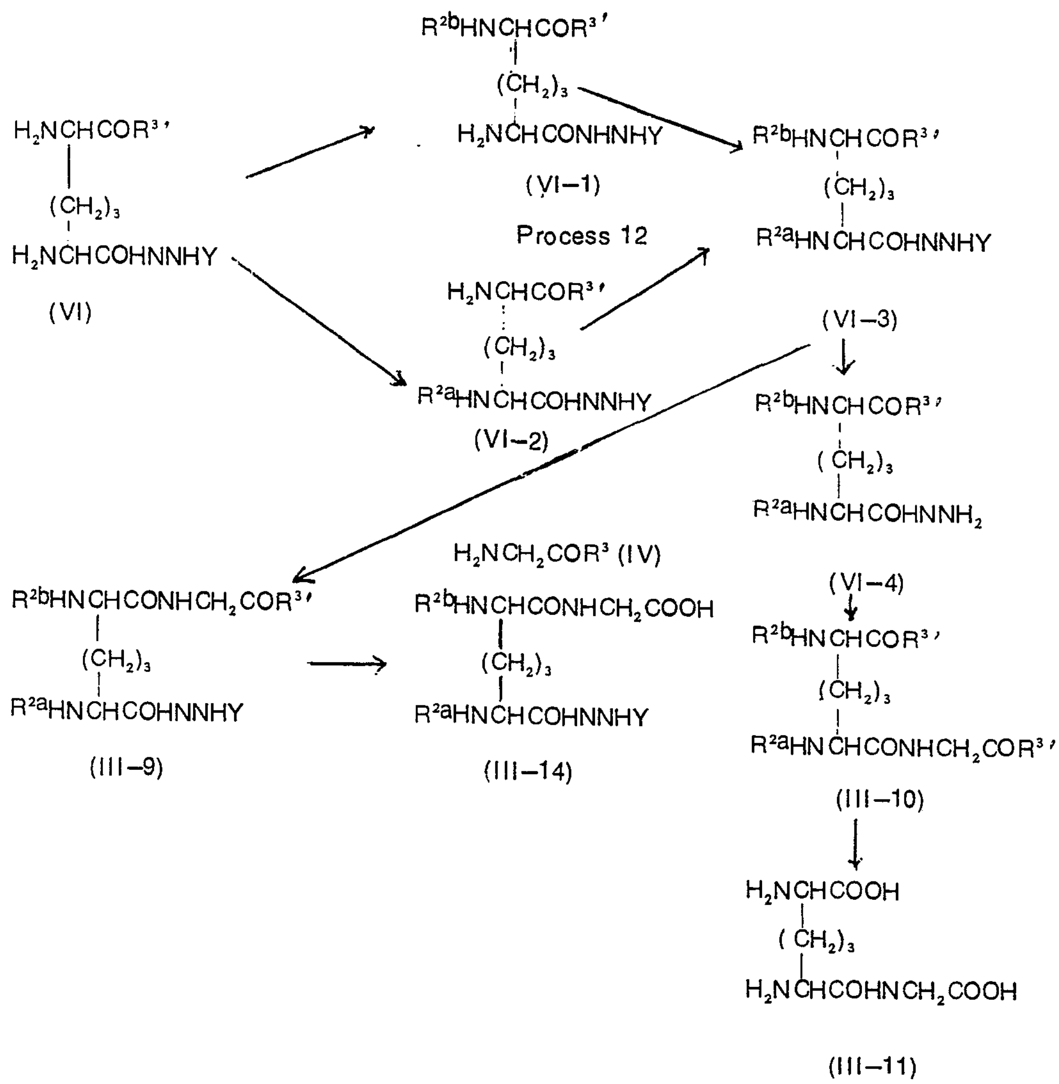

(VI) (VI–1) (VI–2) (VI–3) (VI–4) (III–9) (III–14) (III–10) (III–11)

In the above formulae, $R^{2b}$ is an amino protective group, and $R^{2a}$, $R^1$, $R^3$, $R^{3'}$ and $R^4$ are each as defined above, and $R^{3'}$ is a protective hydroxy.

Particulars of the definition for $R^{2b}$ and $R^{3'}$ and preferred example thereof are the same as those for $R^{2a}$ and $R^3$.

The lactyl tetrapeptide (I) and its pharmaceutically acceptable salts of this invention have been found to possess enhancing activities of immune response (i.e. enhancing activities of cellular immunity and humoral antibody production) and reticuloendotherial system, enhancing activity of blood stream clearance of carbon, inducing activity of interferon, mitogenic activity, protective efficacy in experimental infection, and anticancer activity.

Accordingly, the lactyl tetrapeptide (I) and its pharmaceutically acceptable salts are useful for the therapeutic treatment of infectious diseases caused by pathogenic microorganism, especially gram-negative bacteria and gram-positive bacteria and fungi, and of cancer in human being and animals.

Further, Compounds (II) and (III) are useful as intermediate for preparing Compound (I) having biologically active properties as mentioned above.

For the purpose of showing pharmaceutical utility of the lactyl tetrapeptide (I), pharmacological test data thereof are illustrated in the following.

In the following tests, test compounds are represented in the following formula:

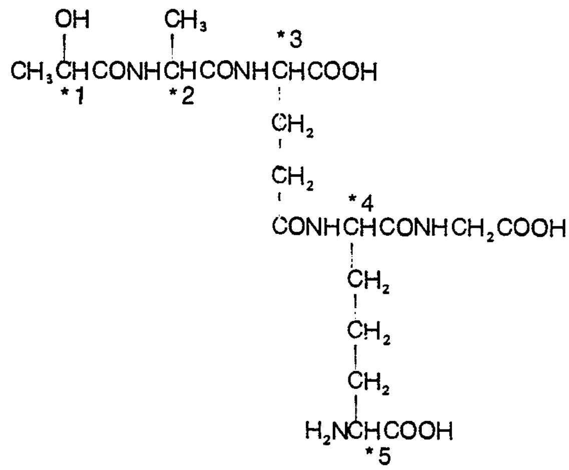

1. ENHANCING ACTIVITIES OF CELLULAR IMMUNITY AND HUMORAL ANTIBODY PRODUCTION

Guinea pigs (groups of five) were given 0.1 ml of FIA (Freund's Incomplete Adjuvent) emulsion containing 500 μg of ovalbumin in both posterior footpads. Control groups received antigen in FIA only, whereas the test groups received the antigen with test compound in FIA. The animals were skin-tested on day 14 and bled on day 16.

The results are as the following table 1.

TABLE 1

| Test Compound *1 *2 *3 *4 *5 | Dose (μg/ site) | Cellular immunity *1 skin reaction (mm diameter, M±S.E. | Humoral Hemagglutination titer (M±S.E. $log_2$)*2 | immunity hemolysin titer (M±S.E. $log_2$)*2 |
|---|---|---|---|---|
| D L D L D | 0 | 0 | 9.1±0.19 | 4.5±0.45 |
| | 0.1 | 8.2±2.8 *3 | 9.9±0.10 *3 | 6.4±0.76 |
| | 1 | 14.5±2.1 *3 | 11.5±0.61 *3 | 7.7±0.64 *3 |
| | 10 | 11.0±1.3 *3 | 12,2±0.56 *3 | 7.1±0.58 |
| | 100 | 4.2±1.7 *3 | 10.0±1.01 | 5.9±0.89 |
| D L L L D | 0 | 0.9±0.9 | 8.5±0.4 | — |
| | 1 | 2.4±1.5 | 10.4±0.5 *3 | — |
| | 10 | 7.9±2.1 *3 | 11.0±0 *3 | — |
| D L D DL (*4 *5) | 0 | 0 | 9.2±0.3 | 5.9±0.1 |
| | 1 | 4.3±1.8 *3 | 10.2±1.1 *3 | 6.5±0.4 |
| | 10 | 9.0±3.2 *3 | 10.4±0.3 *3 | 7.5±1.2 *3 |
| | 100 | 6.4±1.7 *3 | 10.9±0.2 *3 | 6.7±1.2 *3 |
| L L D L D | 0 | 0 | 6.8±0.3 | — |
| | 1 | 6.7±0.8 *3 | 8.2±0.1 *3 | — |
| | 10 | 10.2±1.6 *3 | 7.6±0.5 *3 | — |

Footnotes to Table 1

Note: *1: The skin test was performed on the back by intradermal injection of 5 $\mu$g of antigen dissolved in 0.1 ml of saline.
Skin reaction of the test site was measured at 48 hours.

*2: Antibody estimation was carried out as follows:
Ovalbumin-coated sheep red blood cells were prepared by chromium chloride.
Antibody titer was expressed as the reciprocal of the highest dilution of serum evoking threshold hemogglutination and hemolysin.
The results were converted to $\log_2$ unit.

*3: Significance was calculated by Student's t-test; $P<0.05$

2. MITOGENIC ACTIVITIES FOR MOUSE SPLEEN CELLS

[Materials and Methods]

(1) Animal:
Mice used for this experiment were male BALB/C strain, aged 13 weeks (Test 1) or female BALB/C strain, aged 9 weeks (Test 2).

(2) Tissue Culture Medium:
The tissue culture medium employed was a complete medium designated Roswell Park Memorial Institute (RPMI)—1640. All media employed contained 100 units/ml of penicillin G and 100 $\mu$g/ml of streptomycin sulfate and 10% fetal calf serum.

(3) Cell Preparation:
Spleens were removed under sterile conditions, and washed with Hanks solution and then teased in the tissue culture medium. The cells were suspended in the tissue culture medium to contain $8 \times 10^6$ cells/ml.

(4) Culture Conditions:
Into each hole of Microtest II tissue culture plate (8 × 12 hole) (maker: Falcon Plastics Co.) were poured 0.1 ml of the above cells suspension and 0.1 ml of the prescribed concentrate of the test compound as described below and then the cultures were incubated in triplicate at 37°C in a humidified atmosphere (95% air, 5% $CO_2$) for 48 hours.
The control culture contained 0.1 ml of the culture medium instead of the medium containing the test compound.

(5) [3H] Thymidine uptake:
In all tests, 20 $\mu$l of 10 micro-curine ($\mu$ Ci)/ml of tritiated thymidine (3H-thymidine) was added to each hole for the final 24 hours of culture. After the culture was completed, the resultant cells were filtered with a filter paper, Whatman GF83 and washed successively with saline and with 5% trichloroacetic acid. The filter paper was dried and placed in a scintillator (toluene 1 l containing 0.1 g of p-bis[5-phenyloxazoyl)-benzene and 4 g of 2,5-diphenyloxazoyl), and 3H-thymidine incorporated into DNA was measured.

(6) Stimulation Index:

$$\text{Stimulation Index} = \frac{\text{3H-thymidine uptake (net cpm) at treatment}}{\text{3H-thymidine uptake (net cpm) at control}}$$

TABLE 2

| Test Compound *1 | *2 | *3 | *4 | *5 | Test | Concentration (μg/ml) | 3H-thymidine uptake net cpm: av±S.E. | Stimulation Index |
|---|---|---|---|---|---|---|---|---|
| D | L | D | L | D | 1 | 100 | 2,028±89 | 4.9 |
| | | | | | | 10 | 1,486±120 | 3.6 |
| | | | | | | 1 | 835±64 | 2.0 |
| | | | | | | 0 | 417±13 | 1.0 |
| | | | | | 2 | 100 | 3,666±42 | 4.1 |
| | | | | | | 10 | 3,223±402 | 3.6 |
| | | | | | | 1 | 2,741±319 | 3.0 |
| | | | | | | 0 | 901±105 | 1.0 |
| D | L | D | DL | | 1 | 100 | 1,284±131 | 3.1 |
| | | | | | | 10 | 1,414±38 | 3.4 |
| | | | | | | 1 | 996±35 | 2.4 |
| | | | | | | 0 | 417±13 | 1.0 |
| L | L | D | L | D | 2 | 100 | 2,772±215 | 6.1 |
| | | | | | | 10 | 2,326±140 | 5.1 |
| | | | | | | 1 | 1,713±102 | 3.8 |
| | | | | | | 0 | 452±30 | 1.0 |
| D | L | D | L | L | 1 | 100 | 1,271±29 | 3.2 |
| | | | | | | 10 | 1,003±50 | 2.5 |
| | | | | | | 1 | 742±61 | 1.9 |
| | | | | | | 0 | 399±51 | 1.0 |

3. PROTECTIVE EFFICACY IN EXPERIMENTAL INFECTION IN MICE

(1) In determining the protective efficacy against experimental infections in mice, the FR-900156 substance was dissolved and diluted in sterile water to provide three-fold concentrations of drug for testing.

Male DDY-strain mice, aged 6 weeks and averaging 24—26 g in weight, were used in groups of four mice each.

Overnight cultures of Escherichia coli No. 22 in Difco Nutrient Broth was diluted to 1/100 in fresh medium and incubated at 30°C with shaking. When the cell density of 1 × $10^8$/ml was obtained, 0.2 ml of the culture was injected intraperitoneally. All the animals receiving the challenge and not treated with the drug died within 48 hours of the infection.

One-fifth ml of the FR-900156 substance solution was injected subcutaneously, 1, 4, 5, and 6 days before the infection.

Two days after infection, the test was considered complete and survival records of that day were made. The test results are shown in Table 3.

TABLE 3

| Dose (mg/mouse/day) | Survival/infected |
|---|---|
| 0.01 | 10/10 |
| 0.003 | 10/10 |
| 0.001 | 10/10 |
| 0.0003 | 8/10 |
| 0.0001 | 4/10 |
| Control | 0/10 |

(2) In determining the protective efficacy against experimental infections in mice, the test compound was dissolved in and diluted with sterile saline to provide prescribed concentrations of drug.

Male ICR-strain mice, aged 4 weeks were used in groups of ten mice. E. coli 22 was cultivated overnight at 37°C on Trypticase soy agar and then were suspended in a sterile saline to obtain microbial cell concentration of $1.6 \times 10^8$ CFU/ml. Mice were inoculated intraperitoneally with 0.2 ml of the suspension. Each of the test drugs was given intraperitoneally in various doses to a group of ten mice 24 hours before challenge.

Survival percent were found from the number of the surviving animals after three days of injection. Results are shown in Table 4.

TABLE 4

| Test Compound | | | | | Survival (%) | |
|---|---|---|---|---|---|---|
| *1 | *2 | *3 | *4 | *5 | Dose 10 mg/kg | Dose 1 mg/kg |
| D | L | L | L | D | — | 50.0 |
| L | L | D | L | D | 87.5 | 62.5 |
| D | D | L | L | D | 100 | 87.5 |
| D | D | D | L | D | 75.0 | 75.0 |

4. ANTICANCER ACTIVITY

Female rats of Donru Strain, aged 6 weeks, were used in groups of three rats each.

Suspension of ascites hepatoma AH 66 in 0.5 ml of Hank's solution was transplanted intraperitoneally ($5 \times 10^4$ cells/rat). About 13 days later, all the control animals died of ascites. Prolongation of survival time in comparison with the controls is the criterion of effectiveness.

Therapy was given 5, 6, 7, 8, 9, days before tumor transplantation. The FR-900156 substance was dissolved and diluted in sterile saline water to provide three-fold dilutions for testing and the said sterile saline solution of the FR-900156 substance was given intraperitoneally to the animals. The test results are shown in Table 5.

TABLE 5

| Dose (mg/rat/day) | Life span (days) | | |
|---|---|---|---|
| 1.0 | 14, | >30, | >30 |
| 0.3 | 13, | >30, | >30 |
| 0.1 | 13, | >30, | >30 |
| 0.03 | 13, | 13, | 13 |
| Control (saline) | 13, | 13, | 13 |

5. BLOOD STREAM CLEARANCE OF CARBON

Reagents:

1. Carbon suspension. Rotring drawing ink (170 mg carbon/ml) was diluted to 1/5 of the original concentration in saline containing 1% gelatin.
2. 0.1% aqueous sodium carbonate solution.

Procedure:

Mice (DDY male 5—6 W) were injected via the tail vein with a dose of 0.01 ml/g body weight of carbon. Blood samples were taken by means of a pointed capillary pippet calibrated to hold a 50 $\mu$l and previously washed in heparin. This was plunged in the retroorbital venous sinus at the nasal angle of the eye. The samples were removed at 3 and 6 min. The blood was immediately discharged into 3.0 ml of the sodium carbonate solution. This hemolyzed the blood and allowed the quantitation of carbon. The samples were then read in a spectrophotometer at 660 nm, the log concentration being obtained from a previously determined standard curve. The clearance value K may be determined by plotting log carbon concentration against time according to the following relationship;

$$K = \frac{(\log C_1 - \log C_2)}{T_2 - T_1}$$

in which $T_1$ and $T_2$ represent the time in min when the same were withdrawn and $C_1$ and $C_2$ represent the concentrations of carbon in the blood at the time $T_1$ and $T_2$, respectively.

(1) EXAMINATION OF EFFECT OF THE FR-900156 SUBSTANCE ON CARBON CLEARANCE

The aqueous solution of the drug as given was administered subcutaneously to mice. Twenty-four hours later, blood stream clearance of carbon was measured. K value obtained with treated mice was compared with that of control mice. The test results are shown in Table 6.

EFFECT OF VARIOUS DRUGS ON CARBON CLEARANCE

TABLE 6

| Drug | Dose (mg/mouse) | $K_{treated}/K_{control}$ |
|---|---|---|
| Krestin | 10 | 1.1 |
| | 1 | 1.0 |
| Levamisol | 10 | — (death) |
| | 1 | 0.5 |
| Tuftsin | 400 | 1.4 |
| | 125 | 1.0 |
| FR-900156 substance | 0.25 | 2.9 |
| | 0.06 | 1.5 |
| Control (saline) | | 1.0 |

(2) EXAMINATION OF EFFECT OF THE LACTYL TETRAPEPTIDE ON CARBON CLEARANCE

The aqueous solution of the test compound as given was administered subcutaneously to mice. Twenty-four hours later, blood stream clearance of carbon was measured. K value obtained with treated mice was compared with that of control mice. The test results are shown in Table 7.

TABLE 7

| Test Compound *1 | *2 | *3 | *4 | *5 | Dose (mg/kg | $K_{treated}/K_{control}$ | Reference Compound FR-900156 substance |
|---|---|---|---|---|---|---|---|
| D | L | L | L | D | 100 | 2.0 | — |
| | | | | | 1 | 0.9 | 1.9 |
| D | D | L | L | D | 100 | 1.7 | — |
| | | | | | | 1 | 1.6 |
| D | L | D | L | L | 100 | 2.7 | — |
| | | | | | 1 | 0.9 | 2.5 |
| L | L | D | L | D | 100 | 2.6 | — |
| | | | | | 1 | 2.4 | 2.2 |
| D | D | D | L | D | 1 | 2.2 | 1.8 |
| | | | | | 0.1 | 1.9 | 1.2 |

6. INDUCING ACTIVITY OF INTERFERON

Male ICR strain mice aged 4 to 6 weeks were used in these experiments. Spleens of these mice were removed and the capacity of spleen cells to produce interferon in vitro by the treatment of test compound was tested.

Single spleen cell suspensions were prepared in medium RPMI—1640 supplement with streptomycin (100 γ/ml), penicillin G (100 γ/ml), 1% glutamine and 2-mercaptoethanol ($5 \times 10^{-5}$ M).

These were cultured at 37°C at a concentration of $4 \times 10^7$ cells/ml with or without test compound dissolved with culture medium (dose: 100, 10, 1 μg/ml). After 24 hours, supernatants of these cultures were collected and those interferon activities were titrated by cytopathic effect (CPE) inhibition test in L cell-VSV system. Antiviral titer was expressed in IU/ml base on standard interferon.

The results are as the following table 8.

TABLE 8

| Test Compound *1 | *2 | *3 | *4 | *5 | Dose (μg/ml) | IF Titer (IU/ml) |
|---|---|---|---|---|---|---|
| D | L | D | L | D | 100 | 14 |
| | | | | | 10 | 16 |
| | | | | | 1 | 10 |
| | | | | | 0 | <6.5 |
| D | L | D | DL | | 100 | 62 |
| | | | | | 10 | 40 |
| | | | | | 1 | 39 |
| | | | | | 0 | 7.5 |

TABLE 8 (continued)

| Test Compound *1 | *2 | *3 | *4 | *5 | Dose (μg/ml) | IF Titer (IU/ml) |
|---|---|---|---|---|---|---|
| D | D | D | L | D | 100 | 29 |
| | | | | | 10 | 23 |
| | | | | | 1 | 11 |
| | | | | | 0 | <11 |
| D | D | L | L | D | 100 | 38 |
| | | | | | 10 | 11 |
| | | | | | 1 | <11 |
| | | | | | 0 | <11 |

7. ACUTE TOXICITY IN MICE
   1 g/kg (i.v.): no toxicity

8. CELL TOXICITY (mouse L cell)
   500 μg/ml: no toxic effect

The pharmaceutical composition of this invention can be used in the form of a pharmaceutical preparation, for example, in solid, semisolid or liquid form, which contains an active substance of this invention in admixture with an organic or inorganic carrier or excipient suitable for external, enteral or parenteral applications. The active ingredient may be compounded, for example, with the usual non-toxic, pharmaceutically acceptable carriers for tablets, pellets, capsules, suppositories, solutions, emulsions, suspensions, and any other form suitable for use. The carriers which can be used are water, glucose, lactose, gum acacia, gelatin, mannitol, starch paste, magnesium trisilicate, talc, corn starch, keratin, colloidal silica, potato starch, urea and other carriers suitable for use in manufacturing preparations, in solid, semisolid, or liquid form, and in addition auxiliary, stabilizing, thickening and coloring agents and perfumes may be used. The pharmaceutical compositions can also contain preservative or bacteriostatic agents to keep the active ingredient in the desired preparations stable in activity. The active object compound is included in the pharmaceutical composition in an amount sufficient to produce the desired therapeutic effect upon the process or condition of diseases.

For applying this composition to humans, it is preferably to apply it by intravenous, intramuscular or oral administration. While the dosage or therapeutically effective amount of the object compound of this invention varies from and also depends upon the age and condition of each individual patient to be treated, a daily dose of about 2—100 mg of the active ingredient/kg of a human being or an animal is generally given for treating diseases, and an average single dose of about 50 mg, 100 mg, 250 mg, and 500 mg is generally administered.

The following examples are given for purpose of illustrating this invention.

In the following examples, starting compounds and object compounds are expressed using the following abbreviations:

| | | |
|---|---|---|
| Lac | : | Lactyl |
| Ala | : | Alanyl |
| Glu | : | Glutamyl |
| Gly | : | Glycyl |
| DAP | : | $\alpha,\beta$-Diaminopimelyl |
| Z | : | benzyloxycarbonyl |
| Boc | : | t-butoxycarbonyl |

| | | |
|---|---|---|
| Bzl | : | benzyl |
| Me | : | methyl |
| Et | : | ethyl |
| Su | : | N-hydroxysuccinimide |
| Bzh | : | benzhydryl |

Preparation 1

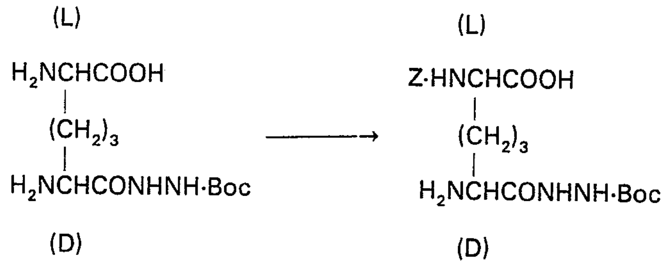

(1) (2)

meso-DAP(D)-NHNH·Boc (1) (5.4 g) and cupric chloride (dihydrate) (1.52 g) were dissolved in a mixture of 0.5N aqueous sodium hydroxide (60 ml). The solution was stirred for thirty minutes at ambient temperature.

The solution was cooled to 0°C and benzyloxycarbonyl chloride (4.6 g) was added dropwise thereto in the course of twenty minutes, maintaining the pH over 11.

The stirring was continued for an additional one hour at the same temperature. After the reaction was completed, the solution was adjusted to pH 7 with 5% aqueous hydrochloric acid and then hydrogen sulfide gas was passed through the solution for about fifteen minutes. To the reaction mixture was added ethyl acetate (50 ml) and the mixture was adjusted to pH 4 with 5% aqueous hydrochloric acid. The ethyl acetate layer and the aqueous layer were divided out, and the ethyl acetate layer was extracted four times with water (30 ml). All of the aqueous layers were combined and concentrated to 40 ml under reduced pressure to give crystals. The crystals were collected by filtration, washed with water to give Z-(L)meso-DAP(D)-NHNH·Boc (2) (3.5 g). The mother liquor and the washings were collected and extracted twice with n-butanol (40 ml). The extracts were combined and concentrated under reduced pressure to give a residue.

The residue was triturated with ether and collected by filtration to give additional Z-(L)meso-DAP(D)-NHNH.Boc (2) (1.2 g) as a crude solid, which was recrystallized from water. mp 194—196 (dec.).

N.M.R. ($CD_3OD$), $\delta$ (ppm):
1.67 (9H, s), 1.6—2.1 (6H, m), 3.6—4.2 (2H, m), 5.12 (2H, s), 7.43 (5H, s)
$[\alpha]_D = -20.8°$ (C: 0.5 methanol)

Preparation 2

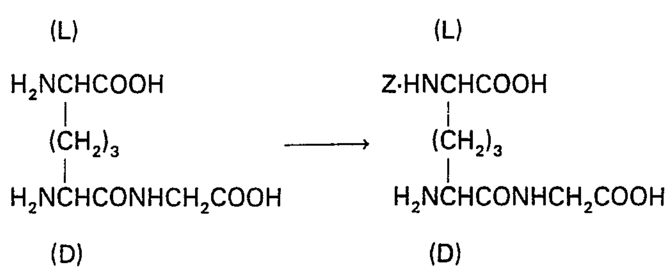

(1) (2)

3N Aqueous sodium hydroxide (6.0 ml) was added to a solution of meso-DAP-(D)-GlyOH (1) (1.85 g) and cupric chloride (dihydrate) (1.28 g) in water (190 ml) at 5°C.

To the solution was added benzyloxycarbonyl chloride (5.4 ml), and the resulting solution was

stirred at 5°C, maintaining the pH 11—12 with 3N aqueous sodium hydroxide. The stirring was continued for 4.5 hours and an additional benzyloxycarbonyl chloride (3.2 ml.) was added to the reaction mixture. The mixture was stirred at 5°C for six hours, maintaining the pH 11—12 with 3N aqueous sodium hydroxide.

The reaction mixture was acidified to pH 2 with 3N aqueous hydrochloric acid and washed twice with ether.

The separated aqueous layer was adjusted to pH 4.5 with 3N aqueous sodium hydroxide and buffled with hydrogen sulfide and then filtered. The filtrate was concentrated to about 100 ml and the concentrate was allowed to stand in a refrigerator to give crystals.

The crystals were collected by filtration and washed successively with water, methanol and ether to give Z-(L)-meso-DAP-(D)-GlyOH (2) (230 mg). mp. 161.5—162.0 (dec.).

N.M.R. ($CD_3OD$—$D_2O$) $\delta$ (ppm): 1.2—2.2 (6H, m), 3.7—4.3 (4H, m), 5.10 (2H, s), 7.39 (5H, s)
$[\alpha]_D = -25.3°$ (C: 0.5 methanol).

The filtrate was concentrated and chromatographed on a macroporous non-ionic adsorption resin, HP20 (trade mark, maker: Mitsubishi Chemical Industry Co., Ltd.) (125 ml). After washing with water, elution was carried out with 50% aqueous methanol and then 80% aqueous methanol. The eluates were combined and evaporated to dryness and the crude crystals thus obtained was dissolved in hot methanol (80 ml) and filtered. To the filtrate was added water (15 ml) and the solution was concentrated to give crude crystals. The crude crystals were washed successively with isopropanol ether and ether to give Z-(L)-meso-DAP-(D)-GlyOH (2) (2.22 g). The same object compound (2) (165 mg) was additionally obtained from the mother liquor.

Preparation 3

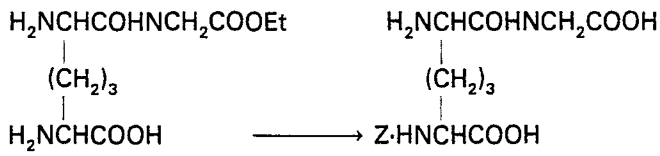

(meso) (meso)

(1) (2)

meso DAP(OH)GlyOEt (1) (4.10 g) was dissolved in water (30 ml) and cupric chloride (dihydrates) (2.08 g) was added to the solution. The mixture was stirred at 5—7°C for five hours, maintaining pH 11 with 2% aqueous sodium hydroxide.

To the reaction mixture was added benzyloxycarbonyl chloride (10.0 g) and the resulting mixture was stirred at 5—7°C for five hours, maintaining pH 11. An additional benzyloxycarbonyl chloride (4.0 g) was added to the reaction mixture. The resulting mixture was stirred for four hours and adjusted to pH 1 with dil. hydrochloric acid and washed with ethyl acetate.

The aqueous layer was adjusted to pH 7 and hydrogen sulfide gas was passed through the solution. The precipitates were filtered off and the filtrate was concentrated. The concentrate was adjusted to pH 3.2—3.4 and passed through a column packed with a microporous non-ionic adsorption resin, HP20 (180 ml).

Elution was carried out with 50% aqueous methanol and the eluate was evaporated to dryness to give Z-meso-DAP(OH)GlyOH (2) (4.3 g) as a solid.

N.M.R. ($D_2O$+$NaHCO_3$) $\delta$ (ppm): 1.38—1.88 (6H, m), 3.75 (2H, s), 3.79 (1H, t, J=4Hz), 3.96 (1H, t, J=4Hz), 5.11 (2H, s), 7.44 (5H, s).

Preparation 4

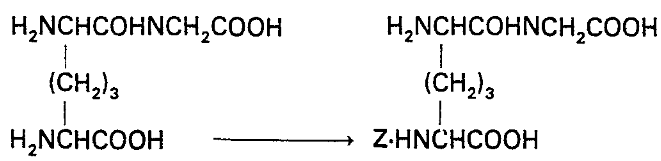

(meso+DL) (meso+DL)

(1) (2)

Cupric carbonate (1.173 g) was added to a solution of DAP(OH)GlyOH (1) (1.7 g) in water (50 ml) and the mixture was refluxed for two hours. The reaction mixture was filtered and the filtrate was adjusted to pH 11.5 with 2N aqueous sodium hydroxide. Benzyloxycarbonyl chloride (3.23 ml) was added to the reaction mixture at 0°C and the resulting mixture was stirred at 0°C for six hours, maintaining pH 11.5. The reaction mixture was neutralized with dil. aqueous hydrochloric acid and dechelated by hydrogen sulfide gas. The resulting mixture was filtered and the filtrate was chromatographed on a macroporous non-ionic adsorption resin, HP20 (100 ml). Elution was carried out with 80% aqueous methanol and the eluate was evaporated to dryness to give Z-DAP(OH)GlyOH (2) (1.4 g) as a solid.

N.M.R. ($CD_3OD$) $\delta$ (ppm): 1.38—1.88 (6H, m), 3.75 (2H, s), 3.79 (1H, t, J=4Hz), 3.96 (1H, t, J=4Hz), 5.11 (2H, s), 7.44 (5H, s).

Preparation 5

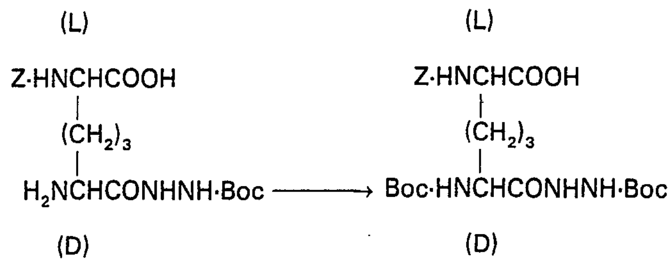

(1) (2)

(1) Preparation 5—1

Z-(L)meso-Dap(D)-NHNHBoc (1) (5.0 g), t-butylcarbonic anhydride (3.0 g) and triethylamine (2.54 g) in a mixture of water (50 ml) and dioxane (50 ml) were stirred for three hours at ambient temperature. Dioxane was distilled off under reduced pressure and the resulting aqueous layer was washed with ether. The aqueous layer was adjusted to pH 2 with 5% aqueous hydrochloric acid and extracted with ethyl acetate. The extract was washed with saturated aqueous sodium chloride and then dried over anhydrous magnesium sulfate to give Z-(L)-Boc(D)meso-DAP(D)NHNHBoc (2) (6.0 g), which was recrystallized from isopropyl ether. mp 146—148 (dec).

N.M.R. ($CDCl_3$), $\delta$ (ppm): 1.4—2.1 (6H, m), 1.67 (9H, s), 4.1—4.5 (2H, m), 5.13 (2H, s), 5.50 (1H, broad s), 6.0 (1H, broad s), 7.05 (1H, broad s), 7.35 (5H, s), 8.37 (1H, broad s), 8.90 (1H, broad s)

(2) Preparation 5—2

Z-(L)-Boc(D)meso-DAP(D)-NHNHBoc (2) (0.50 g) was obtained from Z-(L)meso-DAP(D)-NHNHBoc (1) (438 mg) by substantially the same manner as that of Example 5—1 provided that t-butyl S-4,6-dimethylpyrimidine-2-ylthiocarbonate (288 mg) was employed in place of t-butylcarbonic anhydride.

Preparation 6

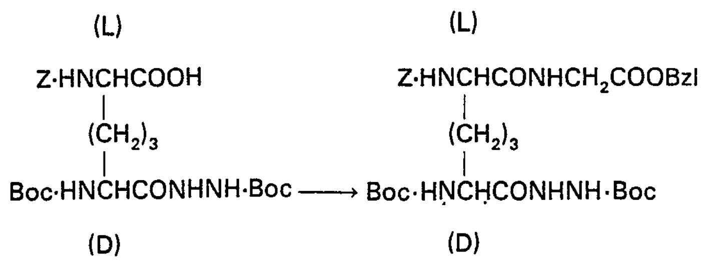

(1) (2)

Z-(L)-Boc(D)meso-DAP(D)-NHNHBoc (1) (10.8 g) and N-methyl-morpholine (2.02 g) were dissolved in methylene chloride (110 ml) and stirred at −10—−15°C. Isobutyl chlorocarbonate (2.73 g) was added dropwise to the solution and the mixture was stirred for thirty minutes at ambient temperature. To the solution was added dropwise a solution of glycine benzyl ester p-toluenesulfonate (6.75 g) and N-methyl morpholine (2.02 g) in methylene chloride (110 ml).

The solution was stirred for two hours at −10—−15°C and for an hour at ambient temperature. Methylene chloride was distilled off under reduced pressure and the residue was dissolved into a mixture of ethyl acetate (150 ml) and 1% aqueous hydrochloric acid (60 ml). The ethyl acetate layer was separated and washed successively with water 2% aqueous sodium bicarbonate and aqueous sodium chloride in turn. The ethyl acetate layer was dried over anhydrous magnesium sulfate and evaporated to dryness under reduced pressure. The residue thus obtained was recrystallized from ether to give Z-(L)-Boc(D)-meso-DAP(I)GlyoBzl-(D)-NHNHBoc. (2) (12.4 g), mp. 85—87.

N.M.R. ($CDCl_3$), δ (ppm): 1.43 (18H, s), 1.5—2.2 (6H, m), 4.10 (2H, d, J=6Hz), 4.1—4.5 (2H, m), 5.10 (2H, s), 5.17 (2H, s) 5.40 (1H, d, J=8Hz), 5.90 (1H, d, J=8Hz), 6.73 (1H, broad s), 7.33 (10H, s), 7.73 (1H, broad s), 8.4—8.6 (1H, m).

Preparation 7

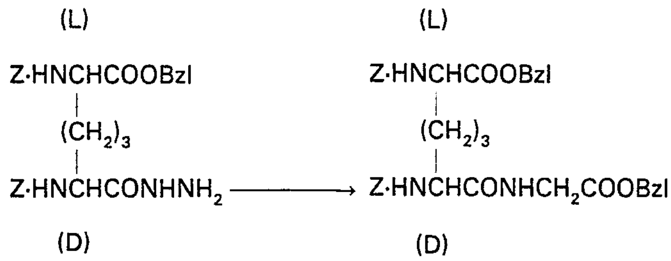

(1) (2)

To a solution of di-Z-meso-DAP(L)oBzl-(D)-$NHNH_2$ trifluoroacetic acid salt (1) (6.435 g) in dimethylformamide (25 ml) was added 3.29N hydrochloric acid in dimethylformamide (5.80 ml) at −50°C and then isoanylnitrite (1.41 ml) was added. The solution was allowed to warm to −20°C and then stirred for ten minutes. The resulting solution was cooled to −50°C and triethylamine (3.97 ml) was added thereto.

To the solution was added a mixture of glycine benzyl ester p.toluensulfonate (6.41 g) and triethylamine (7.95 ml) in dimethylformamide (12 ml). The heterogeneous mixture was stirred at 3—7°C for six hours and allowed to stand in a refrigerator for 48 hours. The reaction mixture was diluted with a mixture of ethyl acetate (170 ml) and methylene chloride (30 ml) and washed successively with 5% aqueous phosphoric acid, water dil. sodium bicarbonate, water, and brine. The mixture was dried over anhydrous magnesium sulfate and the solvent was evaporated to dryness to give crystalline materials (6.4 g). The crystalline materials were chromatographed on silica gel (90 g) and eluted with a mixture of methylene chloride and ethyl acetate (3:1). The eluate was evaporated to give a residue. Crystallization was carried out from ethyl acetate to give crude crystals of di-Z-meso-DAP(L)oBzl-(D)-GlyoBzl (2) (5.32 g). mp 131—133°C.

An aliquote (450 mg) of the above crystals were further purified by chromatography on silica gel (eluted with a mixture of methylene chloride and ethyl acetate) (1:3), followed by recrystallization from a mixture of ethyl acetate and ether (5:2) to give fine needles. mp 138—139.5°C.

$[\alpha]_D = +4.6°$ (C=1, chloroform)

N.M.R. ($CDCl_3$), δ (ppm): 1.1—2.1 (6H, m), 3.98 (2H, d, J=6Hz), 3.8—4.5 (2H, m), 5.04 (4H, s), 5.08 (2H, s), 5.10 (2H, s), 5.49 (2H, t, J=7Hz), 6.64 (1H, t, J=6Hz), 7.28 (20H, s).

Preparation 8

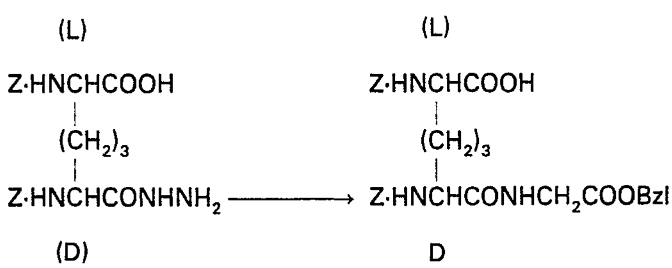

(1) (2)

A solution of sodium nitrite (0.97 g) in water (10 ml) was added to a two-phase solution of di-Z-meso-DAP(D)-$NHNH_2$ (6.50 g) in a mixture of 1N aqueous hydrochloric acid (42 ml) and ethyl acetate (30 ml) at 0°C. The resulting mixture was stirred at 0°C for five minutes and a cooled ethyl acetate (30 ml) was added thereto.

The ethyl acetate layer was collected and washed four times with dil. sodium chloride, dried over anhydrous magnesium sulfate and then filtered. To the filtrate was added a solution of H-GlyoBzl in methylene chloride (30 ml), which was prepared by desalting glycine benzyl ester p-toluenesulfonate (11.6 g) with potassium carbonate in a mixture of methylene chloride and water in a conventional manner, and then triethylamine (1.92 ml) was added. The resulting mixture was kept at 4°C for 20 hours. The reaction mixture was washed successively with dil. hydrochloric acid and (three times), brine, dried over anhydrous magnesium sulfate and evaporated to dryness to give an oil (6.5 g), which was chromatographed on silica gel (45 g).

Elution was carried out with a mixture of methylene chloride and methanol (100:5) to give amorphous solid (5.15 g). The solid was crystallized from a mixture of ethyl acetate and ether (1:2) to give di-Z-meso-DAP(D)GlyoBzl (2) (1.54 g).

N.M.R. (acetone-$d_6$), $\delta$ (ppm): 1.3—2.4 (6H, m), 3.9—4.6 (2H, m), 4.06 (2H, d, J=6Hz), 5.12 (4H, s), 5.18 (2H, s), 6.3—6.9 (2H, broad d, J=8Hz), 7.38 (15H, s), 7.77 (1H, t, J=6Hz)

Preparation 9

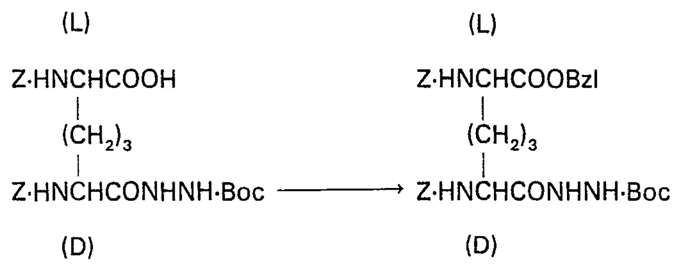

(1) (2)

A mixture of di-Z-meso-DAP(D)-NHNHBoc (1) (8.83 g), benzyl bromide (3.66 ml) and triethylamine (4.30 ml) in dimethylformamide (40 ml) was stirred at ambient temperature for 19 hours. The reaction mixture was diluted with ethyl acetate (150 ml) and the solution was washed successively with dil. phosphoric acid, water, dil. sodium bicarbonate, water and brine.

The solution was dried over anhydrous magnesium sulfate and evaporated to give an amorphous solid (9.0 g), which was purified by column chromatography using silica gel (90 g), and fractions eluted with a mixture of methylene chloride and ethyl acetate (1:3) were combined and evaporated to dryness to give a solid (8.65 g). Recrystallization from a mixture of ether and hexane gave di-Z-meso-DAP(L)oBzl-(D)-NHNHBoc (2) (7.40 g).

N.M.R. ($CDCl_3$), $\delta$ (ppm): 1.40 (9H, s), 1.1—2.0 (6H, m), 4.0—4.7 (2H, m), 5.17 (4H, s), 5.23 (2H, s), 6.87 (1H, broad s), 7.48 (15H, s), 8.57 (1H, broad s)
$[\alpha]_D^{23} = +13.2°$ (C=1, chloroform).

Preparation 10

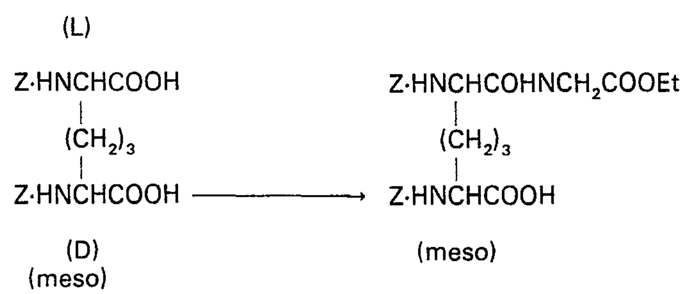

(1) (2)

To a cold mixture of di-Z-(D)meso-DAP (1) (13.8 g) and triethylamine (6.1 g) in methylene chloride (150 ml) was added dropwise in the course of five minutes a cold solution of isobutoxycarbonyl chloride (5.33 g) in methylene chloride (30 ml) at −10°C.

The mixture was stirred at −10°C—−12°C for twenty five minutes. To the mixture was added dropwise a solution of glycine ethyl ester hydrochloride (5.44 g) and triethylamine (3.94 g) in methylene chloride (200 ml) at −10°C ~ −12°C in the course of twenty minutes. The solution was stirred at −10°C for thirty minutes. To the reaction mixture was added water and the mixture was adjusted to pH 2 with 1N aqueous hydrochloric acid, and then washed with saturated aqueous sodium chloride and with 5% aqueous sodium bicarbonate. The methylene chloride layer was washed with water and then concentrated. To the concentrate was added ethyl acetate and insoluble materials were filtered off and the filtrate was extracted three times with 5% aqueous sodium bicarbonate (pH 8). The extract was adjusted to pH 2 with 1N aqueous hydrochloric acid and then extracted three times with ethyl acetate. The extract was washed twice with water and dried over anhydrous magnesium sulfate. The extract was filtered and the filtrate was concentrated. The oily residue thus obtained was allowed to stand in a refrigerator to give crystals. The crystals was washed with isopropyl ether and dried over anhydrous magnesium sulfate to give di-Z-meso-DAP(OH)GlyOEt (7.73 g).

N.M.R. (DMSO-$d_6$), δ (ppm): 1.20 (3H, t, J=7Hz), 1.0—1.9 (6H, m), 3.7—4.2 (2H, m), 3.80 (2H, d, J=6Hz), 4.03 (2H, q, J=7Hz), 5.00 (4H, s), 7.33 (10H, s).

Preparation 11

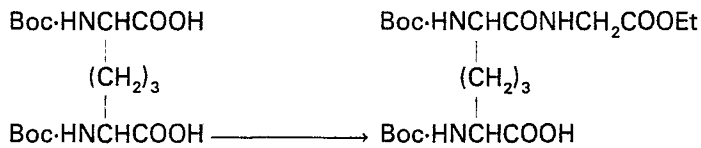

(meso+DL) (meso+DL)

(1) (2)

A mixture of di-Boc-meso-DAP di-dicyclohexylamine salt (1) (18.5 g) in methylene chloride (160 ml) was added to a solution of triethylamine hydrochloride (8.0 g) in methylene chloride (200 ml) and the mixture was stirred at ambient temperature for two hours and cooled to −10°C. To the cold mixture was added dropwise a solution of ethoxycarbonyl chloride (3.46 g) in methylene chloride (10 ml), maintaining the temperature at −10°C. The solution was stirred at −10°C for thirty minutes. To the reaction mixture was added dropwise a mixture of glycinethyl ester hydrochloride (4.45 g) and triethylamine (3.23 g) in methylene chloride (200 ml) in the course of thirty minutes, maintaining the temperature at −10°C.

The mixture was stirred at the same temperature for thirty minutes. The reaction mixture was adjusted to pH 2 with 1N aqueous hydrochloric acid and then washed twice with water and twice with saturated aqueous sodium chloride. The organic layer was concentrated and the concentrate was dissolved in ethyl acetate. Extraction was carried out twice with 5% aqueous sodium bicarbonate. The extract was adjusted to pH 2 with 1N aqueous hydrochloric acid.

Extraction was carried out with ethyl acetate and the organic layer was washed successively with saturated aqueous sodium chloride, 5% aqueous sodium bicarbonate, and further washed with saturated aqueous sodium chloride. The organic layer was dried over anhydrous magnesium sulfate and filtered. The filtrate was concentrated to give an oily di-Boc-meso-DAP(OH)GlyOEt (2) (5.16 g).

N.M.R. ($CDCl_3$), δ (ppm): 1.30 (3H, t, J=7Hz), 1.43 (18H, s), 1.1—2.1 (6H, m), 4.07 (2H, t, J=6Hz), 3.8—4.5 (2H, m), 4.15 (2H, q, J=7Hz).

Preparation 12

(L) (L)

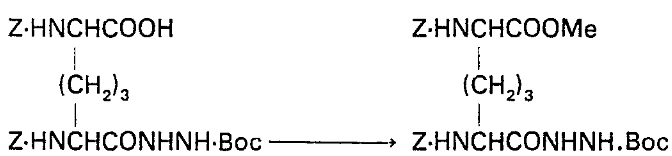

(D) (D)

(1) (2)

A solution of diazomethane (1 g/100 ml, 6 ml) was added to a solution of di-Z-meso-DAP-(D)-NHNHBoc (1) (0.62 g) in ether (15 ml) at 0°C until the solution became yellow. The reaction mixture was evaporated to dryness and the residue was chromatographed on silica gel (14 g) and elution was carried out with a mixture of methylene chloride and ethyl acetate (5:3) to give di-Z-meso-DAP(L)oMe-(D)NHNHBoc.

N.M.R. ($CDCl_3$), $\delta$ (ppm): 1.2—2.2 (6H, m), 1.33 (9H, s), 3.73 (3H, s), 4.1—4.6 (2H, m), 5.12 (4H, s), 5.74 (1H, d, J=8Hz), 5.78 (1H, d, J=8Hz), 6.68 (1H, broad s), 7.35 (10H, s), 8.50 (1H, broad s)
$[\alpha]_D^{23} = +12.7°C$ (C=1, ethanol)

Preparation 13

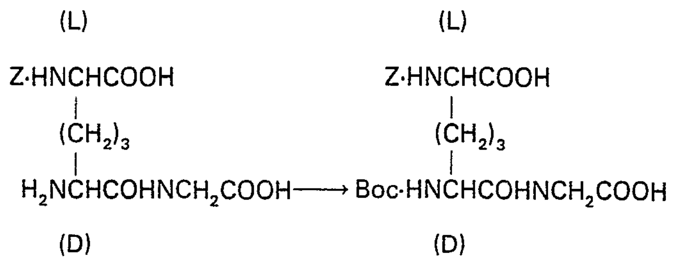

(1) (2)

To a suspension of Z-(L)-meso-DAP-(D)-GlyOH (1) (2.072 g) in water (20 ml) was added triethylamine (2.90 ml) and the mixture was stirred at 0°C for a few minutes. A solution of t-butylcarbonic anhydride (1.54 g) in dioxane (20 ml) was added to the resulting solution. The solution was stirred at ambient temperature for 6.5 hours and concentrated. The concentrate was diluted with water (50 ml) and ethyl acetate (100 ml) was added thereto. The mixture was cooled to 0°C and acidified to pH 2 with 1N aqueous hydrochloric acid.

The organic layer was separated and washed with water and brine, dried over magnesium sulfate and then evaporated to give Z-(L)-Boc(D)meso-DAP(D)GlyOH (2) (2.88 g) as an amorphous solid.

N.M.R. (DMSO-$d_6$), $\delta$ (ppm): 1.37 (9H, s), 1.1—1.9 (6H, m), 3.6—4.2 (2H, m), 3.73 (2H, d, J=5.5Hz), 5.02 (2H, s), 6.75 (1H, m), 7.33 (5H, s), 7.50 (1H, broad d, J=9Hz), 8.07 (1H, broad t, J=5.5Hz), 12.6 (2H, m).

Preparation 14

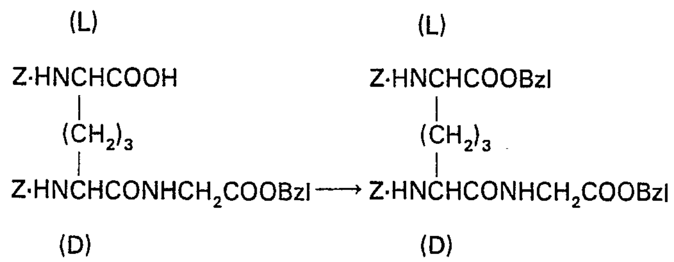

(1) (2)

Triethylamine (0.84 ml) was added to a solution of di-Z-meso-DAP-(D)-GlyoBzl (1) (3.0 g) in dimethylformamide (15 ml) at 0°C and then benzyl bromide (0.72 ml) was added thereto. The mixture was stirred at ambient temperature for 7.5 hours and triethylamine (0.42 ml) and benzyl bromide (0.36 ml) were added thereto. The mixture was stirred at ambient temperature overnight and poured into dil. phosphoric acid and then extracted with ethyl acetate (100 ml). The extract was washed successively with dil. phosphoric acid, water, dil. sodium bicarbonate, dil. sodium chloride and saturated sodium chloride, and then dried over anhydrous magnesium sulfate. The solvent was evaporated to give an oil (3.4 g). The oil was chromatographed on silica gel (55 g) and elution was carried out with a mixture of ethyl acetate and methylene chloride (35:65) to give crude crystals. The crude crystals were recrystallized from ethyl acetate to give di-Z-meso-DAP(L)oBzl-(D)-GlyOBzl (2) (1.38 g).

N.M.R. ($CDCl_3$), $\delta$ (ppm): 1.1—2.1 (6H, m), 3.98 (2H, d, J=6Hz), 3.8—4.5 (2H, m), 5.04 (4H, s), 5.08 (2H, s), 5.10 (2H, s), 5.49 (2H, t, J=7Hz), 6.64 (1H, t, J=6Hz), 7.28 (20H, s)

## Preparation 15

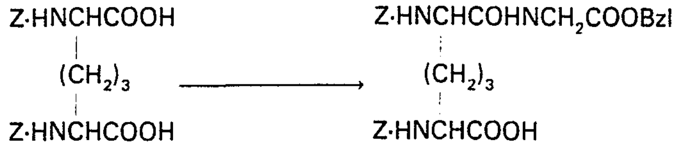

(meso+DL) (meso+DL)

Di-Z-DAP(OH)$_2$ (1) (4.58 g) was dissolved in tetrahydrofuran )50 ml) and N-hydroxysuccinimide (1.15 g) was added to the solution. To the resulting solution was added a mixture of glycine benzyl ester p-toluenesulfonate (3.21 g) and triethylamine (1.4 ml) in tetrahydrofuran. N,N'-Dicyclohexylcarbodiimide (2.06 g) was added to the resulting solution under ice-cooling and the solution was stirred at ambient temperature. N,N'-Dicyclohexylurea was filtered off and the filtrate was evaporated to dryness. The residue was dissolved in ethyl acetate and washed successively with dil. hydrochloric acid, saturated sodium chloride, saturated sodium bicarbonate and saturated sodium chloride, in turn. The organic layer was dried over magnesium sulfate and concentrated. The concentrate was chromatographed on silica gel (100 g) and eluted with a mixture of chloroform and methanol (10:1). The eluate was concentrated to dryness to give crystals of di-Z-DAP(OH)GlyoBzl (2) (2.67 g).

N.M.R. ($CDCl_3$), $\delta$ (ppm): 0.83—2.0 (6H, m), 3.3—4.5 (4H, m), 4.97 (6H, broad s), 5.0—5.6 (2H, m), 5.8—6.6 (2H, m), 7.18 (15H, m)

## Preparation 16

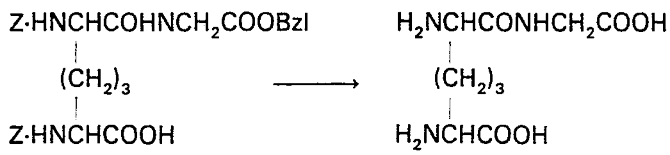

(meso+DL) (meso+DL)

(1) (2)

A solution of di-Z-DAP(OH)GlyoBzl (1) (5.1 g) in methanol (80 ml) was hydrogenated over 5% palladium black (2 g). The reaction mixture was filtered and the filtrate was evaporated to give DAP(OH)GlyOH (2) (1.77 g).

N.M.R. ($D_2O$), $\delta$ (ppm): 1.3—2.3 (6H, m), 3.73 (1H, t, J=5.5Hz), 3.80 (2H, s), 4.00 (1H, t, J=6Hz)

## Preparation 17

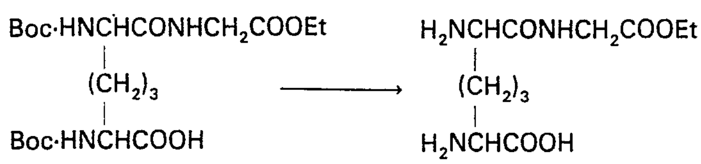

(meso+DL) (meso+DL)

(1) (2)

To a solution of di-Boc-DAP(OH)GlyOEt (1) (6.0 g) in ethyl acetate (10 ml) was added 6N hydrochloric acid/ethyl acetate (20 ml) under cooling. The mixture was stirred under cooling for four hours to give crystals. The crystals were collected and washed with ethyl acetate to give DAPGlyOEt-dihydrochloric acid salt monohydrate (2) (4.18 g).

N.M.R. ($D_2O$), $\delta$ (ppm): 1.30 (3H, t, J=7Hz), 1.1—2.2 (6H, m), 3.8—4.3 (2H, m), 4.11 (2H, s), 4.26 (2H, q, J=7Hz)

Preparation 18

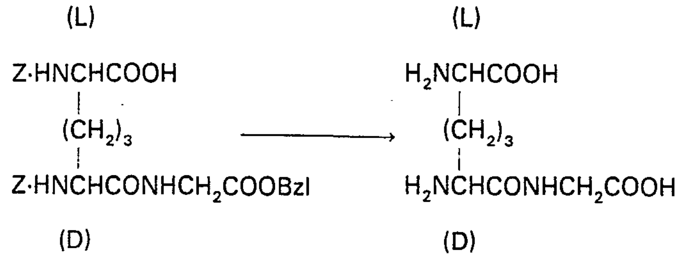

(1) (2)

A mixture of di-Z-meso-DAP(D)-GlyoBzl (1) (60 mg) and 10% palladium black (25 mg) in acetic acid (1 ml) was stirred at ambient temperature under hydrogen atmosphere for 4.5 hours. The reaction mixture was filtered through Celite. The filtrate was evaporated to give meso-DAP(D)GlyOH (2) (39 mg).

N.M.R. ($D_2O$), $\delta$ (ppm): 1.3—2.3 (6H, m), 3.73 (1H, t, J=5.5Hz), 3.80 (2H, s), 4.00 (1H, t, J=6Hz)

Preparation 19

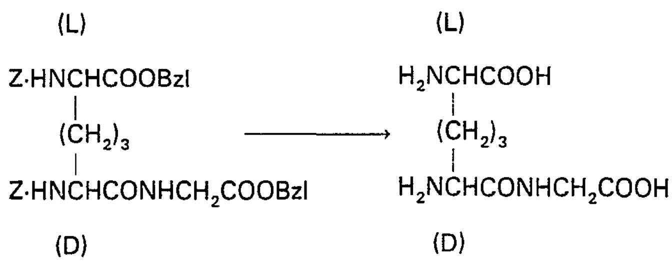

(1) (2)

To a solution of di-Z-meso-DAP(L)oBzl-(D)-GlyoBzl (1) (6.20 g) in acetic acid (100 ml) was added 10% palladium black (1.73 g) and the mixture was stirred at ambient temperature under hydrogen atmosphere for twenty hours. The reaction mixture was filtered and the catalyst was washed with acetic acid. The filtrate and the washing were combined and concentrated.

The concentrate was dissolved in a small amount of water and the solution was evaporated to dryness. This operation was repeated twice. The residue was chromatographed on a macroporous non-ionic adsorption resin, HP20 (150 ml) and elution was carried out with water. The desired fraction was evaporated. The residue was dissolved in a small amount of water and lyophilized to give amorphous solid (2.28 g).

A 300 mg portion of this amorphous solid was dissolved in water (3 ml) and the solution warmed to 50°C and then methanol was added thereto until becoming cloudy. Occasional addition of methanol was required for completion of crystallization. The crystals were collected by filtration to give meso-DAP-(D)GlyOH (2) (270 mg). mp 235°C (dec).

N.M.R. ($D_2O$), $\delta$ (ppm): 1.3—2.3 (6H, m), 3.73 (1H, t, J=5.5Hz), 3.80 (2H, s), 4.00 (1H, t, J=6Hz), $[\alpha]_D^{20} = -60.6$ (C=0.507, water)

Preparation 20

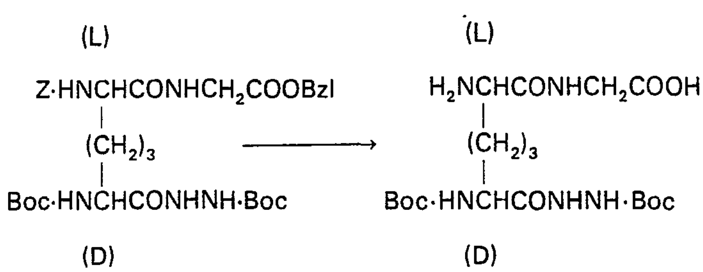

(1) (2)

Z-(L)-Boc(D)-meso-DAP(L)-GlyoBzl-(D)-NHNHBoc (1) (12.0 g) was hydrogenated in a mixture of methanol (100 ml) and acetic acid (2.4 ml) over 10% palladium black (2 g). After completion of the reaction, the catalyst was removed by filtration and the filtrate was evaporated to dryness under reduced pressure. To the residue was added water (30 ml) and the solution was evaporated to dryness. This operation was repeated three times. The residue thus obtained was triturated with ether to give Boc(D)-meso-DAP(L)GlyOH-(D)-NHNHBoc (2) (7.80 g). mp 130—138°C (dec).

N.M.R. ($CD_3OD$), δ (ppm): 1.60 (9H, s), 1.63 (9H, s), 1.7—2.0 (6H, m), 3.92 (2H, s), 3.8—4.1 (2H, m)

Preparation 21

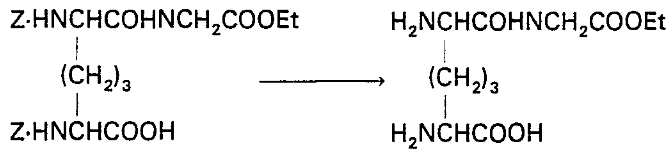

(meso) (meso)

(1) (2)

Di-Zmeso-DAP(OH)GlyOEt (1) (9.33 g) was hydrogenated in acetic acid (80 ml) with 10% palladium black (0.93 g) under conditions of two atmospheric pressure of hydrogen. The reaction mixture was filtered and the filtrate was concentrated. Ethyl acetate was added to the concentrate to give crystals of meso-DAPGlyOEt acetic acid salt (2) (5.74 g). mp 188—189°C (dec.).

N.M.R. ($D_2O$), δ (ppm): 1.44 (3H, t, J=7Hz), 1.2—2.2 (6H, m), 1.93 (3H, s), 3.76 (1H, t, J=6Hz), 3.9—4.2 (1H, m), 4.10 (2H, s), 4.20 (2H, q, J=7Hz).

Preparation 22

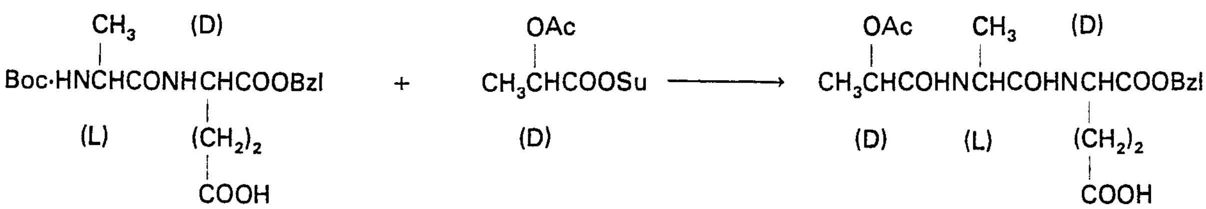

(1) (2) (3)

BOC-L-Ala-D-Glu(α-oBzl) (1) (298 mg) and anisole (0.2 ml) were added to trifluoroacetic acid (1 ml) at 0°C and the mixture was stirred at the same temperature for two hours. Trifluoroacetic acid was evaporated to dryness and the residue was triturated with ether. The resulting compound was dissolved in water (3 ml) and adjusted to pH 7 with triethylamine and additional 0.1 ml of triethylamine was added to the solution. To the resulting solution was added a solution of Lac(oAc)OSu (2) (167 mg) in dimethylformamide (3 ml) at 0°C. The resulting mixture was stirred overnight at ambient temperature and the reaction mixture was acidified with a diluted aqueous hydrochloric acid and then extracted with ethyl acetate.

The extract was washed with water and evaporated to dryness. The residue thus obtained was purified by preparative thin layer chromatography using a pre-coated silica gel plate, developing with a mixture of chloroform and methanol (8:2) to give D-Lac(oAc)-L-Ala-γ-D-Glu(OH) (α-oBzl) (3) (270 mg).

N.M.R. ($CD_3OD$), δ (ppm): 1.40 (3H, d, J=7Hz), 1.47 (3H, d, J=7Hz), 2.13 (3H, s), 2.1—2.5 (4H, m), 4.4—5.3 (3H, m), 5.20 (2H, s), 7.40 (5H, s)

Preparation 23

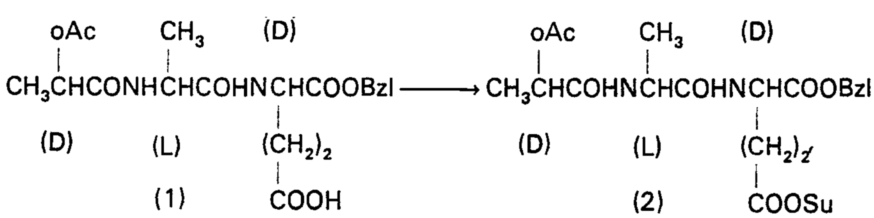

(1) (2)

Dicyclohexylcarbodiimide (32 mg) was added to a mixture of D-Lac(oAc)-L-Ala-D-Glu($\alpha$-oBzl)(OH) (1) (65 mg) and N-hydroxysuccinimide (18 mg) in dioxane (3 ml) at 0°C. The resulting mixture was stirred overnight at ambient temperature and filtered. The filtrate was evaporated to dryness to give D-Lac(oAc)-L-Ala-D-Glu($\alpha$-oBzl)(oSu) (2) (80 mg).

## Preparation 24

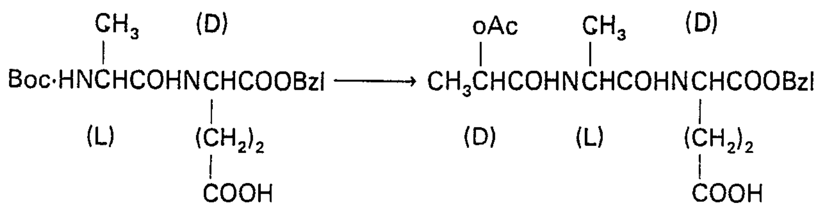

(1) (2)

Boc-L-Ala-D-Glu($\alpha$-oBzl)(OH) (1) (65 g) was dissolved in trifluoroacetic acid (260 ml) and the solution was stirred for fifteen minutes at ambient temperature. The solution was evaporated under reduced pressure. The residue thus obtained was dissolved in 50% aqueous acetone (600 ml).

O-acetyl-D-lactic chloride (28.8 g) was added dropwise to the solution while stirring under ice-cooling, maintaining the pH 7—8.

The stirring was continued for an hour at the same temperature and then acetone was evaporated under reduced pressure. The resulting aqueous solution was washed with ethyl acetate (300 ml) and adjusted to pH 2 with 10% aqueous hydrochloric acid. Extraction was carried out twice with ethyl acetate (600 ml and 300 ml). The ethyl acetate layer was washed with saturated aqueous sodium chloride solution and dried over anhydrous magnesium sulfate and then evaporated under reduced pressure to give D-Lac(oAc)-L-Ala-$\gamma$-D-Glu($\alpha$-oBzl)(OH) (2) (64.7 g).

N.M.R. ($CDCl_3$), $\delta$ (ppm): 1.40 (3H, d, J=7Hz), 1.47 (3H, d, J=7Hz), 2.13 (3H, s), 2.1—2.5 (4H, m), 4.4—5.3 (3H, m), 5.20 (2H, s), 7.40 (5H s).

## Preparation 25

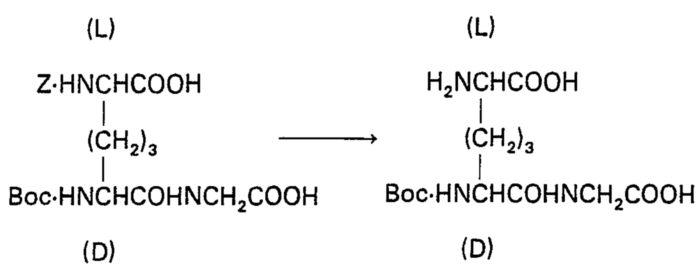

(1) (2)

Z-(L)-Boc-(D)meso-DAP-(D)GlyOH (1) (2.80 g) was hydrogenated in methanol (56 ml) over 10% palladium black (0.82 g) under conditions of ordinary atmospheric pressure of hydrogen at ambient temperature for four hours. To the reaction mixture was added methanol (50 ml) and the resulting mixture was warmed to 40°C and then filtered. The catalyst was washed well with methanol and filtered. The filtrate and washings were combined and concentrated to give crystals. The crystals was diluted with isopropanol and cooled in a refrigerator and then washed with ether to give Boc-(D)meso-DAP(D)GlyOH (2) (1.687 g)

N.M.R. ($CD_3OD+D_2O$), $\delta$ (ppm): 1.3—2.2 (6H, m), 1.40 (9H, s), 3.66 (1H, t, J=5Hz), 3.7—4.2 (1H, m), 3.88 (2H, s).

$[\alpha]_D^{23} = +12.2°$ (C=0.5, methanol)

Preparation 26

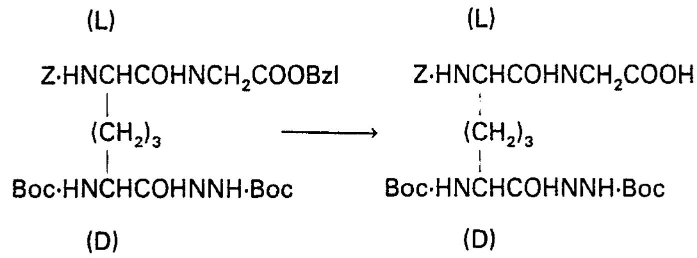

(1) (2)

To a solution of Z-(L)-Boc-(D)-mesoDAP-(D)-NHNHBoc-(L)-GlyoBzl (1) (105 mg) in methanol was added 0.1N aqueous sodium hydroxide (1.5 ml). The mixture was stirred at ambient temperature for two hours and evaporated. The resulting aqueous solution was acidified to pH 2 with 5% aqueous hydrochloric acid and extracted with ethyl acetate.

The extract was washed with water, dried over magnesium sulfate and evaporated to give Z-(L)-Boc(D)-mesoDAP(D)-NHNHBoc-(L)GlyOH (2) (85 mg).

N.M.R. ($CDCl_3$), $\delta$ (ppm): 1.43 (18H, s), 1.3—2.1 (6H, m), 3.9—4.5 (4H, m), 5.10 (2H, s), 6.2—6.4 (4H, m), 7.10 (1H, broad s), 7.33 (5H, s), 8.95 (1H, broad s)

Preparation 27

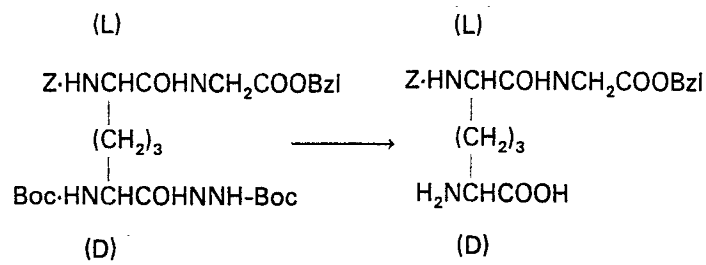

(1) (2)

Z-(L)-Boc-(D)-mesoDAP-(D)-NHNHBoc-(L)-GlyoBzl (1) (2.0 g) was added to trifluoroacetic acid (10 ml) and the mixture was stirred at ambient temperature for 15 minutes.

Trifluoroacetic acid was distilled off and the residue was dissolved in 50% aqueous dioxane (30 ml) and then cooled to 0°C. To this solution was added N-bromosuccinimide (1.04 g) and the mixture was stirred at the same temperature for an hour. Dioxane was distilled off and the residue was adjusted to pH 6 with saturated aqueous sodium bicarbonate. The precipitated crystalline solid was filtered and washed with water to give Z-(L)-mesoDAP-(L)-GlyoBzl (2) (1.15 g).

N.M.R. (DMSO-$d_6$), $\delta$ (ppm): 1.2—2.0 (6H, m), 3.8—4.2 (4H, m), 5.05 (2H, s), 5.15 (2H, s), 7.40 (10H, s)

Preparation 28

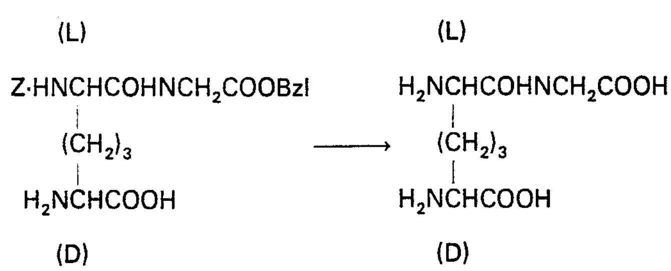

(1) (2)

A solution of Z-(L)-mesoDAP-(L)GlyoBzl (1) (100 mg) in a mixture of methanol (10 ml) and water

(3 ml) was hydrogenated over 10% palladium-black (50 mg) under ordinary atmospheric pressure of hydrogen.

The reaction mixture was filtered and the filtrate was evaporated. The residue was pulverized to give mesoDAP-(L)GlyOH (2) (44 mg).

N.M.R. ($D_2O$), δ (ppm): 1.3—2.1 (6H, m), 3.73 (1H, t, J=6Hz), 3.80 (2H, s), 4.01 (1H, t, J=6Hz)

Preparation 29

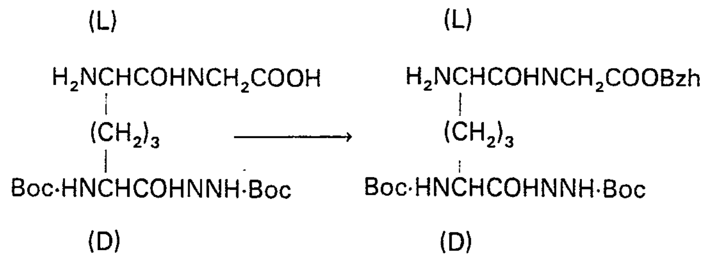

(1) (2)

P-Toluenesulfonic acid (monohydrate) (0.56 g) was added to a solution of Boc-(D)-mesoDAP-(D)-NHNHBoc-(L)-GlyoH (1) (1.39 g). To the mixture was added a solution of diphenyldiazomethane (0.62 g) in methanol (1 ml). The resulting mixture was stirred at ambient temperature for 30 minutes.

To the reaction mixture was added an additional diphenyldiazomethane (0.78 g) until the starting material (1) was disappeared on thin layer chromatography.

An excess of the reagent was destroyed by adding acetic acid and the mixture was adjusted to pH 8 with saturate aqueous sodium bicarbonate and then evaporated.

The residue was dissolved in ether (3 ml) and triturated with n-hexane (5 ml). The solvents were removed by decantation. This operation was further repeated twice. The residue was put on a column of silica-gel (30 g) and eluted with a mixture of ethyl acetate and methanol (10:1).

The fractions containing the object compound (2) were combined and evaporated to give a solid, which was purified by reprecipitation from a mixture of ether and n-hexane (1:2) to give Boc-(D)-mesoDAP-(D)-NHNHBoc-(L)-GlyoBzh (2) (1.22 g).

N.M.R. ($CDCl_3$), δ (ppm): 1.33 (18H, s), 1.1—1.8 (6H, m), 3.1—3.5 (1H, m), 3.9—4.3 (3H, m), 5.2—5.5 (1H, m), 6.75 (1H, s), 7.3 (10H, s), 7.6—8.0 (1H, m)

Preparation 30

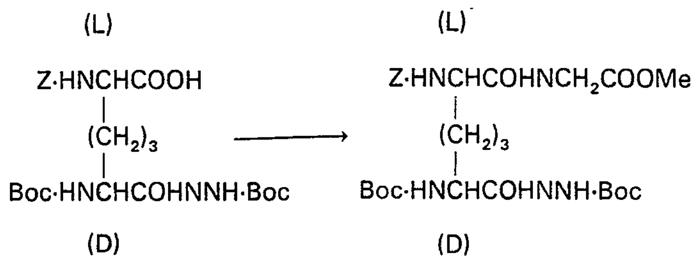

(1) (2)

To a mixture of Z-(L)-Boc-(D)-mesoDAP-(D)-NHNHBoc (1) (1.40 g) and glycine methyl ester hydrochloride (392 mg) in dimethyl formamide (10 ml) were added N-hydroxysuccinimide (0.45 g) and dicyclohexylcarbodiimide (0.645 g) at 0°C, and the resulting mixture was stirred at the same temperature for 30 minutes and at ambient temperature for overnight. The resulting precipitate was filtered off and the filtrate was concentrated. The concentrate was dissolved in ethyl acetate (50 ml) and the solution was washed successively with 0.5N hydrochloric acid, water, 2.5% sodium bicarbonate and water.

The organic layer was dried over magnesium sulfate and evaporated to give Z-(L)-Boc-(D)-mesoDAP-(D)-NHNHBoc-(L)-GlyoMe (2) (1.45 g).

N.M.R. ($CDCl_3$), δ (ppm): 1.40 (18H, s), 1.5—2.0 (6H, m), 3.70 (3H, s), 3.98 (2H, d, J=6Hz), 4.0—4.5 (2H, m), 5.07 (2H, s), 5.45 (1H, d, J=8Hz), 5.93 (1H, d, J=8Hz), 6.83 (1H, broad s), 7.1—7.3 (1H, m), 7.33 (5H, s), 8.63 (1H, broad s).

Preparation 31

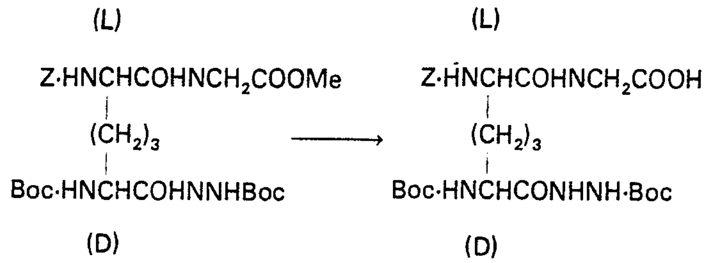

(1) (2)

To a solution of Z-(L)-Boc-(D)-mesoDAP-(D)-NHNHBoc-(L)-GlyOME (1) (1.41 g) in a mixture of water (14 ml) and methanol (10 ml) was added 1N sodium hydroxide (2.3 ml) at 0°C.

The mixture was stirred at the same temperature for 30 minutes and at ambient temperature for 3.5 hours.

Methanol was distilled off and the resulting solution was washed with ethyl acetate. The aqueous layer was adjusted to pH 3 with 5% hydrochloric acid and extracted with ethyl acetate.

The extract was washed with a saturated sodium chloride, dried over magnesium sulfate and then evaporated to give Z-(L)-Boc-(D)-mesoDAP-(D)-NHNHBoc-(L)-GlyOH (2) (1.24 g).

N.M.R. ($CDCl_3$), $\delta$ (ppm): 1.43 (18H, s), 1.3—2.1 (6H, m), 3.9—4.5 (4H, m), 5.10 (2H, s), 6.2—6.4 (4H, m), 7.10 (1H, broad s), 7.33 (5H, s), 8.95 (1H, broad s)

Preparation 32

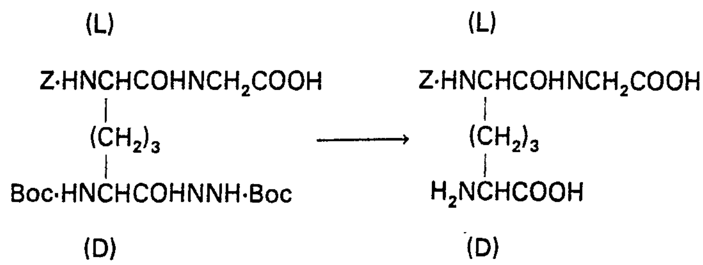

(1) (2)

Z-(L)-Boc-(D)-mesoDAP-(D)-NHNHBoc-(L)-GlyOH (1) (270 mg) was added to trifluoroacetic acid (5 ml) and the mixture was stirred at ambient temperature for 15 minutes.

Trifluoroacetic acid was distilled off and the residue was pulverized with ether. The solvent was removed by decantation and the solid material thus obtained was dissolved in 60% aqueous acetic acid (30 ml) and manganese dioxide (180 mg) was added to the solution.

The resulting mixture was stirred at ambient temperature for an hour. The reaction mixture was filtered and the filtrate was evaporated. The residue was dissolved in water and the solution was evaporated.

The residue was dissolved in 50% ethanol (60 ml) and adjusted to pH 9 with dil. aqueous ammonia and stored in a refrigerator. The solution was treated with an active carbon (30 mg) and filtered. The filtrate was concentrated and the concentrate was dissolved in water (20 ml) and put on a column of carbon (15 ml).

The column was washed with water and eluted with 70% aqueous acetone. The extract was evaporated to give Z-(L)-mesoDAP-(L)-GlyOH (2) (85 mg).

N.M.R. ($D_2O$-$NaHCO_3$), $\delta$ (ppm): 1.3—2.2 (6H, m), 3.77 (2H, s), 4.1 (1H, m), 5.0 (1H, m), 5.18 (2H, s), 7.65 (5H, s).

Preparation 33

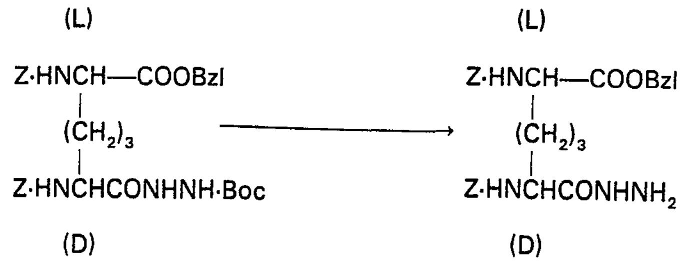

(1) (2)

A solution of di-Z-meso-DAP(L)oBzl-(D)-NHNHBoc (1) (6.68 g) in trifluoroacetic acid (30 ml) was stirred at ambient temperature for twenty minutes. The resulting solution was concentrated and the residue was dissolved in methylenechloride. The solution was evaporated under reduced pressure and the residue was crystallized from ether to give di-Z-meso-DAP(L)oBzl-(D)-$NHNH_2$ trifluoroacetic acid salt. (2) (6.627 g). mp 113—114°C.

N.M.R. (DmSo-$d_6$), $\delta$ (ppm): 1.1—2.0 (6H, m), 3.6—4.3 (2H, m), 5.03 (4H, s), 5.11 (2H, s), 7.33 (15H, s), 7.1—8.4 (6H, m)

Preparation 34

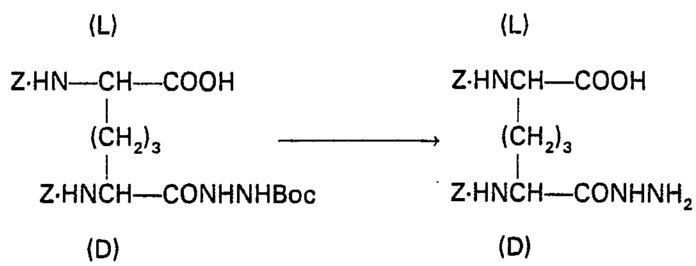

(1) (2)

A mixture of di-Z-meso-DAP(D)-NHNHBoc (1) (6.41 g) and anisole (3 ml) in trifluoroacetic acid (17 ml) was stirred at 0°C for 1.5 hours. The reaction mixture was concentrated under reducing pressure and ether (50 ml) was added to the concentrate. The ether solution was triturated to give precipitates. The precipitates were washed twice with ether and pumped to give di-Z-meso-DAP(D)$NHNH_2$ trifluoroacetic acid salt (2) (7.0 g).

N.M.R. ($CD_3OD$), $\delta$ (ppm): 1.2—2.1 (6H, m), 3.9—4.5 (2H, m), 5.05 (4H, s), 7.25 (10H, s)

Preparation 35

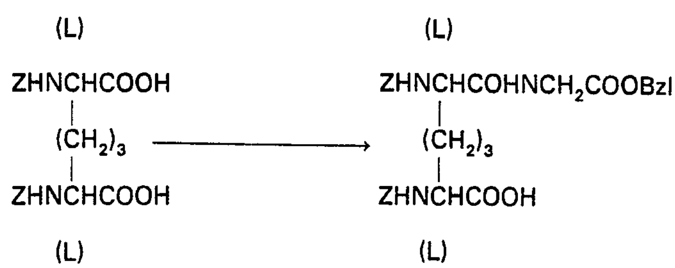

(1) (2)

To a mixture of di-Z-L-DAP (1) (4.59 g) and N-methylmorpholine (2.2 ml) in methylene chloride (50 ml) was added iso-butoxycarbonyl chloride (1.69 ml) at −20°C and the mixture was stirred at −10°C ∼ −15°C for 30 minutes. The reaction mixture was cooled to −30°C and a mixture of H-GlyoBzl p-toluenesulfonate (4.38 g) and N-methylmorpholine (1.43 ml) in methylene chloride (25 ml) was added thereto. The mixture was stirred at −10°C for an hour and then allowed to warm to ambient temperature. The reaction mixture was concentrated and the concentrate was poured into a mixture of

ethyl acetate and dil. hydrochloric acid.

Insoluble materials were filtered off and the organic layer was separated and washed successively with dil. sodium bicarbonate, dil. hydrochloric acid, water and brine. During this operation, crystals were separated out which was collected by filtration to give di-Z-L-DAP-mono-GlyoBzl (2) (0.79 g). The filtrate was dried over magnesium sulfate and evaporated. The residue was combined with the insoluble materials described above and then recrystallized from ethyl acetate to give additional di-Z-L-DAP-mono-GlyoBzl (2) (2.55 g).

N.M.R. (DmSo-$d_6$): 1.1—1.9 (6H, m), 3.89 (2H, broad d, J=5Hz), 3.7—4.4 (2H, m), 5.00 (4H, s), 5.10 (2H, s), 7.1—7.6 (2H, m), 7.32 (15H, s), 8.31 (1H, t, J=5Hz).

## Preparation 36

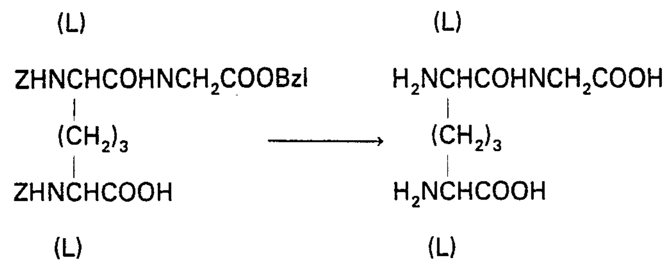

(1) (2)

Di-Z-L-DAP-mono-GlyoBzl (1) (3.12 g) was dissolved in a mixture of methanol (10 ml) and acetic acid (60 ml) by warming. To the solution was added 10% palladium black (850 mg) and the mixture was stirred under an atmospheric pressure of hydrogen overnight, during which time a white precipitate was appeared.

The catalyst and the precipitate were collected by filtration and the filter cake was washed with 50% aqueous acetic acid and water. The filtrate and the washings were combined and evaporated. The residue was dissolved in water and the solution was evaporated in order to remove acetic acid. This operation was repeated twice and the residue was triturated with hot water and then methanol was added thereto. The suspension was cooled in a refrigerator and the resulting precipitate was filtered and washed with methanol and ether to give L-DAP-monoGlyOH (2) (1.295 g).

I.R. (Nujol): 1660, 1640 (shoulder), 1590 (broad), 1530 $cm^{-1}$

N.M.R. ($D_2O$), $\delta$ (ppm): 1.2—2.3 (6H, m), 3.5—4.3 (2H, m), 3.87 (2H, s)

## Preparation 37

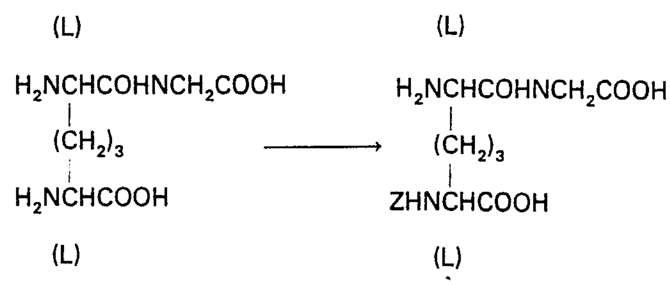

(1) (2)

To a mixture of L-DAP-monoGlyOH (1) (1.25 g) and cupric chloride (dihydrate) (1) (0.86 g) in water (120 ml) were added 3N sodium hydroxide (5.5 ml) and carbobenzyloxy chloride (3.6 ml) at 5°C. The mixture was stirred at 5°C for 4.25 hours, during which time the pH of the reaction mixture was kept at 11—11.5 by adding 3N sodium hydroxide.

To the reaction mixture was added an additional carbobenzyloxy chloride (1.08 ml) and the resulting mixture was stirred at the same temperature for 6 hours, maintaining pH 11—11.5 with 3N sodium hydroxide.

Another portion of carbobenzyloxy chloride (1.08 ml) was added to the reaction mixture and the resulting mixture was stirred at 5°C for 2.5 hours, keeping the pH at 11—11.5. The reaction mixture was acidified to pH 2 with dil. hydrochloric acid and washed twice with ether. The aqueous layer was

adjusted to pH 4.5 with dil. sodium hydro(de and then hydrogen sulfide gas was bubbled at 5°C. The resulting precipitate was filtered off and the filtrate was concentrated to 50 ml. The concentrate was adjusted to pH 2 and chromatographed on a column of a macroporous non-ionic adsorption resin, HP 20 (70 ml). Fractions eluted with 50% aqueous methanol were collected and evaporated. The residue was triturated with ether containing a small amount of methanol to give z-($\alpha$-L-DAP-($\varepsilon$)-GlyOH(2) (1.30 g.).

N.M.R. ($D_2O$), $\delta$ (ppm): 1.2—2.2 (6H, m), 3.88 (2H, d, J=2.5Hz), 3.8—4.3 (2H, m), 5.10 (2H, s), 7.43 (5H, s)

Example 1

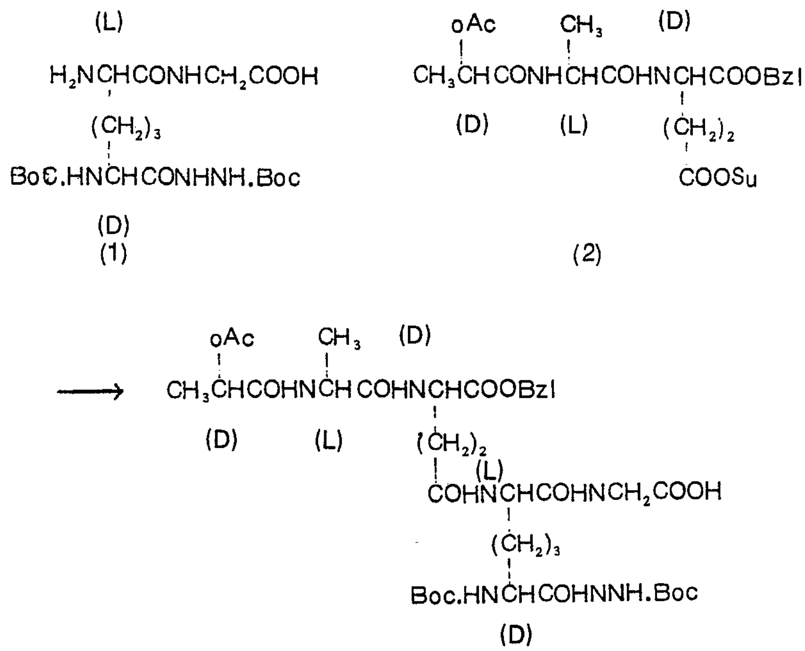

(1) (2)

(3)

(1) Example 1-1

Boc(D)-meso-DAP(L)GlyOH-(D)-NHNHBoc (1) (7.7 g) was dissolved in water (150 ml) and the solution was adjusted to pH 8 with N-methylmorpholine. To the solution was added a dioxane solution (180 ml) of D-Lac(oAc)-L-Ala-$\gamma$-D-Glu($\alpha$-oBzl)(oSu) (2) (containing 26.7 mmole of compound (2) as activated ester).

The mixture was stirred for four hours at ambient temperature. Dioxane was distilled off under reduced pressure and the resulting mixture was washed with ether (100 ml) and adjusted to pH 3 with 5% aqueous hydrochloric acid. The solution was extracted with ethyl acetate (300 ml and 150 ml) and the extract was washed with water and dried over anhydrous magnesium sulfate and then evaporated to dryness. The residue was crystallized from a mixture of chloroform and ether (1:3) to give D-Lac(oAc)-L-Ala-$\gamma$-D-Glu($\alpha$-oBzl)-(L)-Boc-(D)-meso-DAP(L)GlyOH-(D)-NHNHBoc (3) (10.8 g). mp 105—113°C (dec.).

N.M.R. ($CD_3OD$), $\delta$ (ppm): 1.3—1.8 (30H, m), 2.08 (3H, s), 2.1—2.4 (4H, m), 3.92 (2H, s), 4.2—4.6 (4H, m), 4.8—5.1 (1H, m), 5.17 (2H, s), 7.33 (5H, s)

(2) Example 1-2

D-Lac(oAc)-L-Ala-$\gamma$-D-Glu(OH)oBzl (5.2 g) was dissolved in methylene chloride (60 ml) and N-methylmorpholine (1.10 g) was added thereto.

To the mixture was added dropwise isobutyl chloroformate (1.50 g) at −10—−15°C and the mixture was stirred at −10—−15°C for thirty minutes.

To the mixture was added a solution prepared by dissolving bis(trimethylsilyl)acetamide (9 ml) and Boc(D)meso-DAP(L)GlyOH-(D)-NHNHBoc (1) (4.60 g) in a mixture of methylene chloride (50 ml) and dimethylformamide (10 ml).

The resulting solution was stirred at −10—−15°C for two hours and at 0°C for an hour and then concentrated to about 30 ml. To the concentrate were added ethyl acetate (200 ml) and 2% aqueous hydrochloric acid (100 ml). The ethyl acetate layer was separated and washed with water and then

dried over anhydrous magnesium sulfate.

Ethyl acetate was evaporated to dryness under reduced pressure and the residue thus obtained was washed with ether to give D-Lac(oAc)-L-Ala-γ-D-Glu(α-oBzl)-(L)-Boc-(D)-meso-DAP(L)GlyOH-(D)-NHNHBoc (3) (8.2 g) of which N.M.R. spectrum was identical with that of the product prepared in Example 1-1.

Example 2

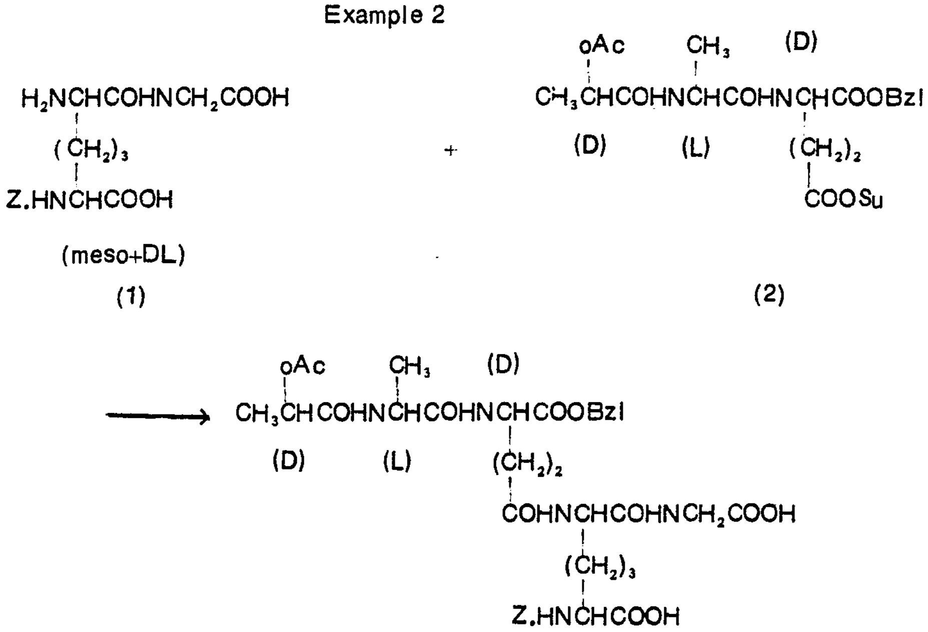

(meso+DL)
(1)

(2)

(3)

ZDAP(OH)GlyOH (1) (80 mg) was added to a solution of D-Lac(oAc)-L-Ala-γ-D-Glu(oSu)oBzl (2) (59 mg) in dimethylformamide (4 ml) containing triethylamine (0.03 ml) at 0°C. The mixture was stirred overnight at ambient temperature and then evaporated to dryness. To the residue thus obtained was added dil. hydrochloric acid and then extraction was carried out with ethyl acetate. The extract was washed with water and evaporated to dryness. The residue was recrystallized from a mixture of hexane and chloroform to give D-Lac-(oAc)-L-Ala-D-Glu(α-oBzl)-Z-DAP(OH)GlyOH (3) (86 mg).

N.M.R. ($CD_3OD$), δ (ppm): 1.35 (3H, d, J=7Hz), 1.43 (3H, d, J=7Hz), 1.3—2.2 (8H, m), 2.12 (3H, s), 2.30 (2H, m), 3.95 (2H, s), 4.0—4.6 (4H, m), 5.12 (2H, s), 5.18 (2H, s), 7.36 (10H, s)

Example 3

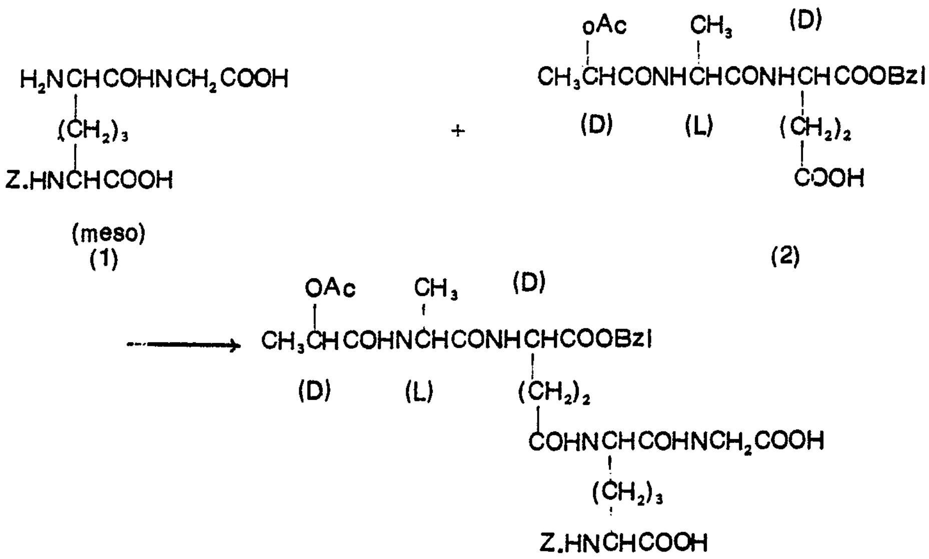

(meso)
(1)

(2)

(3)

Z-meso-DAPGlyOH(1) (1.23 g) and bis(trimethylsilyl)-acetamide (4.00 g) were suspended in methylene chloride (50 ml) and the suspension was stirred at ambient temperature for four hours.

On the other hand, D-Lac-(-oAc)-L-Ala-$\gamma$-D-Glu(OH)oBzl (2) dicyclohexylamine salt (2.34 g) was dissolved in methylene chloride (30 ml), and triethylamine hydrochloric acid salt (0.54 g) was added at ambient temperature to the solution while stirring.

The stirring was continued for two hours and the reaction mixture was cooled to −30°C and a solution of isobutyl chloroformate (0.53 g) in methylene chloride (50 ml) was added dropwise thereto.

The resulting mixture was reacted for twenty minutes at the same temperature. To the reaction mixture was added dropwise a methylene chloride solution of Z-meso-DAPGlyOH prepared above and the resulting mixture was stirred at −30°C for an hour.

The reaction mixture was reacted at ambient temperature for four hours and filtered. The filtrate was washed with 10% aqueous hydrochloric acid and concentrated under reduced pressure and the residue thus obtained was extracted with ethyl acetate.

The ethyl acetate layer was washed with water and dried over anhydrous magnesium sulfate and concentrated under reduced pressure to give an oily D-Lac(oAc)-L-Ala-$\gamma$-D-Gly($\alpha$-oBzl)-Z-meso-DAPGlyOH (3 g).

N.M.R. ($CD_3OD$), $\delta$ (ppm): 1.35 (3H, d, J=7Hz), 1.43 (3H, d, J=7Hz), 1.3—2.2 (8H, m), 2.12 (3H, s), 2.30 (2H, m), 3.95 (2H, s), 4.0—4.6 (4H, m), 5.12 (2H, s), 5.18 (2H, s), 7.36 (10H, s)

## Example 4

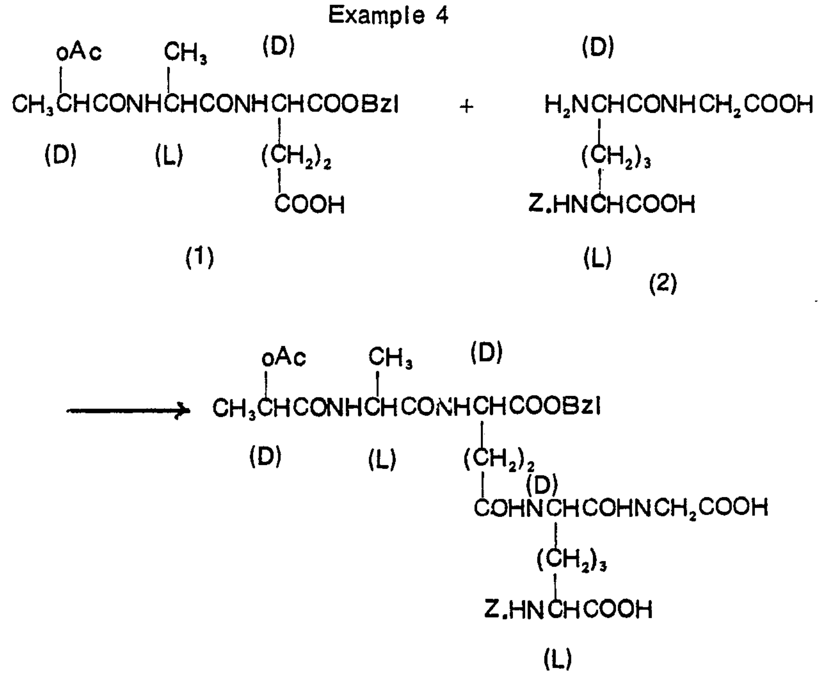

(1) (2) (3)

To a mixture of D-Lac(oAC)-L-Ala-$\gamma$-D-Glu(OH)($\alpha$-oBzl) (1) (635 mg) and N-hydroxysuccinimide (178 mg) in dioxane (7 ml) was added N,N'-dicyclohexylcarbodiimide (320 mg).

The mixture was stirred at 10°C for ten minutes and further stirred at ambient temperature for 14 hours. The reaction mixture was filtered and the filtrate was evaporated to dryness and the residue was dissolved in dioxane (4.5 ml). A 3 ml portion of this solution was added to a mixture of Z-(L)-mesoDAP-(D)-GlyOH (270 mg) and N-methylmorphorine (156 $\mu$l) in dioxane (4 ml) and the mixture was stirred at ambient temperature. After 4.5 hours, a 0.9 ml portion of the above dioxane solution and N-methylmorphorine (100 $\mu$l) was added and the mixture was stirred at the same temperature.

After 4.5 hours an additional 0.6 ml of the above dioxane solution was added and the mixture was stirred for 1.25 hours. The reaction mixture was washed with ether, acidified to pH 2 with a diluted aqueous hydrochloric acid and extracted with a mixture of ethyl acetate and methylene chloride, containing 1N hydrochloric acid, water and brine, dried over magnesium sulfate and evaporated to give an amorphous solid (660 mg) which was dissolved in chloroform and triturated with ether to give D-Lac)oAc)-L-Ala-$\gamma$-D-Glu($\alpha$-oBzl)-(D)-Z-(L)-mesoDAP-(D)-GlyOH (3) (450 mg).

N.M.R. ($CDCl_3$—$CD_3OD$), $\delta$ (ppm): 1.37 (3H, d, J=7Hz), 1.45 (3H, d, J=7Hz), 1.2—1.9 (6H, m), 2.0—2.5 (4H, m), 3.93 (2H, broad s), 4.1—4.7 (3H, m), 5.10 (2H, s), 5.17 (2H, s).

Example 5

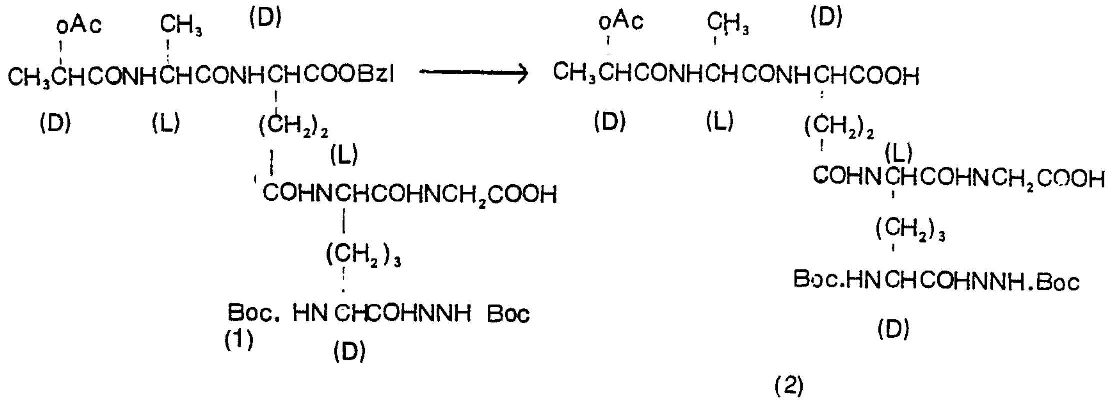

D-Lac(oAc)-L-Ala-$\gamma$-D-Glu($\alpha$-oBzl)-(L)-Boc-(D)meso-DAP(L)GlyOH-(D)-NHNHBoc (1) (9.4 g) was hydrogenated in acetic acid (100 ml) over 10% palladium black (2.0 g) for 2 hours under 1.5 hydrogen atmospheric pressure at ambient temperature.

After completion of the reaction, the catalyst was filtered off and the filtrate was evaporated to dryness under reduced pressure. The residue was triturated with ether to give D-Lac(oAc)-L-Ala-$\gamma$-D-Glu($\alpha$-OH)-(L)-Boc-(D)-meso-DAP-(L)GlyOH-(D)-NHNHBoc (2) (7.50 g).

N.M.R. ($CD_3OD$), $\delta$ (ppm): 1.3—1.9 (m), 2.10 (3H, s), 2.1—2.4 (4H, m), 3.90 (2H, s), 4.2—4.6 (4H, m), 4.8—5.1 (1H, m)

Example 6

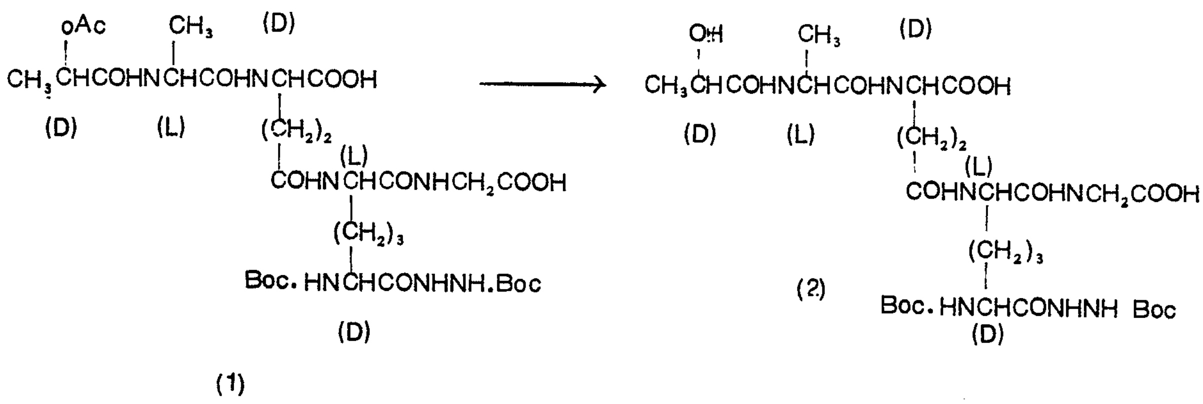

D-Lac(oAc)-L-Ala-$\gamma$-D-Glu($\alpha$-OH)-(L)-Boc-(D)-meso-DAP(L)-Gly(OH)-(D)-NHNHBoc (1) (1.65 g) was dissolved in 50% aqueous methanol (32 ml) and the solution was stirred for 2.5 hours at ambient temperature, maintaining the pH at 9.0 with 5% aqueous solution of potassium carbonate. The solution was evaporated to dryness under reduced pressure. The residue was adjusted to pH 3 with 5% aqueous hydrochloric acid and then passed through a column packed with a macroporous non-ionic adsorption resin, HP20 (500 ml). The resin was washed with water (200 ml) and eluted with a mixture of methanol and water (1:1 volume). The eluate was evaporated to dryness and the residue was washed with ether to give D-Lac-L-Ala-$\gamma$-D-Glu($\alpha$-OH)-(L)-Boc-(D)-meso-DAP(L)GlyOH-(D)-NHNHBoc (2) (1.24 g).

I.R. (Nujol): 3270, 3200—2600, 1720, 1640 $cm^{-1}$

N.M.R. ($CD_3OD$), $\delta$ (ppm): 1.47 (18H, s), 3.93 (2H, s)

Example 7

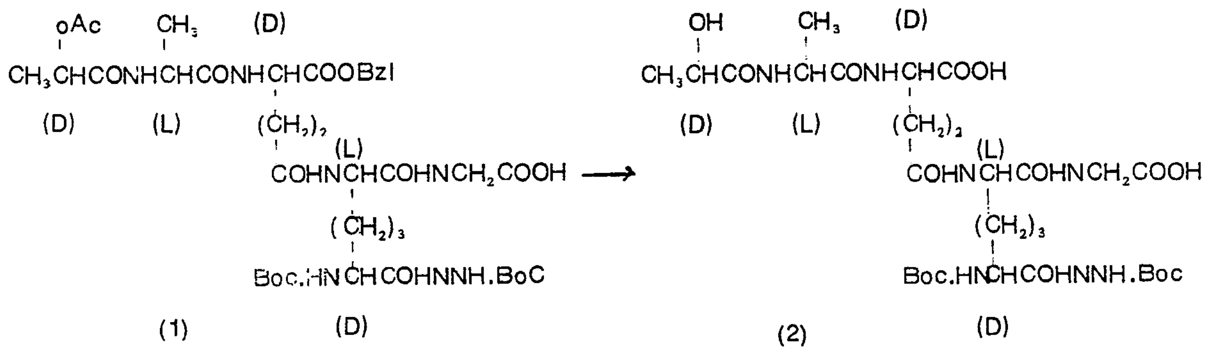

(1) (2)

D-Lac(oAc)-L-Ala-γ-D-Glu(α-oBzl)-(L)-Boc-(D)-meso-DAP(L)GlyOH-(D)-NHNHBoc (1) (1.0 g) was dissolved in 50% aqueous methanol (20 ml), and 1N aqueous sodium hydroxide (3.7 ml) was added thereto.

The resulting solution was stirred for two hours at ambient temperature and concentrated to about 5 ml. The concentrate was adjusted to pH 3 and passed through a column packed with a macroporous non-ionic adsorption resin, HP20 (24 ml). The column was washed with water (100 ml) and eluted with a mixture of methanol and water (1:1) and the eluate was evaporated to dryness under reduced pressure. The residue thus obtained was washed with ether to give D-Lac-L-Ala-γ-D-Glu(α-OH)-(L)-Boc-(D)-meso-DAP(L)GlyOH-(D)-NHNHBoc. (2) (660 mg) which was identified with the product prepared in Example 6.

Example 8

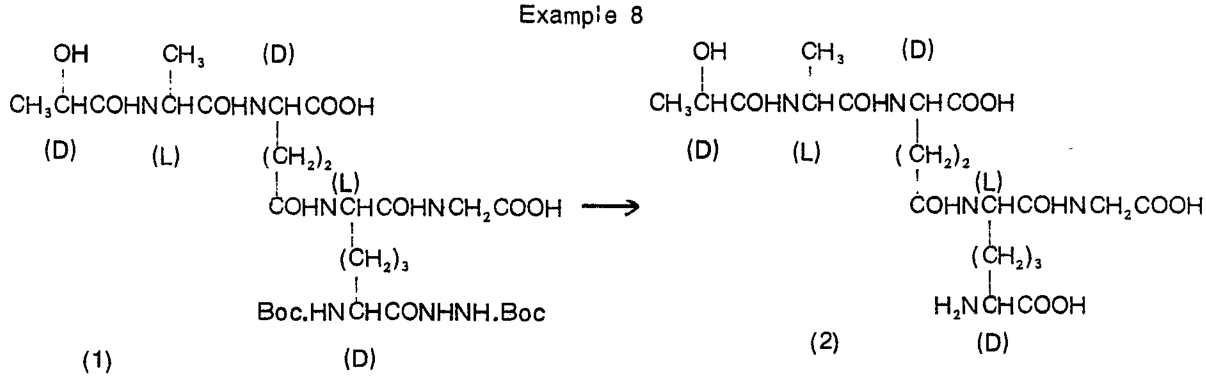

(1) (2)

(1) Example 8-1

D-Lac-L-Ala-γ-D-Glu(α-OH)-(L)-Boc-(D)-meso-DAP(L)-GlyOH-(D)-NHNHBoc (1) (200 mg) was dissolved in trifluoroacetic acid (1 ml) and the solution was stirred for fifteen minutes at ambient temperature.

The solution was evaporated to dryness under reduced pressure. The residue was triturated with ether.

The powder thus obtained was dissolved in water (5 ml) and 0.1N aqueous sulfuric acid (6.8 ml) was added. To the mixture was added dropwise a solution of sodium periodate (146 mg) in water (2 ml). The mixture was stirred for an hour under ice-cooling and then the excess reagent was decomposed with sodium bisulfite. The resulting solution was adjusted to pH 3 with an aqueous saturated sodium bicarbonate and concentrated to 1 ml under reduced pressure. The concentrate was passed through a column packed with a macroporous non-ionic adsorption resin, HP20 and eluted with water. The eluate was lyophilized to give D-Lac-L-Ala-γ-D-Glu(α-OH)-(L)-meso-DAP(L)GlyOH (2) (86 mg) as a solid.

N.M.R. ($D_2O$), δ (ppm): 1.40 (3H, d, J=7Hz), 1.46 (3H, d, J=7Hz), 3.88 (1H, t, J=5Hz), 4.02 (2H, s)
$[\alpha]_D = -30.0$ (c=0.4, water)

(2) Example 8-2

D-Lac-L-Ala-γ-D-Glu(α-OH)-(L)-Boc-(D)-meso-DAP(L)-GlyOH-(D)-NHNHBoc (1) (220 mg) was dissolved in trifluoroacetic acid (1.5 ml) and the solution was stirred for fifteen minutes at ambient temperature. The trifluoroacetic acid was evaporated to dryness under reduced pressure and the residue thus obtained was triturated with ether. The powder was dissolved in a mixture of water (5 ml) and dioxane (7 ml), and N-bromosuccinimide (110 mg) was added under ice-cooling thereto and the

solution was stirred for an hour. The reaction mixture was concentrated to about 1 ml and the concentrate was adjusted to pH 2.5 with 5% aqueous sodium bicarbonate. The solution was passed through a column packed with a macroporous non-ionic adsorption resin, HP20 (16 ml) and elution was carried out with water and the eluate was lyophilized to give D-Lac-L-Ala-γ-D-Glu(α-OH)-(L)-meso-DAP(L)GlyOH (2) (150 mg), which was identified with the product prepared in Example 8-1.

(3) Example 8-3

D-Lac-L-Ala-γ-D-Glu(α-OH)-(L)-Boc-(D)-meso-DAP(L)-GlyOH-(D)-NHNHBoc (1) (200 mg) was dissolved in trifluoroacetic acid (1 ml) and the solution was stirred at ambient temperature for fifteen minutes. Trifluoroacetic acid was evaporated to dryness under reduced pressure. The residue thus obtained was triturated and the powder was dissolved in 60% aqueous acetic acid (15 ml). To the solution was added manganese dioxide (65 mg) while stirring at ambient temperature and the mixture was stirred at the same temperature for 1.5 hours. Insoluble materials were filtered off and then the filtrate was evaporated to dryness under reduced pressure.

The residue thus obtained was dissolved in 50% aqueous ethanol and cooled to −10°——20°C and then adjusted to pH 9 with aqueous ammonium.

The solution was allowed to stand at the same temperature for two hours. Insoluble materials were filtered off and the filtrate was evaporated to dryness under reduced pressure. The residue was passed through a column packed with activated carbon (10 ml). The column was washed with water (30 ml) and eluted with 70% aqueous acetone. The fraction containing the object compound was collected and evaporated to dryness under reduced pressure. The residue was pulverized with a mixture of methanol and ether to give D-Lac-L-Ala-γ-D-Glu(α-OH)-(L)-meso-DAP(L)GlyOH (2) (84 mg), whose structure was confirmed in comparison of data of the products prepared in Example 8-1.

## Example 9

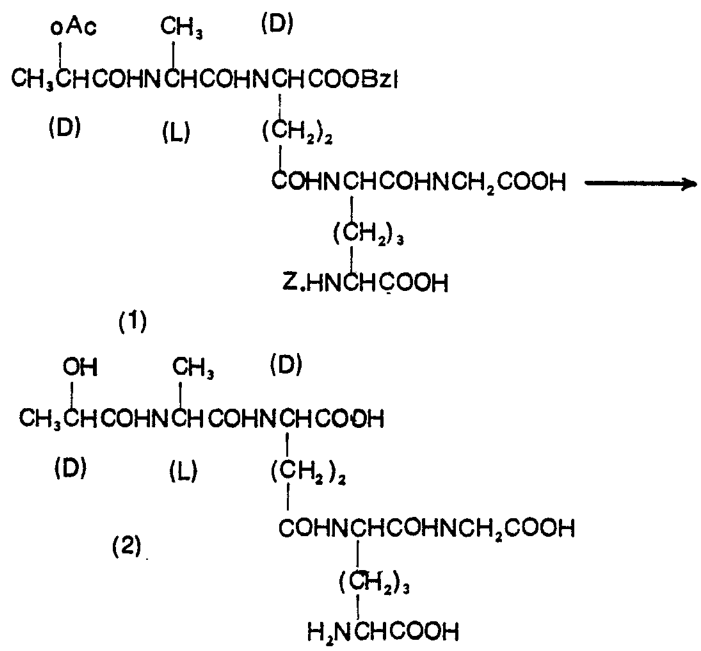

(1)

(2)

D-Lac(oAc)-L-Ala-γ-D-Glu(α-oBzl)-ZDAP(OH)GlyOH (1) (2.80 g) was hydrogenated in acetic acid (15 ml) over 10% palladium black (0.50 g) under two atmospheric pressure of hydrogen for six hours.

The reaction mixture was filtered and the filtrate was concentrated to give an oil. The oil was dissolved in water and the solution was adjusted to pH 9.0—9.5 with diluted aqueous solution of potassium carbonate, and then stirred under ice-cooling for four hours. The reaction mixture was adjusted to pH 7 with dil. hydrochloric acid and concentrated. The concentrate was dissolved in methanol and insoluble materials were filtered off. The methanolic filtrate was evaporated to dryness under reduced pressure, and the residue was dissolved in water (3 ml) adjusted to pH 3—3.2, passed through a column packed with a macroporous non-ionic adsorption resin, HP20 (200 ml) and then eluted with water. The fractions containing the object compound were collected and concentrated to give a powder (400 mg). The powder was dissolved in water and the solution was again passed through a column packed with macroporous non-ionic adsorption resin, HP20 (100 ml). Elution was carried out with water and the fraction containing the object compound was collected and concentrated to give D-Lac-L-Ala-γ-D-Glu(α-OH)-DAP(OH)GlyOH (2) (100 mg), whose structure was confirmed in comparison of data of the products prepared in Example 8-1.

Example 10

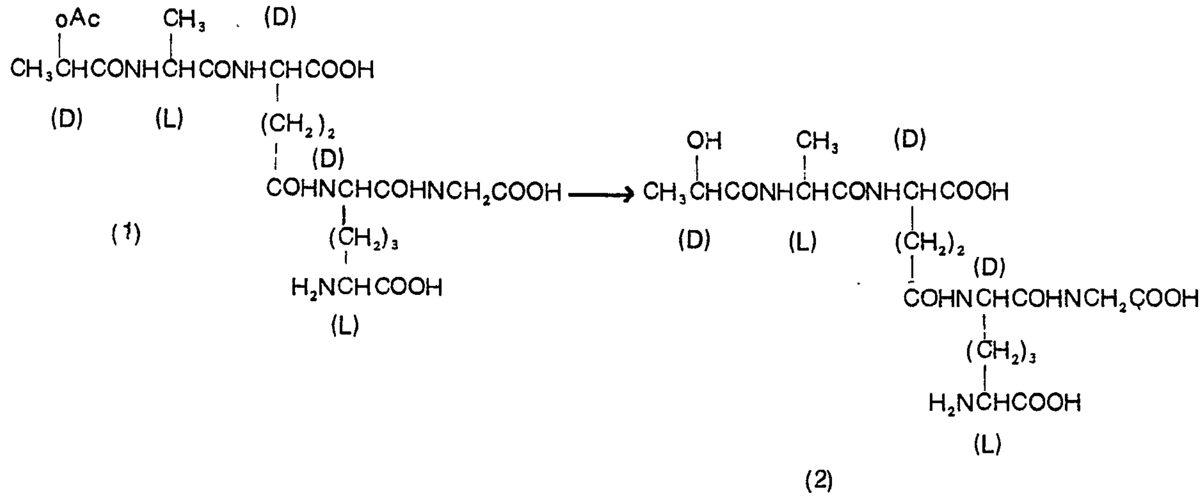

(1) (2)

5% Aqueous potassium bicarbonate (3.25 ml) was added to a solution of D-Lac(oAc)-L-Ala-γ-D-Glu(α-OH)-(D)-mesoDAP(D)GlyOH (1) (278 mg) in 5% aqueous methanol (6 ml) at ambient temperature. The mixture was stirred at ambient temperature with occasional addition of 5% aqueous potassium bicarbonate in order to maintain pH 9.

The reaction mixture was acidified to pH 3.5 with 1N aqueous hydrochloric acid and evaporated to dryness. The residue was dissolved in water and the solution was concentrated to about 2 ml.

The concentrate was chromatographed on macroporous non-ionic adsorption resin, HP20 (15 ml) and elution was carried out with water. The fraction containing the object compound was evaporated, and the residue was dissolved in a small amount of water and lyophilized to give D-Lac-L-Ala-γ-D-Glu(α-OH)-(D)-mesoDAP-(D)-GlyOH (135 mg), whose structure was confirmed in comparison of data of the products prepared in Example 8-1.

Example 11

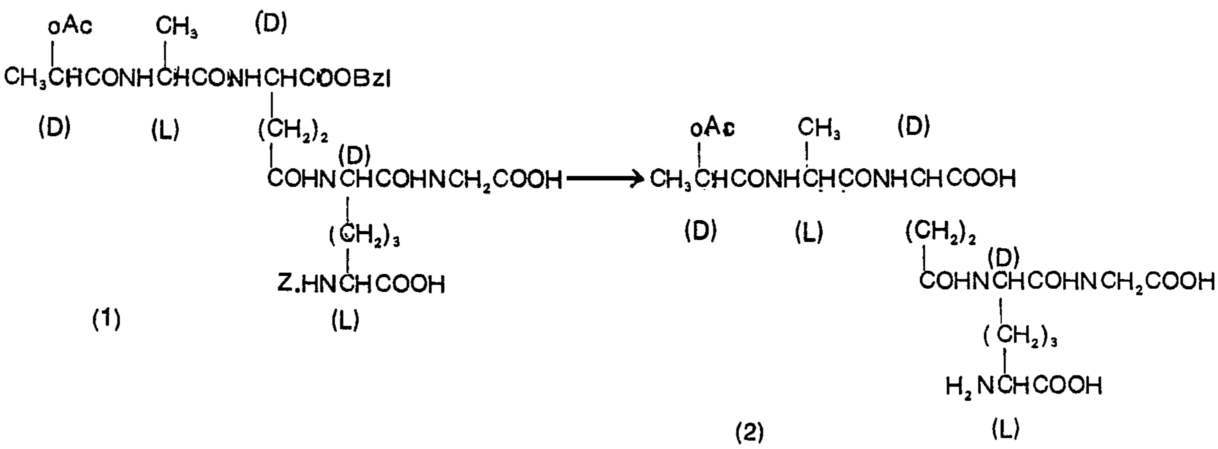

(1) (2)

A mixture of D-Lac(oAc)-L-Ala-γ-D-Glu(α-oBzl)-(D)-Z-(L)-mesoDAP-(D)-GlyOH (1) (440 mg) and 10% palladium black (90 mg) in acetic acid (5 ml) was stirred under hydrogen atmosphere for four hours.

Additional 10% palladium black (60 mg) and acetic acid (3 ml) were added to the reaction mixture and the stirring was continued under hydrogen atmosphere for four hours. The reaction mixture was filtered. The filtrate was concentrated under reduced pressure and the residue was dissolved in water. The aqueous solution was evaporated to dryness. The residue was again dissolved in water and concentrated. The concentrate was lyophilized to give D-Lac(oAc)-L-Ala-γ-D-Glu(α-OH)-(D)-meso-DAP(D)GlyOH (2) (301 mg).

N.M.R. ($D_2O$), δ (ppm): 1.42 (3H, d, J=7Hz), 1.47 (3H, d, J=7Hz), 2.14 (3H, s), 1.0—2.8 (10H, m), 3.80 (1H, t, J=7Hz), 3.94 (2H, s), 4.1—4.6 (3H, m), 5.02 (1H, q, J=7Hz)

Example 12

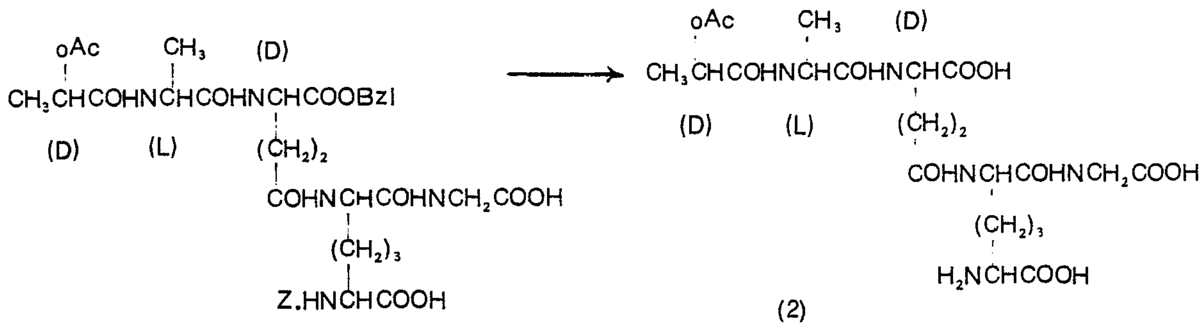

(1) (2)

A solution of D-Lac(oAc)-L-Ala-γ-D-Glu(α-oBzl)-Z-DAP(OH)GlyOH in methanol (1) (20 mg) was hydrogenated over 10% palladium black (50 mg). The reaction mixture was filtered and the filtrate was evaporated to dryness to give D-Lac(oAc)-L-Ala-γ-D-Glu(α-OH)-DAP(OH)GlyOH (2) (10 mg), whose structure was confirmed in comparison of data of the products prepared in Example 11.

Example 13

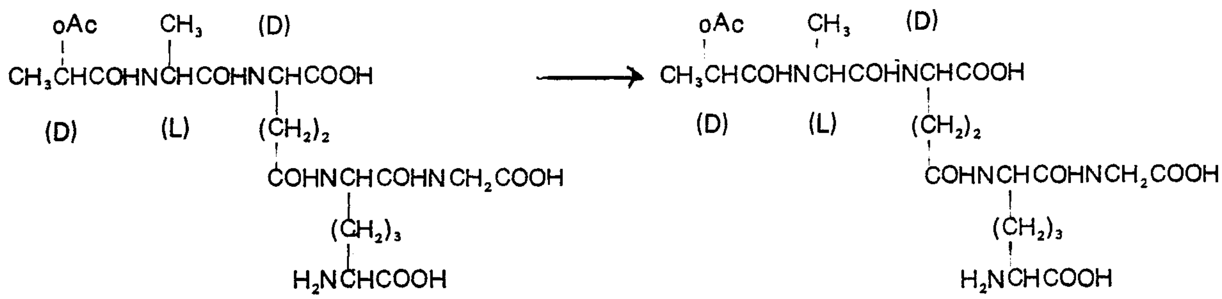

(1) (2)

D-Lac(oAc)-L-Ala-γ-D-Glu(α-OH)-DAP(OH)GlyOH (1) (10 mg) was added to 0.05 M solution of potassium carbonate in 70% methanol (0.6 ml) at 0°C and the mixture was stirred at ambient temperature for an hour. The reaction mixture was neutralized with acetic acid and then evaporated to give a residue. The residue was purified by using carbon column chromatography. Elution was carried out with 50% aqueous acetone to give D-Lac-L-Ala-γ-D-Glu(α-OH)-DAP(OH)GlyOH (2) (7 mg), whose structure was confirmed in comparison of data of the products prepared in Example 8-1.

Example 14

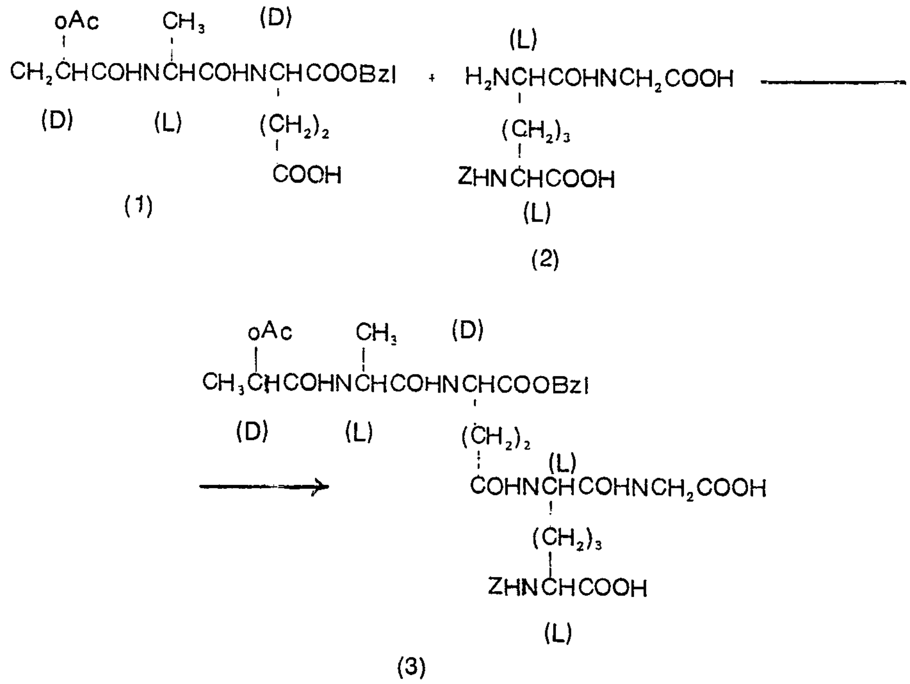

(1) (2) (3)

To a mixture of D-Lac(oAc)-L-Ala-D-Glu(OH)oBzl (1) (2.45 g) and succinic acid (740 mg) in dioxane (20 ml) was added N,N'-dicyclohexylcarbodimido (1.25 g) at 12°C and the mixture was stirred at ambient temperature for 16 hours. The reaction mixture was filtered and N,N'-dicyclohexylurea was washed with dioxane.

The filtrate and the washings were combined and concentrated. The concentrate was dissolved in dioxane (18 ml). This solution was used for the next reaction.

To the mixture of Z-($\alpha$)-L-DAP-($\varepsilon$)-GlyOH (2) (1.21 g) and N-methylmorpholine (1.02 ml) in water (20 ml) was added the above solution at 5°C and the mixture was stirred at 5°C for 40 minutes and at ambient temperature for 4 hours. An additional N-methylmorpholine (0.2 ml) was added to the reaction mixture and the resulting mixture was stirred at the same temperature for two hours. The reaction mixture was concentrated and the concentrate was diluted with water. The solution was washed with ether and acidified with dil. hydrochloric acid to pH 2. To the solution was added a mixture of methylene chloride and ethyl acetate and the mixture was shaken and then filtered to give insoluble materials and the organic layer. The organic layer was washed with water, dried over magnesium sulfate and concentrated. The residue was combined with the insoluble materials described above and dissolved in a mixture of chloroform and methanol (1:1). The solution was concentrated and the concentrate was triturated with a mixture of ethyl acetate and ether (1:2) to give a powder (1.8 g), which was dissolved in a mixture of chloroform and methanol and ethyl acetate (1:1:1) and concentrated. The concentrate was triturated with a mixture of ethyl acetate and ether (5:1) to give D-Lac(oAc)-L-Ala-$\gamma$-D-Glu($\alpha$-oBzl)-($\alpha$)-Z-($\varepsilon$)-L-DAP-($\alpha$)-GlyOH (3) (1.654 g).

N.M.R. ($CDCl_3$-$CD_3OD$), $\delta$ (ppm): 1.37 (3H, d, J=7Hz), 1.46 (3H, d, J=7Hz), 2.11 (3H, s), 1.2—2.6 (10H, m), 3.95 (2H, s), 4.0—4.6 (4H, m), 4.96 (1H, q, J=7Hz), 5.08 (2H, s), 5.15 (2H, s), 7.33 (10H, s).

Example 15

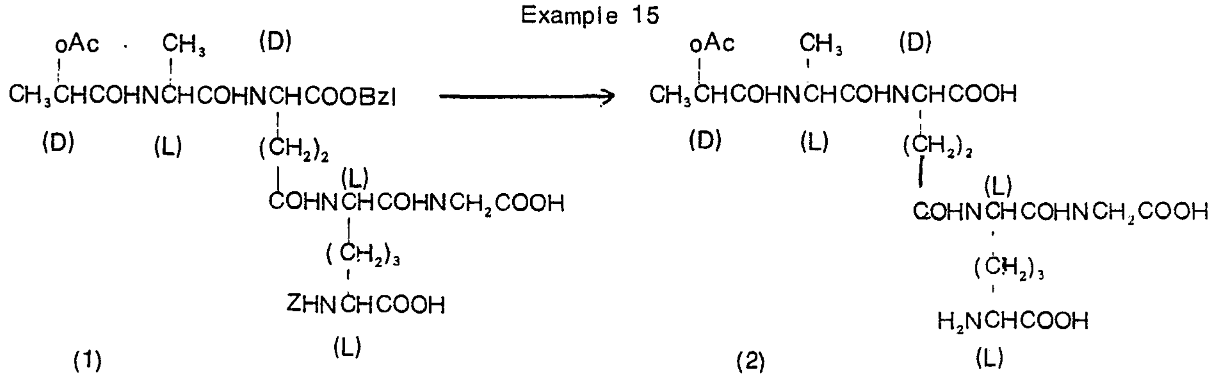

(1) (2)

A solution of D-Lac(oAc)-L-Ala-$\gamma$-D-Glu($\alpha$-oBzl)-($\alpha$)-Z-($\varepsilon$)-L-DAP-($\alpha$)-GlyOH (1) (1.59 g) in acetic acid (40 ml) was hydrogenated over 10% palladium black (500 mg) for 4 hours. The reaction mixture was filtered and the filter cake was washed with 50% aqueous methanol. The filtrate and the washings were combined and evaporated and pumped to give a residue (1.5 g). Contaminated acetic acid was removed by co-evaporation with water and this operation was repeated once more. The residue was dissolved in water and lyophilized to give D-Lac(oAc)-L-Ala-$\gamma$-D-Glu($\alpha$-OH)-($\alpha$)-L-DAP-($\alpha$)-GlyOH (2) (1.10 g).

N.M.R. ($D_2O$), $\delta$ (ppm): 1.43 (3H, d, J=7Hz), 1.48 (3H, d, J=7Hz), 1.3—2.7 (10H, m), 2.15 (3H, s), 3.80 (1H, broad t, J=6Hz), 3.95 (2H, s), 4.0—4.6 (3H, m), 5.05 (1H, q, J=7Hz)

Example 16

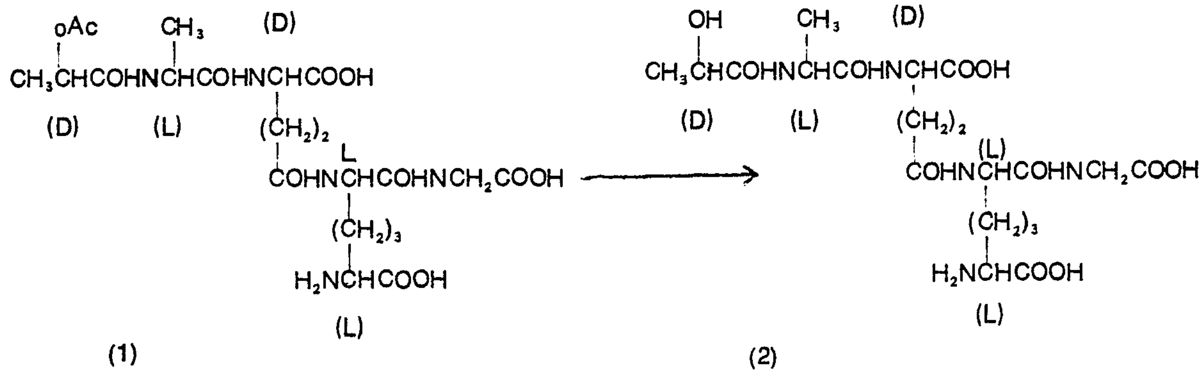

(1) (2)

To a solution of D-Lac(oAc)-L-Ala-$\gamma$-D-Glu($\alpha$-OH)-($\alpha$)-L-DAP-($\alpha$)-GlyOH (1) in a mixture of methanol (15 ml) and water (5 ml) was added 1N sodium hydroxide (7.5 ml) at 5°C. The solution was stirred at the same temperature for 30 minutes and at ambient temperature for 2.25 hours.

The resulting solution was acidified to pH 4 with 3N hydrochloric acid and concentrated. The concentrate was diluted with water to 10 ml, acidified to pH 2.2 with 3N hydrochloric acid and chromatographed on a column of a macroporous non-ionic adsorption resin, HP20 (135 ml). Elution was carried out with water and the eluate was concentrated and lyophilized to give D-Lac-L-Ala-$\gamma$-D-Glu($\alpha$-OH)-($\alpha$)-L-DAP-($\alpha$)-GlyOH (2) (681 mg). mp. 130°C (dec.).

N.M.R. ($D_2O$), $\delta$ (ppm): 1.39 (3H, d, J=7Hz), 1.45 (3H, d, J=7Hz), 1.1—2.6 (10H, m), 3.86 (1H, t, J=6Hz), 4.00 (2H, s), 4.1—4.6 (4H, m)

$[\alpha]_D^{15} = -23.3°$ (C=0.322, water)

Example 17

(1) Step 1

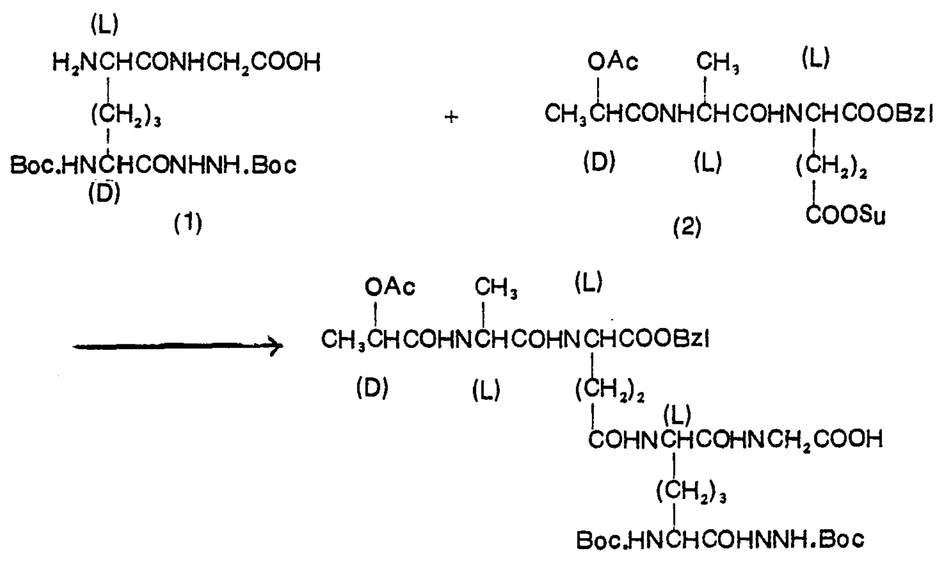

(1) (2)

(3)

D-Lac(OAc)-L-Ala-$\gamma$-L-Glu($\alpha$-OBzl)-(L)-Boc-(D)-meso-DAP(L)-GlyOH-(D)-NHNHBoc (3) was prepared from Boc(D)-meso-DAP-(L)GlyOH-(D)-NHNHBoc (1) and D-Lac(OAc)-L-Ala-$\gamma$-L-Glu($\alpha$-OBzl) (2) in substantially the same manner as that of Example 1-1.

IR (Nujol): 3300, 1735, 1695, 1645 $cm^{-1}$

N.M.R. ($CD_3OD$): 1.40 (9H, S), 1.42 (9H, S), 2.08 (3H, S), 3.90 (2H, broad S), 4.00~5.00 (5H, m), 5.10 (2H, S), 7.30 (5H, S)

(2) Step 2

Compound (3)

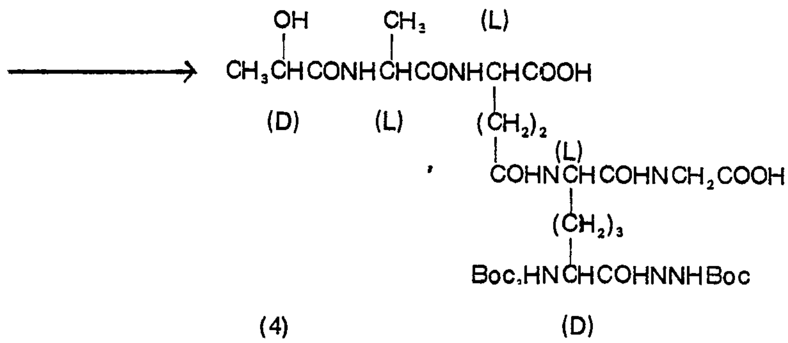

(4)

D-Lac(OH)-L-Ala-$\gamma$-L-Glu($\alpha$-OH)-(L)-Boc-(D)-meso-DAP-(L)-GlyOH-(D)-NHNHBoc (4) was prepared in substantially the same manner as that of Example 7.

N.M.R. ($CD_3OD$), $\delta$ (ppm): 1.44 (9H, S), 1.46 (9H, S), 3.90 (2H, broad S), 4.00~4.60 (5H, m)

(3) Step 3

Compound (4)

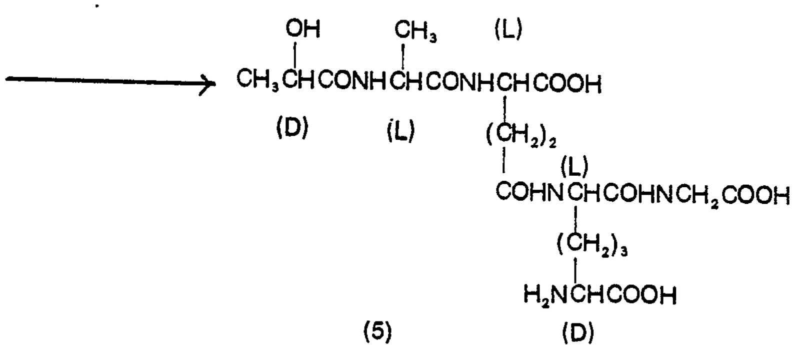

(5)

D-Lac(OH)-L-Ala-$\gamma$-L-Glu($\alpha$-OH)-(L)-meso-DAP(L)GlyOH (5) was prepared in substantially the same manner as that of Example 8-1.

N.M.R. ($D_2O$), $\delta$ (ppm): 1.38 (3H, d, J=7Hz), 1.00~2.60 (10H, m), 3.98 (2H, S), 3.84 (1H, t, J=7Hz), 4.1~4.5 (4H, m)

$[\alpha]_D = -34.7$ (c=0.15 water)

Example 18

(1) Step 1

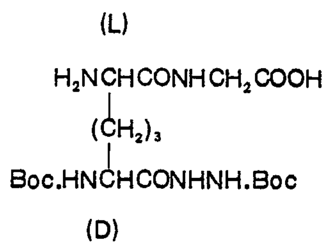

+

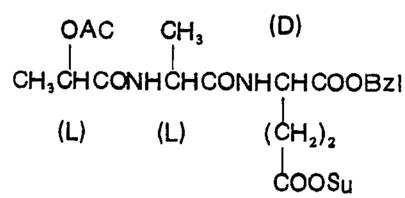

(1) (2)

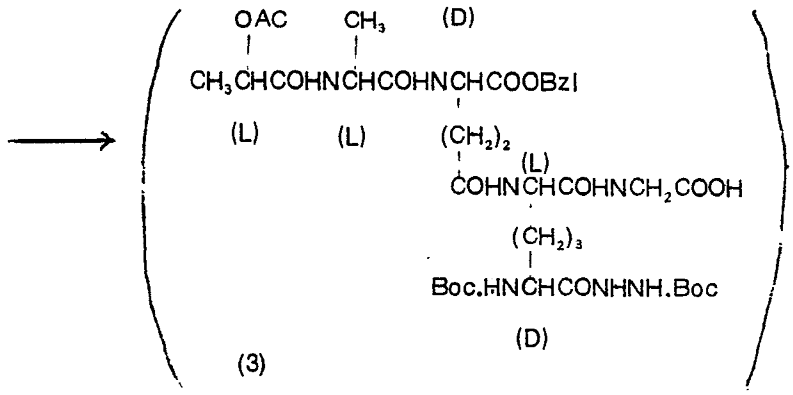

(3)

L-Lac(OAc)-L-Ala-γ-D-Glu(α-oBzl)-(L)-Boc-(D)-meso-DAP(L)-GlyOH-(D)-NHNHBoc (3) was prepared from Boc(D)-mesoDAP-(L)-GlyOH-(D)-NHNHBoc (1) and L-Lac(OAc)-L-Ala-γ-D-Glu(α-OBzl) (2) in substantially the same manner as that of Example 1-1.

(2) Step 2

Compound (3)

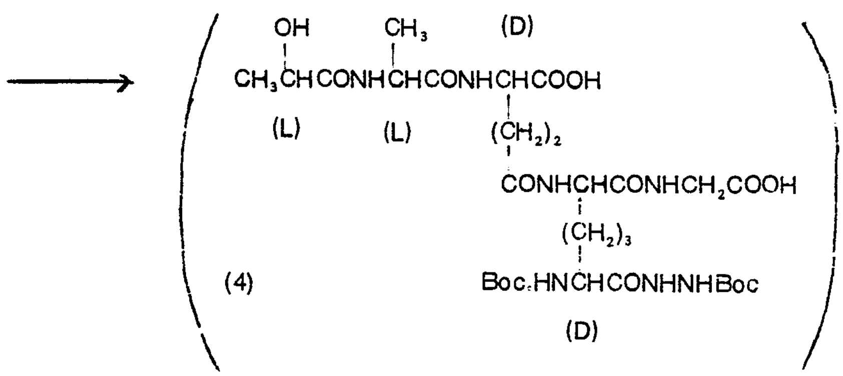

(4)

L-Lac(OH)-L-Ala-γ-D-Glu(α-OH)-(L)-Boc-(D)-meso-DAP(L)-GlyOH-(D)-NHNHBoc (4) was prepared in substantially the same manner as that of Exampe 7.

Step (3)

Compound (4)

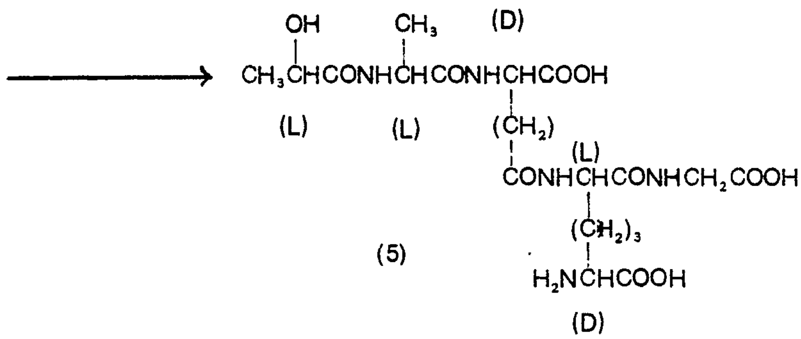

(5)

L-Lac(OH)-L-Ala-γ-D-Glu(α-OH)-(L)-meso-DAP(L)GlyOH (5) was prepared in substantially the same manner as that of Example 8-1.

mp 170~174°C (dec)

$[\alpha]_D = -33.2°$ (C=0.25, water)

N.M.R. ($D_2O$), δ (ppm): 1.38 (3H, J=7Hz), 1.45 (3H, J=7Hz), 3.89 (1H, t, J=7Hz), 4.01 (2H, s), 4.20 4.55 (m).

## Example 19

(1) Step 1

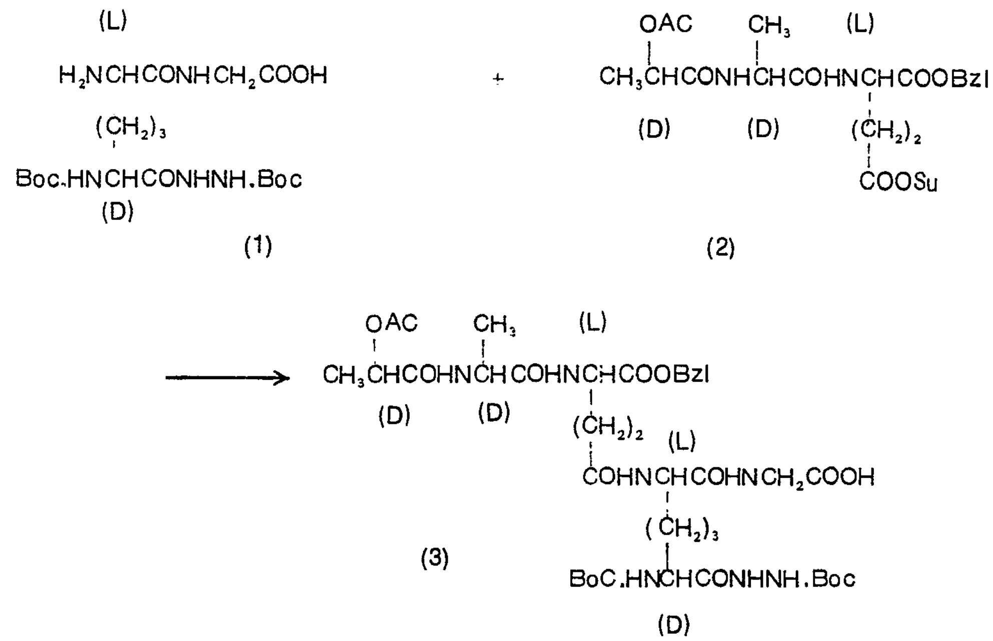

(1) (2)

(3)

D-Lac(OAc)-D-Ala-$\gamma$-L-Glu($\alpha$)OBzl)-(L)-Boc-(D)-meso-DAP(L)-GlyOH-(D)-NHNHBoc (3) was prepared from Boc(D)-mesoDAP-(L)-GlyOH-(D)-NHNHBoc (1) and D-Lac(OAc)-D-Ala-$\gamma$-L-Glu($\alpha$-OBzl) (2) in substantially the same manner as that of Example 1-1.

N.M.R. ($CD_3OD$), $\delta$ (ppm): 1.40 (9H, s), 1.42 (9H, s), 2.06 (3H, s), 3.90 (2H, broad s), 3.90~5.00 (5H, m), 5.15 (2H, s), 7.35 (5H, s)

(2) Step 2

Compound (3)

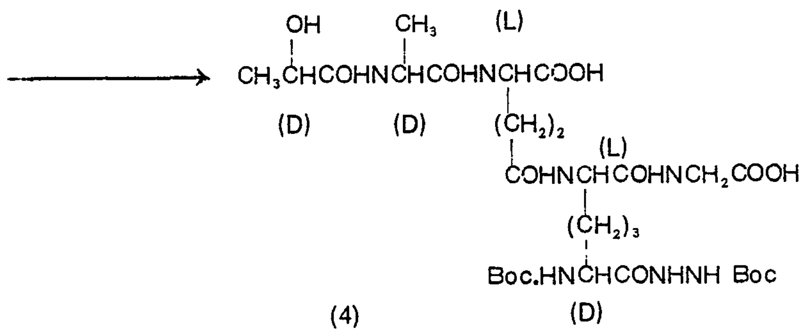

(4)

D-Lac(OH)-D-Ala-$\gamma$-L-Glu($\alpha$-OH)-(L)-Boc-(D)-meso-DAP(L)-GlyOH-(D)-NHNHBoc (4) was prepared in substantially the same manner as that of Example 7.

IR (Nujol): 3300, 1740, 1690 1740 (Broad) $cm^{-1}$)

N.M.R. ($CD_3OD$), $\delta$ (ppm): 3.90 (2H, broad s), 4.00~4.60 (5H, m)

(3) Step 3

Compound (4) 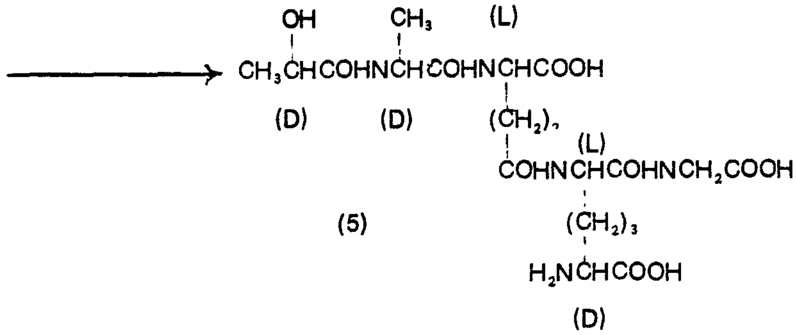

(5)

D-Lac(OH)-D-Ala-$\gamma$-L-Glu($\alpha$-OH)-(L)-mesoDAP(L)GlyOH (5) was prepared in substantially the same manner as that of Example 8-1.

N.M.R. ($D_2O$), $\delta$ (ppm): 1.35 (3H, d, J=7Hz), 1.40 (3H, d, J=7Hz), 1.00~2.60 (10H, m), 3.80 (1H, t, J=7Hz), 3.90 (2H, s), 4.10~4.5 (4H, m)
$[\alpha]_D = +0.76$ (c=0.35, water)

## Example 20

(1) Step 1

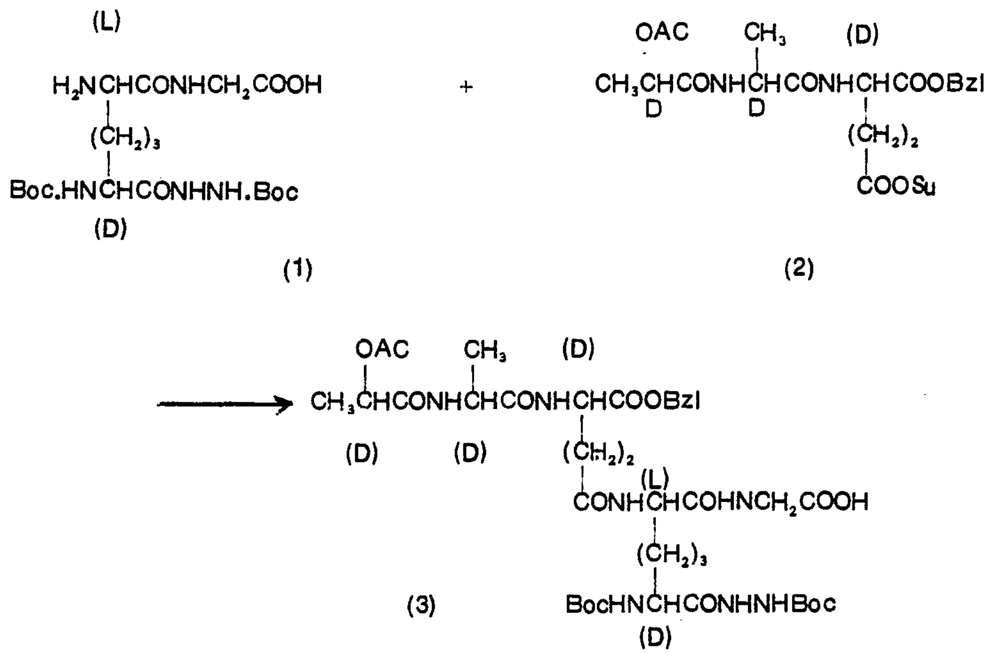

(1) + (2) → (3)

D-Lac(OAc)-D-Ala-$\gamma$-D-Glu($\alpha$-oBzl)-(L)-Boc-(D)-mesoDAP-(D)-NHNHBoc-(L)-GlyOH (3) was prepared from Boc(D)-mesoDAP-(D)-NHNHBoc-(L)-GlyOH (1) and D-Lac(oAc)-D-Ala-$\gamma$-D-Glu($\alpha$-OBzl) (2) in substantially the same manner as that of Example 1-1.

N.M.R. ($CD_3OD$), $\delta$ (ppm): 1.41 (18H, s), 2.12 (3H, s), 3.92 (2H, broad s), 4.00—5.10 (5H, m), 5.16 (2H, s), 7.36 (5H, s)

(2) Step 2

Compound (3) 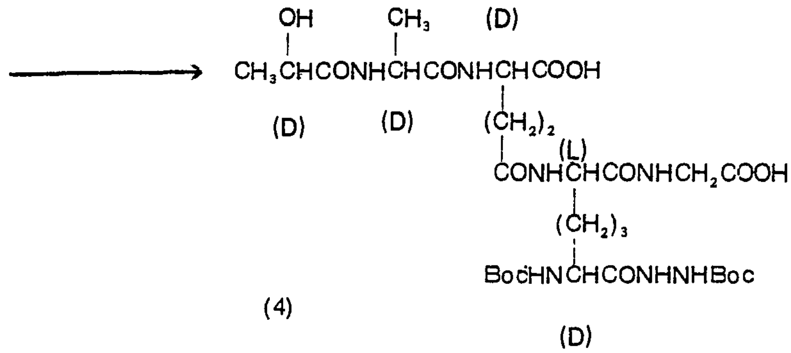

(4)

D-Lac(OH)-D-Ala-γ-D-Glu(α-OH)-(L)-Boc-(D)-mesoDAP-(D)-NHNHBoc-(L)-GlyOH (4) was prepared in substantially the same manner as that of Example 7.

N.M.R. ($CD_3OD$), δ (ppm): 1.41 (18H, s), 3.92 (2H, broad s), 4.00—4.60 (5H, m)

(3) Step 3

Compound (4) 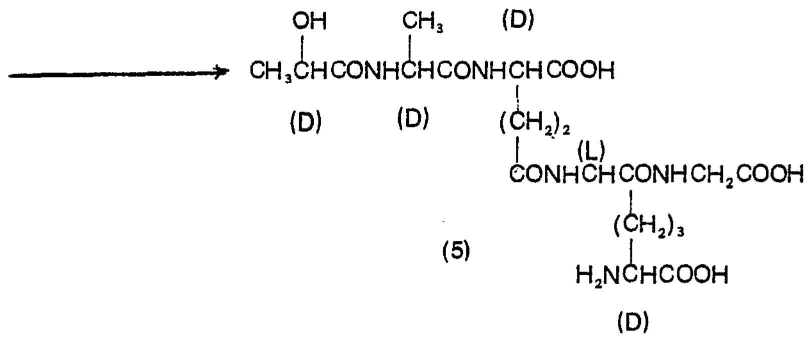

(5)

D-Lac(OH)-D-Ala-γ-D-Glu(α-OH)-(L)-mesoDAP-(L)-GlyOH (5) was prepared in substantially the same manner as that of Example 8-1.

$[\alpha]_D = 11.6$ (c=0.25, water)

N.M.R. ($CD_3OD$), δ (ppm): 1.36 (3H, d, J=7Hz), 1.44 (3H, d, J=7Hz), 1.00—2.60 (10H, m), 3.88 (1H, t, J=7Hz), 3.98 (2H, broad s), 4.10—4.50 (5H, m).

## Example 21

For fermentation:

(1) A vegetative medium 1 (pH 7.0) was prepared from the following ingredients:

Vegetative medium 1

| | |
|---|---|
| Soluble starch | 2% by wt. |
| Gluten meal | 1% by wt. |
| Dried yeast | 1% by wt. |
| Corn Steep Liquor | 1% by wt. |
| Tap water | q.s. |

100 ml. of the medium 1 in each of ten 500 ml. flasks were sterilised in conventional manner and then inocualted with a loopful of culture from a stock slant of Streptomyces olivaceogri ATCC 31427. The organism was grown in the medium at 30°C for 48 hours on a shaker.

Into a 30-litre Jar-fermentor, there were placed 20 litres of the vegetative medium 2 prepared from the following ingredients:

Vegetative medium 2

| | |
|---|---|
| Soluble starch | 2 % by wt. |
| Cottonseed meal | 0.5% by wt. |
| Gluten meal | 0.5% by wt. |
| Dried yeast | 0.5% by wt. |
| Corn Steep Liquor | 0.5% by wt. |
| Tap water | q.s. |

The vegetative medium 2 (pH 7.0) was sterilised in a conventional manner and then inoculated aseptically with the whole volume of the vegetative inoculum culture prepared above. The organism was grown in the medium 2 at 30°C for 24 hours.

The whole volume of the vegetative inoculum thus prepared was aseptically inoculated into a 2000-litre fermentor, containing 1600 litres of the fermentation medium prepared from the following ingredients:

Fermentation medium

| | |
|---|---|
| Soluble starch | 2 % by wt. |
| Cottonseed meal | 0.5% by wt. |
| Wheat germ | 0.5% by wt. |
| Dried yeast | 0.25% by wt. |
| Corn Steep Liquor | 0.25% by wt. |
| $KH_2PO_4$ | 0.5% by wt. |
| $Na_2HPO_4.12H_2O$ | 0.5% by wt. |
| $CoCl_2.6H_2O$ | 1.25 mg/l |
| Tap water | q.s. |

The organism was cultured in the fermentation medium for 72 hours at 30°C. During the growth period, the broth was stirred with a propeller operating at 170 r.p.m. and sterile air was passed through the broth at a rate of 1600 litres per minute.

After the fermentation was completed, 20 kg "Radiolite" (trade name, a filter aid material sold by Showa Chemical Company, Japan) was added to the culture broth and the mixture was filtered to remove mycelia. 1600 litres of the filtrate was passed through a column of activated charcoal (800 litres) and then was washed with 1600 litres of water. Elution was carried out with 3000 litres of 50% aqueous acetone and then the eluate was concentrated to a volume of about 600 litres.

The concentrate was passed through a column of DEAE-Sephadex (trade name, made by Pharmacia A.B.) (200 litres) which has previously been buffered with phosphate buffer (pH 6.0). The column was successively washed with 200 litres of water and 200 litres of 0.1 M sodium chloride solution and then eluted with 400 litres of 0.3 M sodium chloride solution. The aqueous eluate was passed through a column of an activated charcoal (200 l), washed with 200 litres of water and then eluted with 400 litres of 50% aqueous acetone. The eluate was concentrated and then freeze-dried to give 800 g of a white powder. The powder was dissolved into 25 litres of water and the solution was passed through a column of CM-Sephadex ($H^+$ form) (25 l). The column was eluted with 25 litres of water and the eluate was concentrated and then freeze-dried to give 33 g. of yellowish white powder. The powder was placed on the top of a column of cellulose (1.2 l). The column was washed with 1000 ml. of 70% aqueous propanol and then eluted with 1000 ml. of 60% aqueous propanol. The eluate was concentrated and freeze-dried to give 4 g. of white powder. The powder was dissolved into 150 ml. of water and then the solution was subjected to column chromatography on Sephadex G-15 (2.8 l).

The column was developed and eluted with water. The active fractions were collected and concentrated and then freeze-dried to give 4 g. of a white powder. The powder dissolved into 25 ml. of water and the solution was subjected to column chromatography on CM-Sephadex ($H^+$ form) (400 ml).

The column was developed and eluted with water. The active fractions were collected, concentrated and then freeze-dried to give 40 mg. of the FR-900156 substance in the form white powders (purity: about 70%).

(2) Fermentation was carried out in the same manner as described in Example 21 (1). After the fermentation was completed, 20kg. "Radiolite" (trade name, a filter aid material sold by Showa Chemical Company) was added to the culture broth and the mixture was filtered to remove mycelia. 1600 Litres of the filtrate was passed through a column of activated charcoal (800 litres) and then washed with 1600 litres of water. Elution was carried out with 3000 litres of 50% aqueous acetone and then the eluate was concentrated to a volume of about 600 litres. The concentrate was passed through a column of DEAE-Sephadex (trade name, made by Pharmacia A.B.) (200 litres) which has previously been buffered with phosphate buffer (pH 6.0). The column was successively washed with 200 litres of water and 200 litres of 0.1 M sodium chloride solution and then eluted with 400 litres of 0.3 M sodium chloride solution. The aqueous eluate was passed through a column of an activated charcoal (200 litres), washed with 200 litres of water and then eluted with 400 litres of 50% aqueous acetone. The eluate was concentrated and then freeze-dried to give 800 g. of white powder. The powder was dissolved into 25 litres of water and the solution was passed through a column of CM-Sephadex ($H^+$ form) (25 litres). The column was eluted with 25 litres of water and the eluate was concentrated and then freeze-dried to give 33 g. of yellowish white powders. The powders were placed on top of a column of cellulose (1.2 litres). The column was washed with 1000 ml. of 70% aqueous propanol and then eluted with 1000 ml. of 60% aqueous propanol. The eluate was concentrated and freeze-dried to give 4 g. of white powder. The powder was dissolved into 300 ml. of water and then passed through a column of DEAE-Sephadex (trade name, made by Pharmacia A.B.) (1.4 litres) which has previously been buffered with phosphate buffer (pH 6.0). The column was washed with 1.5 litres of 0.1 M sodium chloride solution and then eluted with 3 litres of 0.2 M sodium chloride solution. The active fractions were collected and then passed through a column of activated charcoal (300 ml). The column was washed with water and then eluated with 800 ml. of 50% aqueous acetone. The eluate was concentrated and then freeze-dried to give 700 mg. of white powders. The powders were dissolved into 20 ml. of water and then passed through a column of CM-Sephadex ($H^+$ form) (500 ml). The column was eluted with water and the active fractions were collected, and then concentrated to give 10 ml. of concentrate. The concentrate was subjected to a column chromatography on Sephadex G 15 (500 ml) and developed with water. The active fractions were collected and concentrated and then freeze-dried to give 70 mg. of white powders. The powders were dissolved into 25 ml. of water and subjected to preparative thin layer chromatography on cellulose (made by Eastman Kodak Co.): a developing solvent was mixture of butanol, acetic acid and water (4:1:2). Elution was carried out with 50 ml. of water and the eluate was concentrated and then freeze-dried to give 20 mg of the FR-900156 substance in the form of a white powder.

(3) Fermentation was carried out in the same manner as described in Example 21 (1). After the fermentation was completed, 20 kg. "Radiolite" (trade name, a filter aid material sold by Showa Chemical Company) was added to the culture broth and the mixture was filtered to remove mycelia. 1600 Litres of the filtrate was passed through a column of activated charcoal (800 litres) and then was washed with 1600 litres of water. Elution was carried out with 3000 litres of 50% aqueous acetone and then the eluate was concentrated to a volume of about 600 litres. The concentrate was passed through a column of DEAE-Sephadex (trade name, made by Pharmacia A.B.) (200 litres) which has previously been buffered with phosphate buffer (pH 6.0). The column was successively washed with 200 litres of water and 200 litres of 0.1 M sodium chloride solution and then eluted with 400 litres of 0.3 M sodium chloride solution. The aqueous eluate was passed through a column of an activated charcoal (200 litres), washed with 200 litres of water and then eluted with 400 litres of 50% aqueous acetone. The eluate was concentrated and then freeze-dried to give 800 g. of white powder. The powder was dissolved into 25 litres of water and the solution was passed through a column of CM-Sephadex ($H^+$ form) (25 litres). The column was eluted with 25 litres of water and the eluate was concentrated and then freeze-dried to give 33 g. of yellowish white powder. The powder was placed on top of a column of cellulose (1.2 litres). The column was washed with 1000 ml. of 70% aqueous propanol and then eluted with 1000 ml. of 60% aqueous propanol. The eluate was concentrated and freeze-dried to give 4 g. of white powder. The powder was dissolved into 300 ml. of water and then passed through a column of DEAE-Sephadex (trade name, made by Pharmacia A.B.) (1.4 litres) which has previously been buffered with phosphate buffer (pH 6.0). The column was washed with 1.5 litres of 0.1 M sodium chloride solution and then eluted with 300 litres of 0.2 M sodium chloride solution. The active fractions were collected and then passed through a column of activated charcoal (300 ml). The column was washed with water and then eluated with 800 ml. of 50% aqueous acetone. The eluate was concentrated and then freeze-dried to give 700 mg. of white powders. The powders were dissolved into 20 ml. of water and then passed through a column of CM-Sephadex ($H^+$ form) (500 ml). The column was eluted with water and the active fractions were collected, and then concentrated to give 10 ml. of concentrate. The concentrate was freeze-dried to give 400 mg of a powder. The powder was placed on the top of a column of cellulose (500 ml). Elution was carried out with a mixture of n-butanol, acetic acid and water (4:1:2). The active fractions were collected and freeze-dried to give 200

mg of a powder. The powder was dissolved into 7 ml. of water and then placed on Sephadex G 15 (250 ml). The active fractions were collected, concentrated and then freeze-dried to give 100 mg of the FR-900156 substance in the form of a white powder.

(4) A white powder of FR-900156 substance obtained by the Example 21 (3) was further purified by conducting repeatedly the above purification means to give a more purified product of FR-900156 substance.

(5) 100 ml of a medium containing corn starch 2% (by wt.), gluten meal 1%, dried yeast 1% and corn steep liquor 1% were poured into each of ten 500 ml flasks and sterilised in a conventional manner and then inoculated with a loopful of culture from a stock slant of Streptomyces violaceus ATCC 31481.

The organism was grown in the medium at 30°C for 48 hours on a shaker.

Into a 30-litres Jar-fermentor, there were placed 20-litres of the same medium as above. The medium was sterilised in a conventional manner and then inoculated aseptically with the whole volume of the inoculum culture prepared above. The organism was grown in the medium at 30°C for 30 hours.

The whole volume of the inoculum thus prepared was aseptically inoculated into a 400-litres fermentor, containing 320 litres of the medium (pH 6.5) containing soluble starch 2% (by wt.), gluten meal 1%, cottonseed meal 1% and sodium sulfate (10 hydrates) 2%.

The organism was cultured in the medium at 30°C for 72 hours. During the growth period, the broth was stirred with a propeller operating at 170 r.p.m. and sterile air was passed through the broth at a rate of 320 litres per minute. After the fermentation was complete, 4 kg of "Radiolite" was added to the cultured broth and the mixture was filtered to remove mycelia. 300 litres of the filtrate was passed through a column of activated charcoal (150 litres) and then washed with 300 litres of water. Elution was carried out with 600 litres of 50% aqueous acetone and then the eluate was concentrated to a volume of about 120 litres.

The concentrate was passed through a column of DEAE-Sephadex (30 litres) which has previously been buffered with phosphate buffer (pH 6.0). The column was successively washed with 30 litres of water and 30 litres of 0.1 M sodium chloride and then eluted with 0.3 M sodium chloride. The eluate (60 litres) was passed through a column of an activated charcoal (30 litres), washed with 60 litres of water and then eluted with 60 litres of 50% aqueous acetone. The eluate was freeze-dried to give 120 g of a white powder. The powder was dissolved in 4 litres of water and the solution was passed through a column of CM-Sephadex ($H^+$ form) (14 litres). The column was eluted with water and the eluate was concentrated and then freeze-dried to give 20 g of a powder. The powder was placed on the top of the column of cellulose. Elution was carried out with an aqueous propanol and the active fractions were collected and freeze-dried to give 5 g of a powder. The powder was dissolved in 500 ml of water and the solution was passed through a column of DEAE Sephadex (1.3 litres) which has previously been buffered with phosphate buffer (pH 6). The column was washed with 0.1 M sodium chloride and eluted with 0.2 M sodium chloride. The active fractions were collected and passed through a column of an activated charcoal (900 ml). The column was washed with water and eluted with 50% aqueous acetone (200 ml). The eluate was concentrated and freeze-dried to give 1 g of a powder.

The powder was dissolved in 300 ml of water and the solution was passed through a column of CM-Sephadex ($H^+$ form) (250 ml). The column was developed and eluted with water. The active fractions were collected, concentrated and then freeze-dried to give 800 mg of a powder. The powder was dissolved in 20 ml of water and the solution was mixed with a small amount of cellulose. The mixture was subjected to column chromatograph on cellulose. The column was washed successively with 100 ml of acetone and 300 ml of mixture of n-butanol: acetic acid: water (4:1:1) and then developed and eluted with a mixture of n-butanol: acetic acid: water (4:1:2) (1 litre). The active fractions were collected and freeze-dried to give 30 mg of a powder. The powder was dissolved in 20 ml of water and the solution was passed through a column of Sephadex G 15 (250 ml). The column was developed and eluted with water and the active fractions were collected and then freeze-dried to give 5 mg of FR-900156 substance.

Example 22

for the pharmaceutical composition:

(1) Preparation for injection

The required quantities of the FR-900156 substance were distributed into vials, each containing 500 mg of the active ingredient. The vials were sealed hermetically to exclude bacteria. Whenever the vial is required for use, 2 ml of sterile distilled water for injection is added to the vial and then the aqueous solution is administered by injection.

(2) Preparation of tablet

A suitable formulation for the tablet consists of the following mixture.

| | |
|---|---|
| FR-900156 substance | 200 mg |
| Mannitol | 400 mg |

| | |
|---|---|
| Starch | 50 mg |
| Magnesium stearate | 10 mg |

(3) Preparation of capsule

| | |
|---|---|
| FR-900156 substance | 300 mg |
| Magnesium stearate | 15 mg |

The above ingredients were mixed and then inserted into a hard gelatin capsule in a conventional manner.

**Claims for the Contracting States: BE, CH, DE, FR, GB, IT, LU, NL, SE**

1. A lactyl tetrapeptide of the formula:

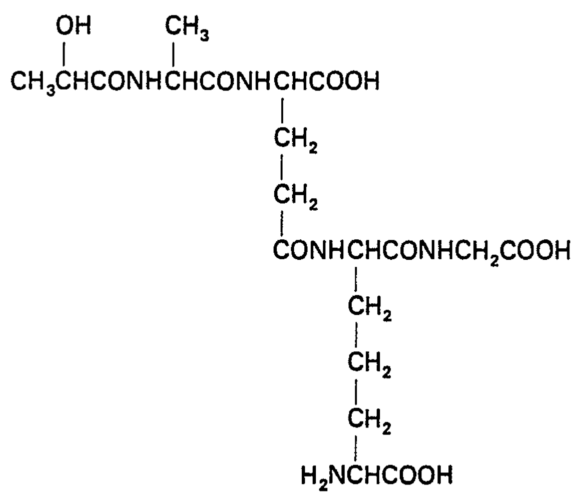

and pharmaceutically acceptable salts thereof.

2. A compound according to claim 1, wherein the compound is represented by the formula:

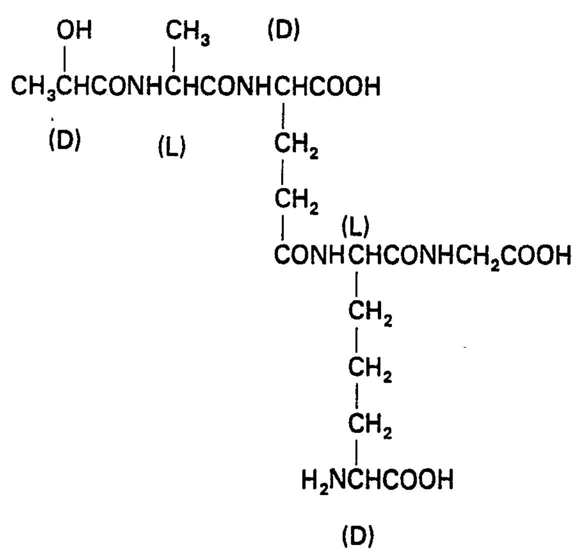

and pharmaceutically acceptable salts thereof.

3. A compound according to claim 1, wherein the compound is represented by the formula:

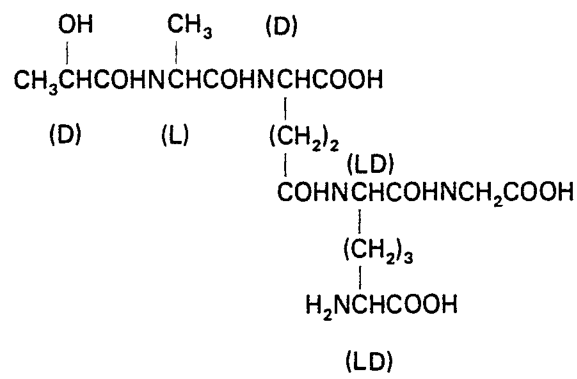

and pharmaceutically acceptable salts thereof.

4. A compound according to claim 1, wherein the compound is represented by the formula:

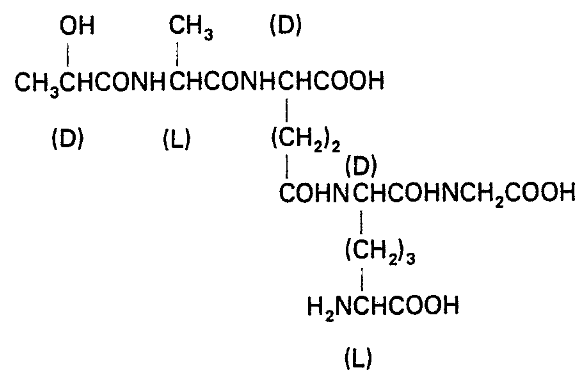

and pharmaceutically acceptable salts thereof.

5. A compound according to claim 1, wherein the compound is represented by the formula:

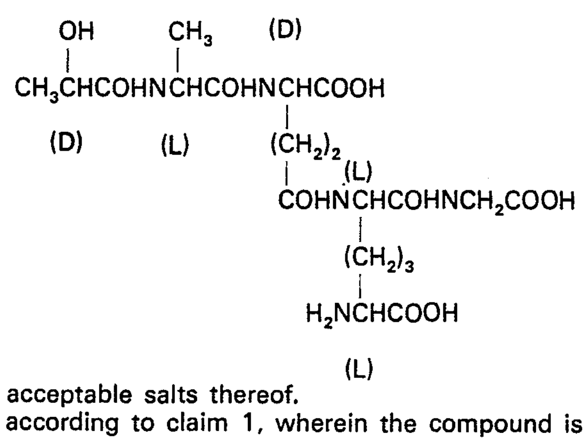

and pharmaceutically acceptable salts thereof.

6. A compound according to claim 1, wherein the compound is represented by the formula:

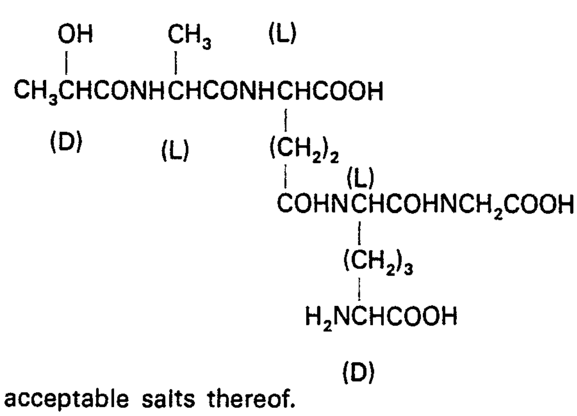

and pharmaceutically acceptable salts thereof.

7. A compound according to claim 1, wherein the compound is represented by the formula:

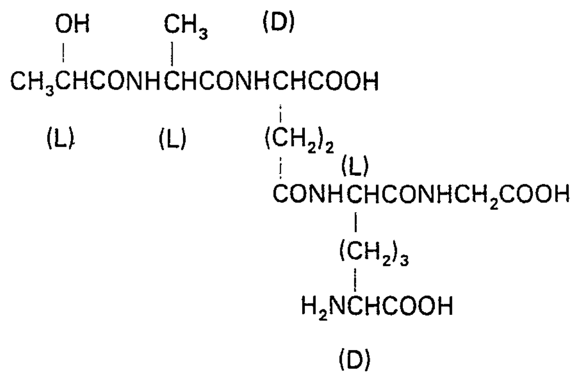

and pharmaceutically acceptable salts thereof.

8. A compound according to claim 1, wherein the compound is represented by the formula:

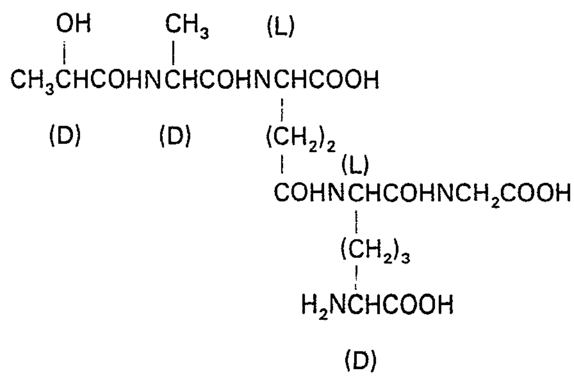

and pharmaceutically acceptable salts thereof.

9. A compound according to claim 1, wherein the compound is represented by the formula:

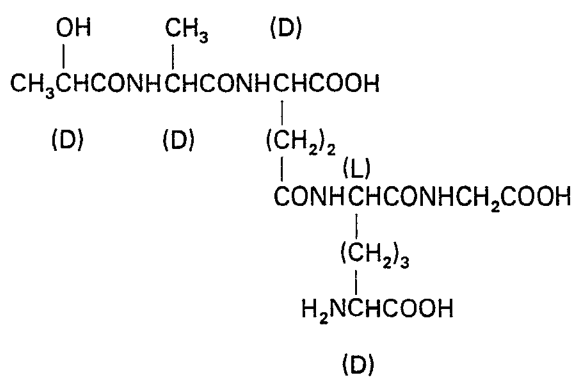

and pharmaceutically acceptable salts thereof.

10. A process for the production of a substance of the formula:

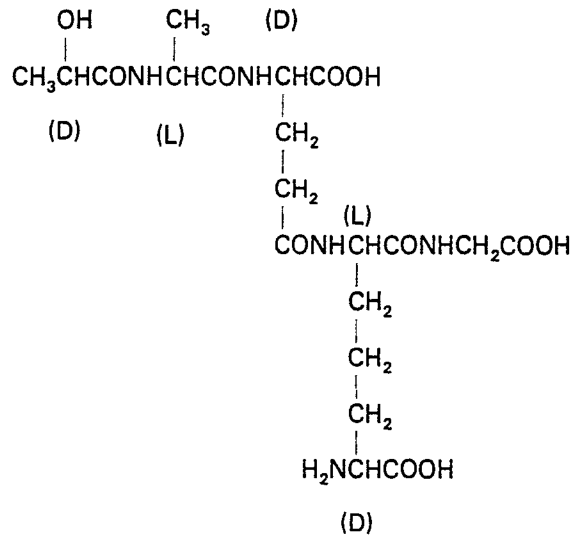

and pharmaceutically acceptable salts thereof which comprises cultivating under aerobic conditions Streptomyces olivaceogrises ATCC 31427 or Streptomyces violaceus ATCC 31481 in a culture medium containing assimilable sources of carbon, nitrogen and inorganic salt.

11. A process for preparation of a compound of the formula or its salt:

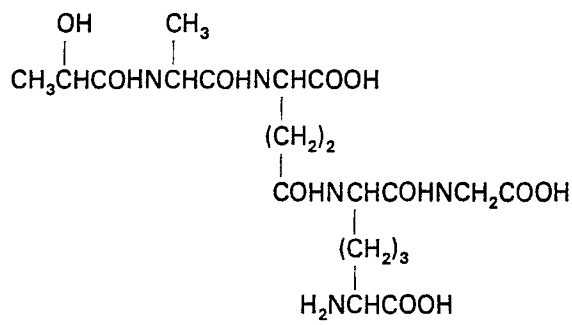

which comprises subjecting a compound of the formula or its salt:

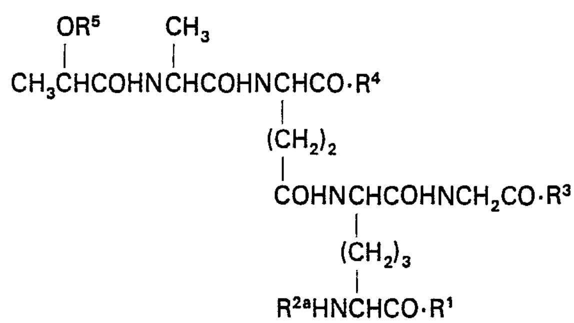

wherein $R^1$ is a hydroxy group, a protected hydroxy group or a protected hydrazino group of the formula: —NHNHY wherein Y is an amino protective group; $R^{2a}$ is an amino protective group; $R^3$ is hydroxy group or a protected hydroxy group; $R^4$ is a protected hydroxy group and $R^5$ is a hydroxy protective group, to elimination reaction of protective groups.

12. A pharmaceutical composition comprising a compound of the formula

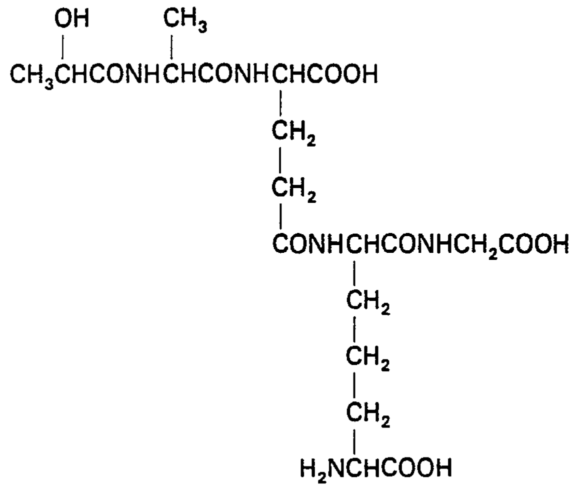

or pharmaceutically acceptable salt thereof in association with a pharmaceutically acceptable, substantially non-toxic carrier or excipient.

**Claims for the Contracting State: AT**

1. A process for the production of a substance of the formula:

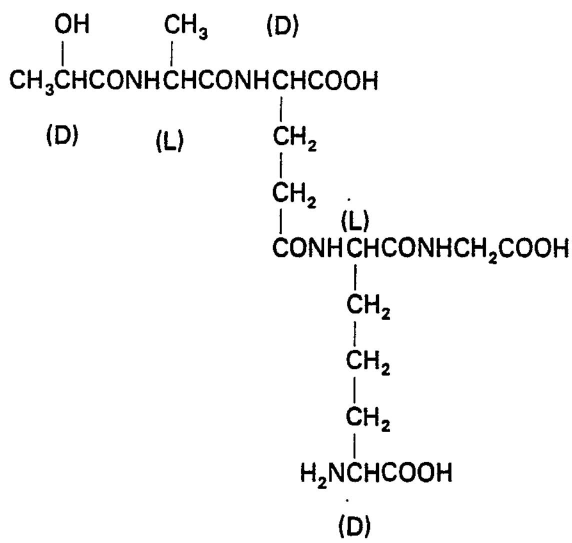

and pharmaceutically acceptable salts thereof which comprises cultivating under aerobic conditions Streptomyces olivaceogrises ATCC 31427 or Streptomyces violaceus ATCC 31481 in a culture medium containing assimilable sources of carbon, nitrogen and inorganic salt.

2. A process for preparation of a compound of the formula or its salt:

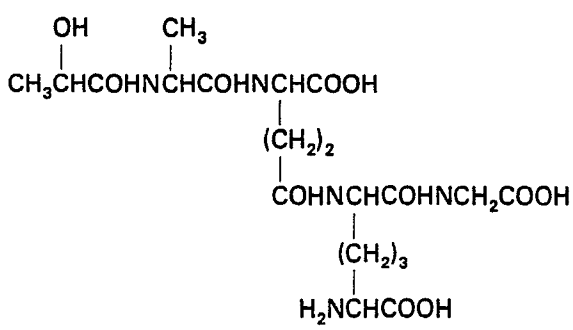

which comprises subjecting a compound of the formula or its salt:

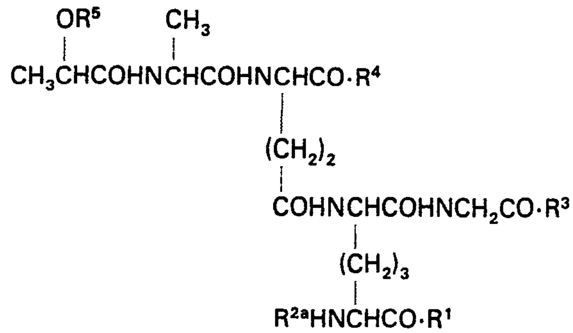

wherein $R^1$ is a hydroxy group, a protected hydroxy group or a protected hydrazino group of the formula —NHNHY wherein Y is an amino protective group; $R^{2a}$ is an amino protective group; $R^3$ is hydroxy group or a protected hydroxy group; $R^4$ is a protected hydroxy group and $R^5$ is a hydroxy protective group, to elimination reaction of protective groups.

**Patentansprüche für die Vertragsstaaten: BE, CH, DE, FR, GB, IT, LU, NL, SE**

1. Lactyltetrapeptid der Formel:

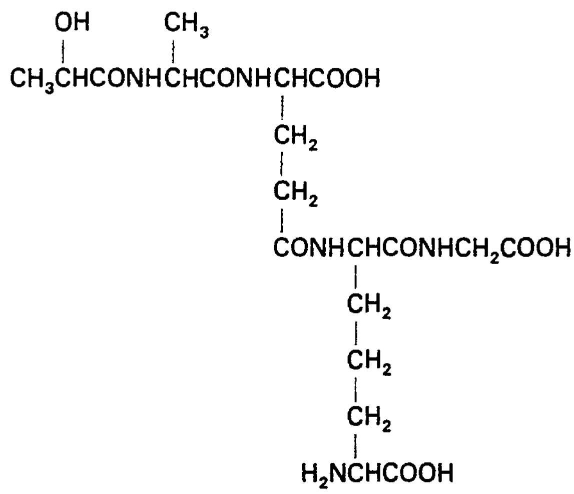

und pharmazeutisch brauchbare Salze davon.

2. Verbindung nach Anspruch 1, dargestellt durch die Formel:

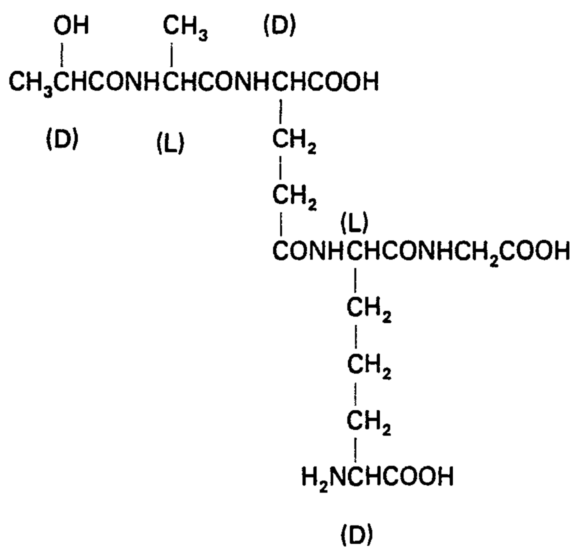

und pharmazeutisch brauchbare Salze davon.

3. Verbindung nach Anspruch 1, dargestellt durch die Formel:

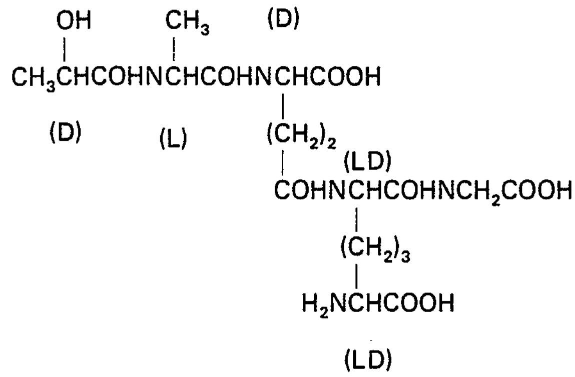

und pharmazeutisch brauchbare Salze davon.

4. Verbindung nach Anspruch 1, dargestellt durch die Formel:

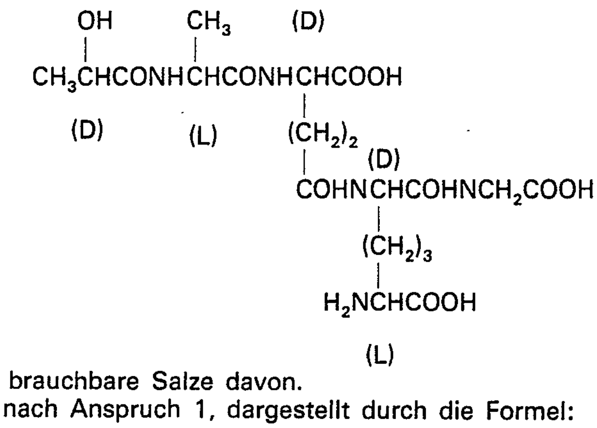

und pharmazeutisch brauchbare Salze davon.

5. Verbindung nach Anspruch 1, dargestellt durch die Formel:

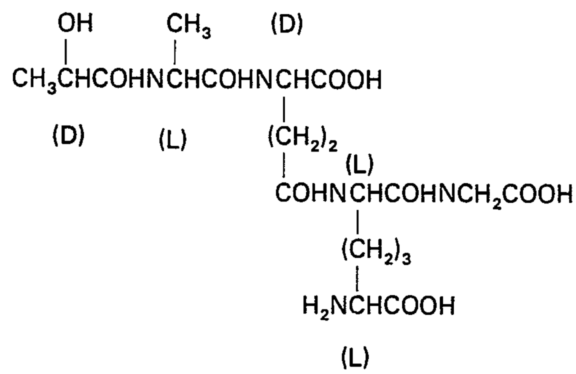

und pharmazeutisch brauchbare Salze davon.

6. Verbindung nach Anspruch 1, dargestellt durch die Formel:

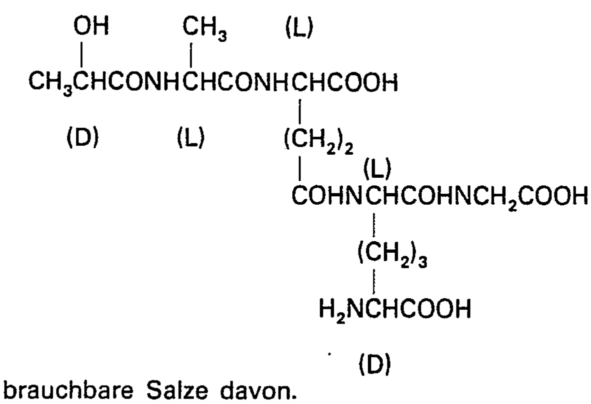

und pharmazeutisch brauchbare Salze davon.

7. Verbindung nach Anspruch 1, dargestellt durch die Formel:

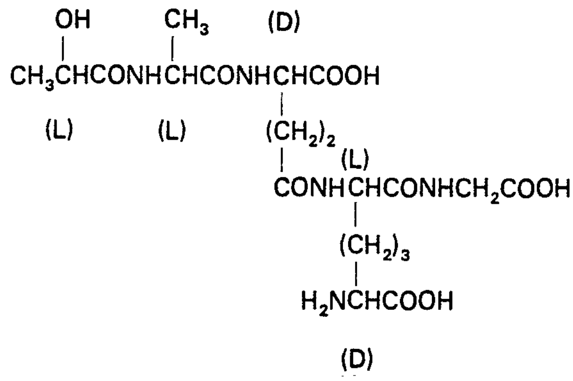

und pharmazeutisch brauchbare Salze davon.

8. Verbindung nach Anspruch 1, dargestellt durch die Formel:

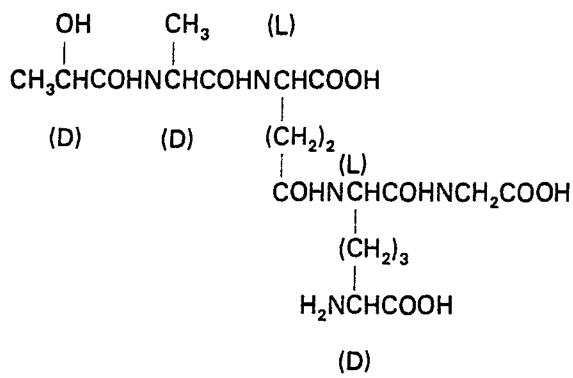

und pharmazeutisch brauchbare Salze davon.

9. Verbindung nach Anspruch 1, dargestellt durch die Formel:

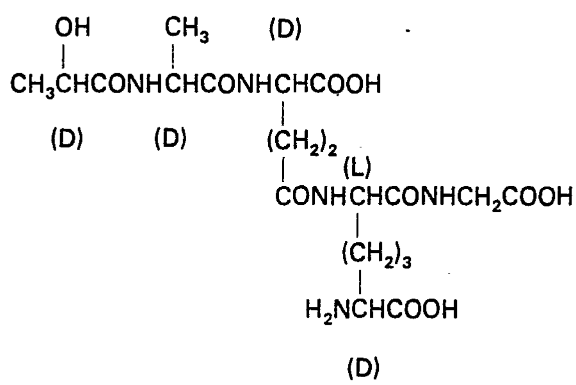

und pharmazeutisch brauchbare Salze davon.

10. Verfahren zur Herstellung einer Substanz mit der Formel:

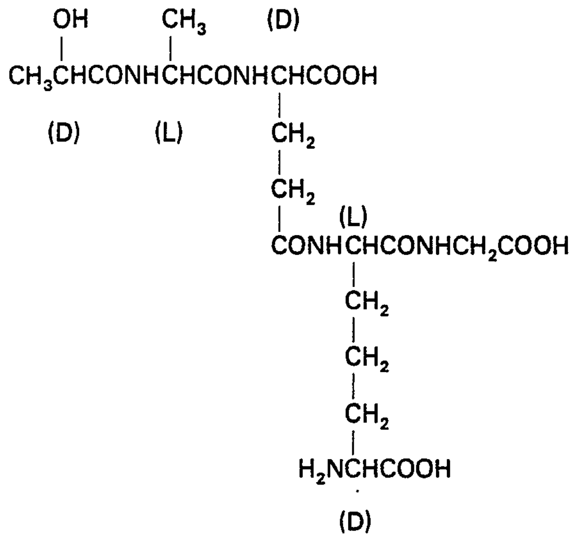

und der pharmazeutisch brauchbaren Salze davon, durch Kultivieren von Streptomyces olivaceogriseus ATCC 31427 oder Streptomyces violaceus ATCC 31481 unter aeroben Bedingungen in einem Kulturmedium, das assimilierbare Quellen für Kohlenstoff, Stickstoff und anorganische Salze enthält.

11. Verfahren zur Herstellung einer Substanz mit der Formel:

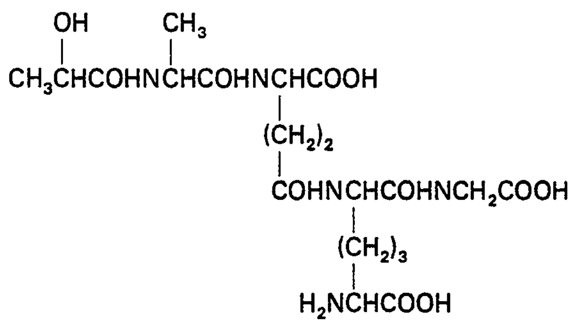

oder der pharmazeutisch brauchbaren Salze davon, dadurch gekennzeichnet, daß man aus einer Verbindung der Formel:

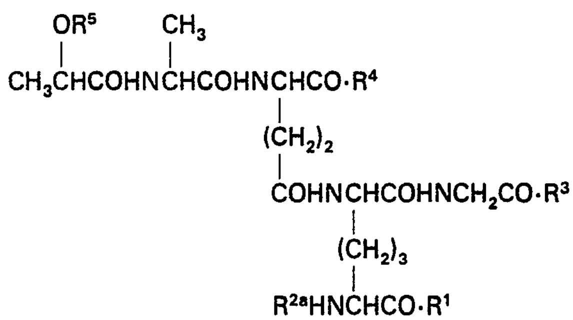

oder einem Salz davon, worin $R^1$ eine Hydroxygruppe, eine geschützte Hydroxygruppe oder eine geschützte Hydrazin-Gruppe der Formel: —NHNHY darstellt, wobei Y eine Aminoschutzgruppe ist; $R^{2a}$ eine Aminoschutzgruppe darstellt; $R^3$ eine Hydroxygruppe oder eine geschützte Hydroxygruppe bedeutet; $R^4$ eine geschützte Hydroxygruppe und $R^5$ eine Hydroxyschutzgruppe sind, die Schutzgruppen abspaltet.

12. Pharmazeutische Zusammensetzung, enthaltend eine Verbindung mit der Formel:

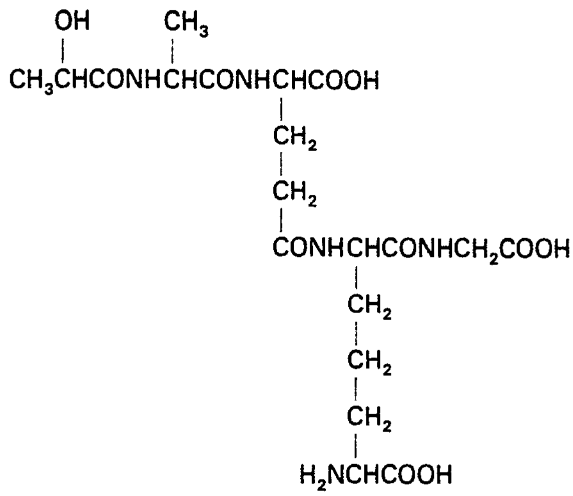

oder ein pharmazeutisch brauchbares Salz davon zusammen mit einem pharmazeutisch brauchbaren im wesentlichen nicht-toxischen Träger oder Excipienten.

**Patentansprüche für den Vertragsstaat: AT**

1. Verfahren zur Herstellung einer Substanz mit der Formel:

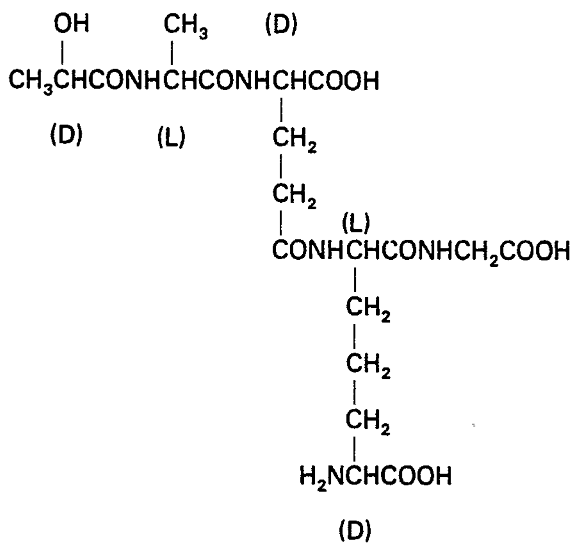

und der pharmazeutisch brauchbaren Salze davon durch Kultivieren von Streptomyces olivaceogriseus ATCC 31427 oder Streptomyces violaceus ATCC 31481 unter aeroben Bedingungen in einem Kulturmedium, das assimilierbare Quellen für Kohlenstoff, Stickstoff un anorganische Salze enthält.

2. Verfahren zur Herstellung einer Substanz mit der Formel:

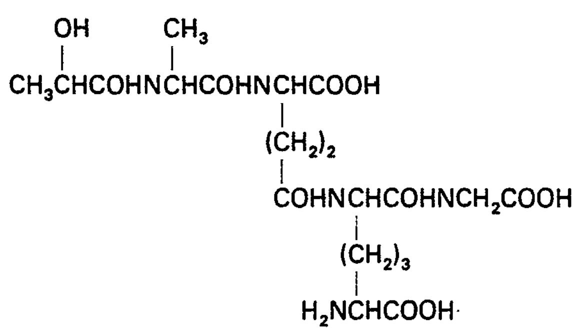

oder der pharmazeutisch brauchbaren Salze davon, dadurch gekennzeichnet, daß man aus einer Verbindung der Formel:

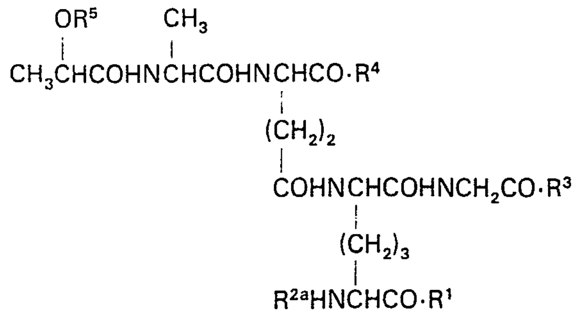

oder einem Salz davon, worin $R^1$ eine Hydroxygruppe, eine geschützte Hydroxygruppe oder eine geschützte Hydrazin-Gruppe der Formel: —NHNHY darstellt, wobei Y eine Aminoschutzgruppe ist; $R^{2a}$ eine Aminoschutzgruppe darstellt; $R^3$ eine Hydroxygruppe oder eine geschützte Hydroxygruppe bedeutet; $R^4$ eine geschützte Hydroxygruppe und $R^5$ eine Hydroxyschutzgruppe sind, die Schutzgruppen abspaltet.

**Revendications pour lès Etats contractants: BE, CH, DE, FR, GB, IT, LU, NL, SE**

1. Lactyl-tétrapeptide répondant à la formule:

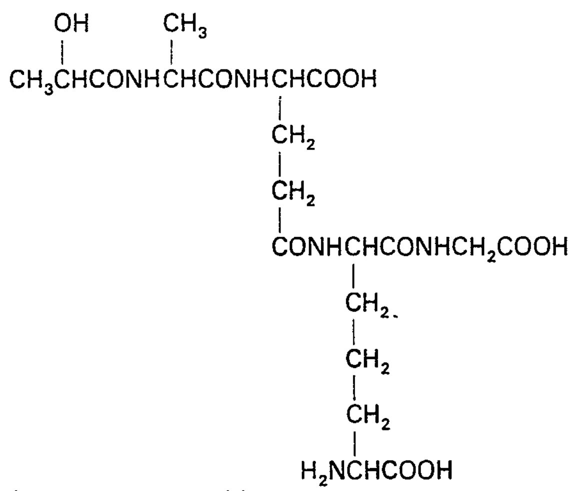

et ses sels pharmaceutiquement acceptables.

2. Composé selon la revendication 1, caractérisé en ce que le composé est représenté par la formule:

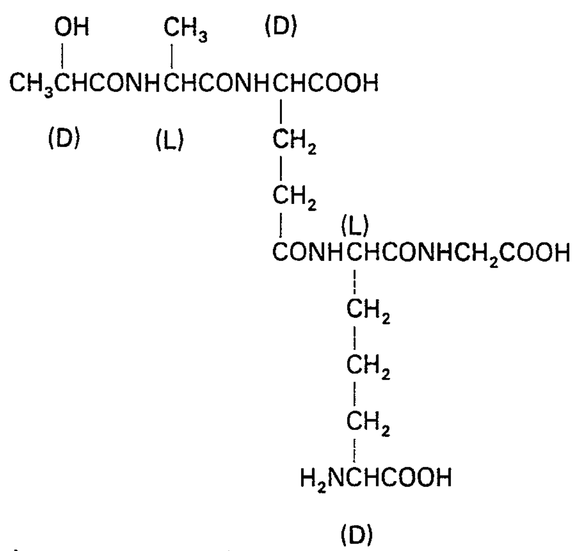

et ses sels pharmaceutiquement acceptables.

3. Composé selon la formule 1, caractérisé en ce que le composé est présenté par la formule:

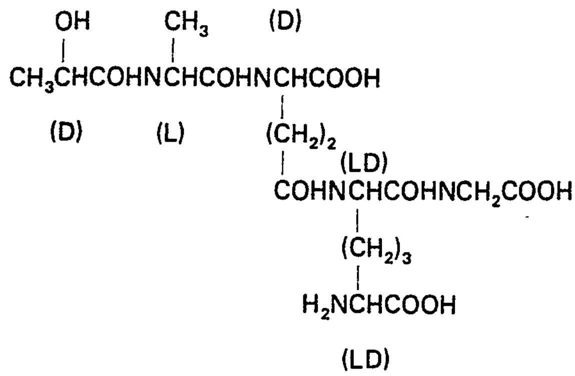

et ses sels pharmaceutiquement acceptables.

4. Composé selon la revendication 1, caractérisé en ce que le composé est représenté par la formule:

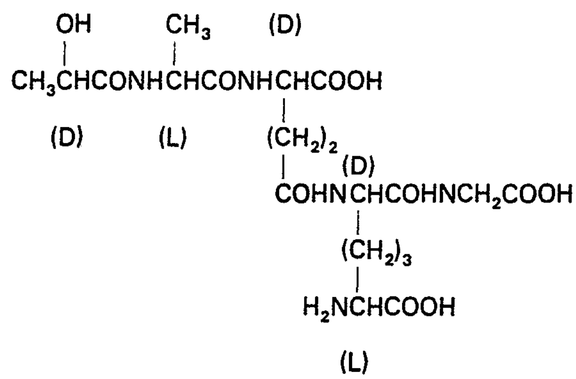

et ses sels pharmaceutiquement acceptables.

5. Composé selon la revendication 1, caractérisé en ce que le composé est représenté par la formule:

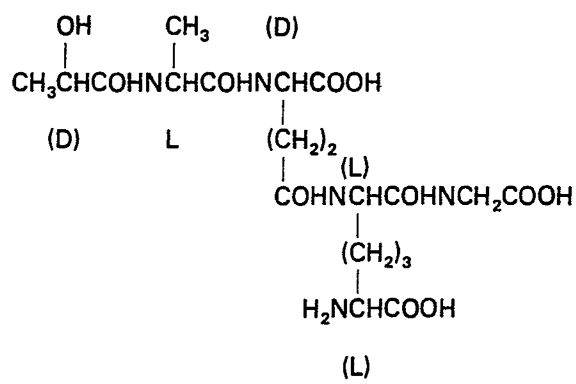

et ses sels pharmaceutiquement acceptables.

6. Composé selon la revendication 1, caractérisé en ce que le composé est représenté par la formule:

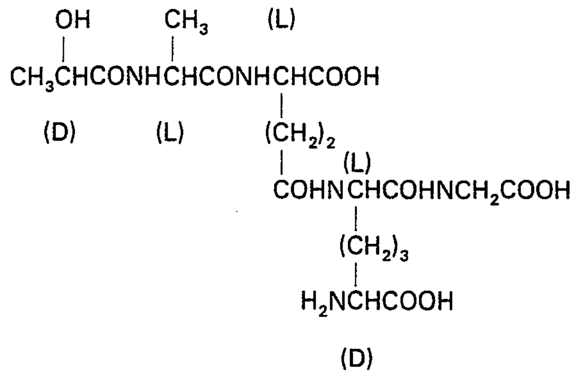

et ses sels pharmaceutiquement acceptables.

7. Composé selon la revendication 1, caractérisé en ce que le composé est représenté par la formule:

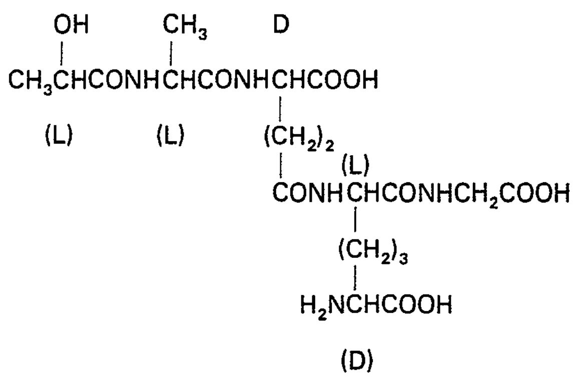

et ses sels pharmaceutiquement acceptables.

8. Composé selon la revendication 1, caractérisé en ce que le composé est représenté par la formule:

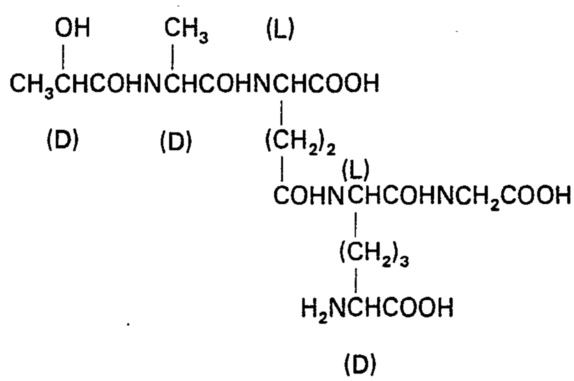

et ses sels pharmaceutiquement acceptables.

9. Composé selon la revendication 1, caractérisé en ce que le composé est représenté par la formule:

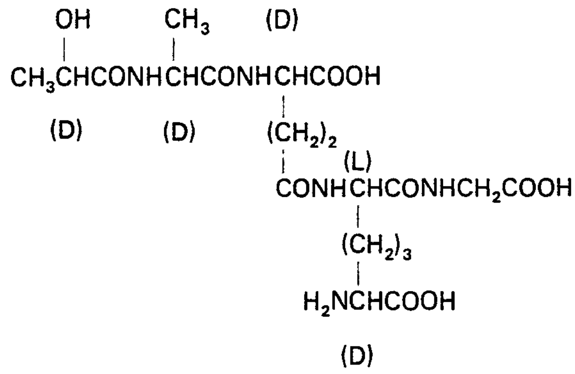

et ses sels pharmaceutiquement acceptables.

10. Procédé pour la production d'une substance répondant à la formule

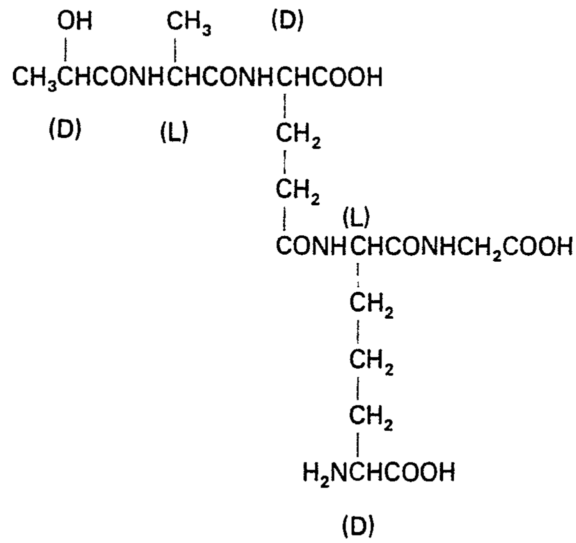

et de sels pharmaceutiquement acceptables, caractérisé en ce qu'il consiste à cultiver, dans des conditions aérobies, Streptomyces olivaceogriseus ATCC 31427 ou Streptomyces violaceus ATCC 31481 dans un milieu de culture contenant des sources assimilables de carbone, d'azote et de sel inorganique.

11. Un procédé pour la préparation d'un composé de formule ou de son sel

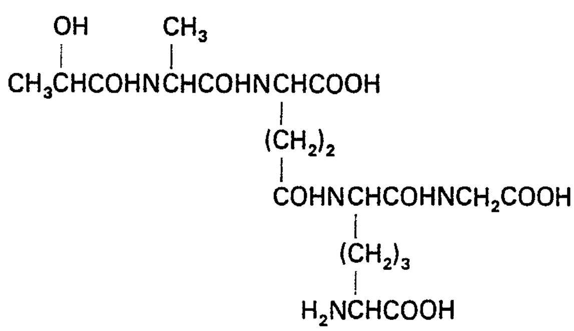

qui comprend l'étape consistant à soumettre un composé de formule ou son sel:

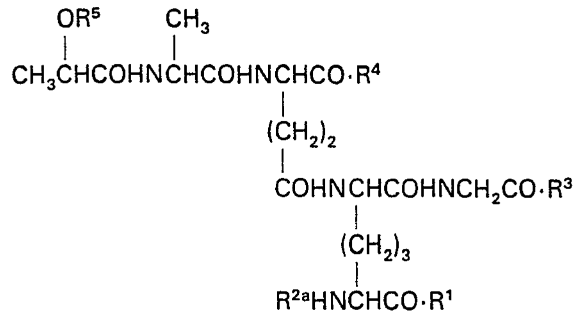

où $R^1$ est un groupe hydroxy, un groupe hydroxy protégé ou un groupe hydrazino protégé de formule —NHNHY où Y est un groupe protecteur du radical amino; $R^{2a}$ est un groupe protecteur du radical amino; $R^3$ est un groupe hydroxy ou un groupe protégé; $R^4$ est un groupe hydroxy protégé et $R^5$ est un groupe protecteur du radical hydroxy, à une réaction d'élimination des groupes protecteurs.

12. Une composition pharmaceutique comprenant un composé de formule

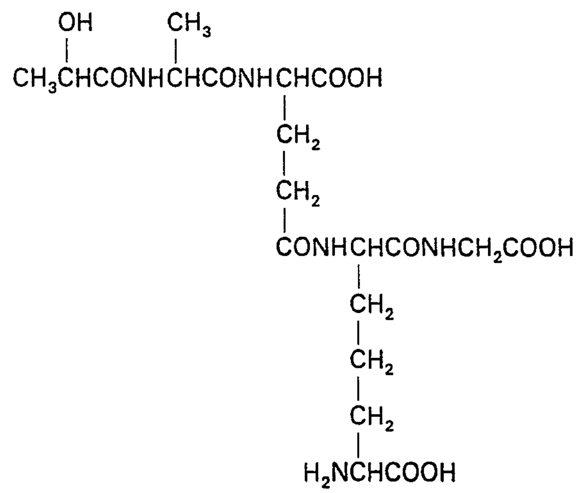

ou un sel pharmaceutiquement acceptable de ce composé en association avec un véhicule ou excipient pharmaceutiquement acceptable, sensiblement non toxique.

**Revendications pour l'Etat contractant: AT**

1. Procédé pour la production d'une substance de formule:

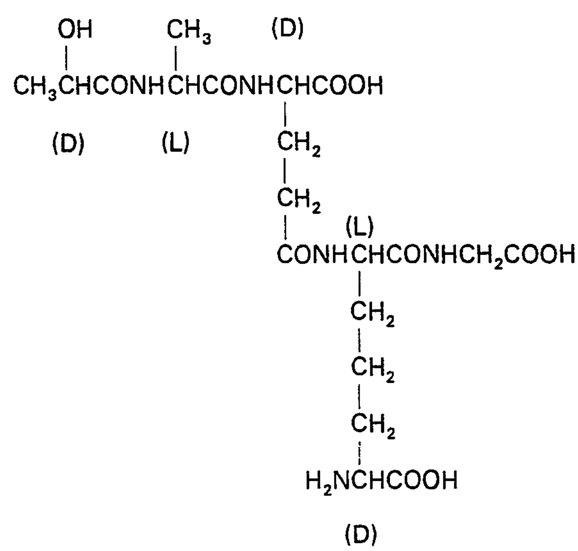

et de ses sels pharmaceutiquement acceptables, caractérisé en ce qu'il consiste à cultiver, dans des

conditions aérobies, Streptomyces olivaceogriseus ATCC 31427 ou Streptomyces violaceus ATCC 31481 dans un milieu de culture contenant des sources assimilables de carbone, d'azote et de sel inorganique.

2. Un procédé pour la préparation d'un composé de formule ou de son sel

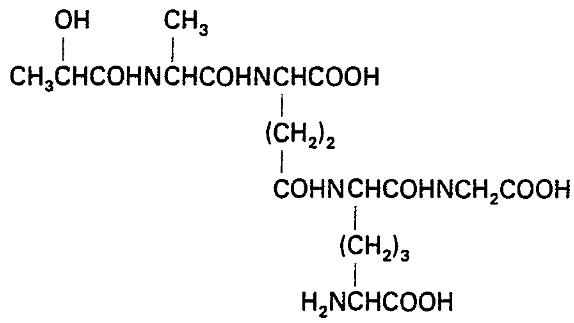

qui comprend l'étape consistant à soumettre un composé de formule ou son sel:

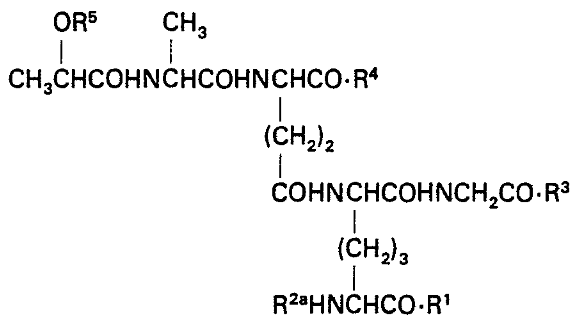

où $R^1$ est un groupe hydroxy, un groupe hydroxy protégé ou un groupe hydrazino protégé de formule —NHNHY où Y est un groupe protecteur du radical amino; $R^{2a}$ est un groupe protecteur du radical amino; $R^3$ est un groupe hydroxy ou un groupe hydroxy protégé; $R^4$ est un groupe hydroxy protégé et $R^5$ est un groupe protecteur du radical hydroxy, à une réaction d'élimination des groupes protecteurs.

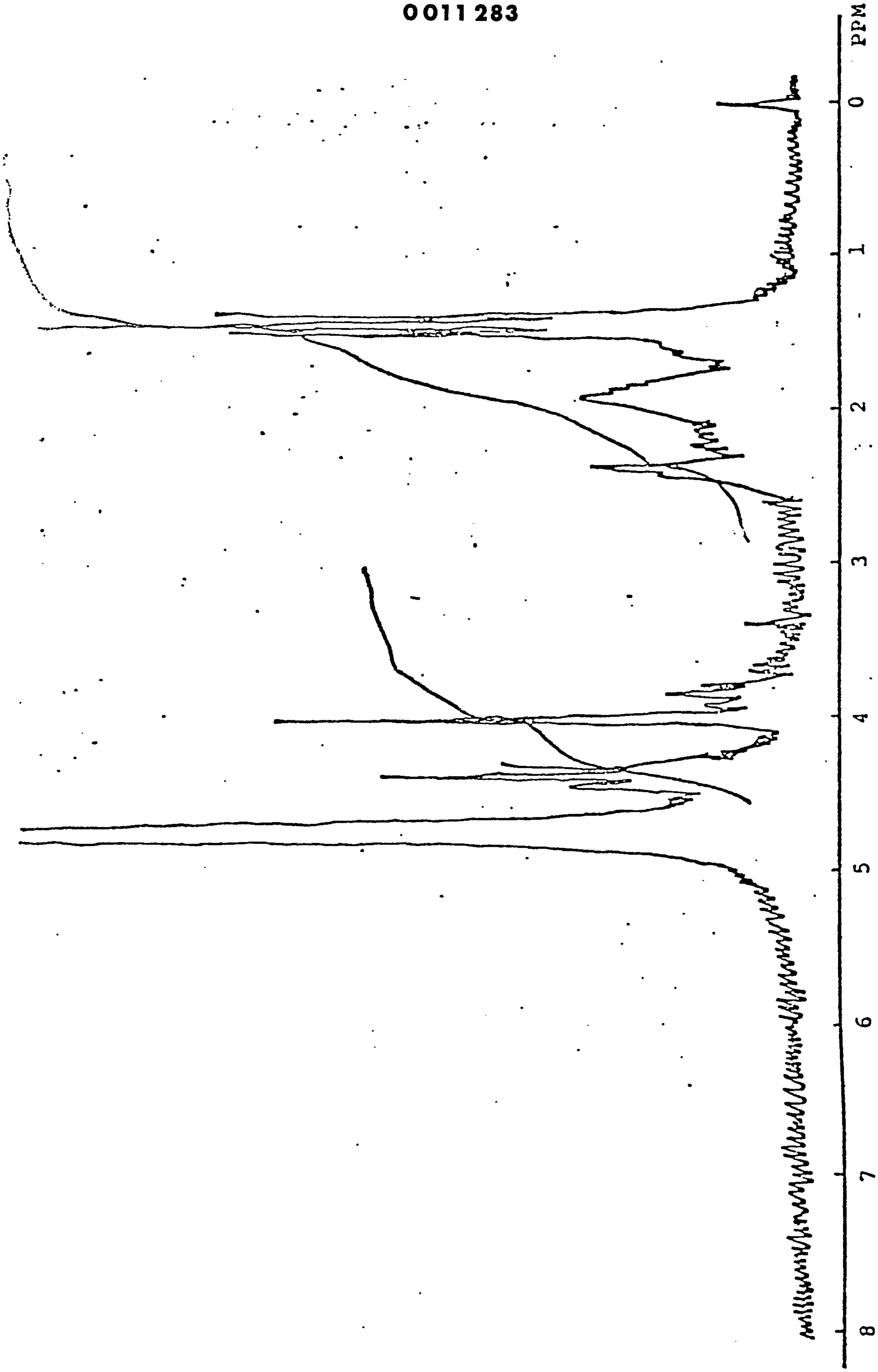


FIGURE